Europäisches Patentamt

European Patent Office

Office européen des brevets

(19)

(11) Numéro de publication: **0 506 574 B1**

(12) **FASCICULE DE BREVET EUROPEEN**

(45) Date de publication du fascicule du brevet: **29.11.95**

(51) Int. Cl.⁶: **C12N 15/19**, C12P 21/02, C07K 14/00, A61K 38/00, C12N 1/21, C12N 5/10, C12N 1/19, //C12N1:21, C12R1:19,C12N1:19,C12R1:865

(21) Numéro de dépôt: **92400858.4**

(22) Date de dépôt: **27.03.92**

(54) **Protéine à activité de type cytokine, ADN recombinant codant pour cette protéine, cellules et microorganismes transformés.**

(83) Déclaration conformément à la règle 28(4) CBE(solution de l'expert)

(30) Priorité: **29.03.91 FR 9103904**
**08.01.92 FR 9200137**

(43) Date de publication de la demande:
**30.09.92 Bulletin 92/40**

(45) Mention de la délivrance du brevet:
**29.11.95 Bulletin 95/48**

(84) Etats contractants désignés:
**AT BE CH DE DK ES FR GB GR IT LI LU NL PT SE**

(56) Documents cités:
**WO-A-90/07009**

(73) Titulaire: **SANOFI**
**32-34, rue Marbeuf**
**F-75008 Paris (FR)**

(72) Inventeur: **Caput, Daniel**
**10, rue Temponnières**
**F-31000 Toulouse (FR)**
Inventeur: **Ferrara, Pascual**
**Lieu-dit : "Libouille",**
**Saint Assiscle-Avignonet**
**F-31290 Villefranche de Lauragais (FR)**
Inventeur: **Guillemot, Jean-Claude, 135, Chemin de la Salade**
**Ponsan,**
**Résidence les Balcons de Pechdavid**
**F-31400 Toulouse (FR)**
Inventeur: **Kaghad, Mourad**
**50, rue Romain Rolland**
**F-31520 Ramonville-Saint Agne (FR)**
Inventeur: **Labit-le Bouteiller, Christine**
**23, rue de l'Amendier**
**F-31000 Toulouse (FR)**

EP 0 506 574 B1

JOURNAL OF IMMUNOLOGY, vol. 142, no. 2, 15 janvier 1989, Baltimore, MD (US); K.D. BROWN et al., pp. 679-687

Inventeur: **Leplatois, Pascal, En Saint-Pierre Cambon les Lavaur F-81470 Cuq Toulza (FR)**
Inventeur: **Magazin, Marilyn 2, chemin de la Crabotte F-31320 Castanet-Tolosan (FR)**
Inventeur: **Minty, Adrian Chemin de Pechmirol F-31320 Mervilla (FR)**

(74) Mandataire: **Gillard, Marie-Louise et al Cabinet Beau de Loménie 158, rue de l'Université F-75340 Paris Cédex 07 (FR)**

## Description

La présente invention a pour objet une nouvelle protéine à activité de type cytokine, les outils de génie génétique pour la produire, à savoir un ADN recombinant, un vecteur d'expression portant cet ADN recombinant, les microorganismes procaryotes et les cellules eucaryotes contenant cet ADN recombinant ainsi qu'un médicament, utile notamment comme immunomodulateur, contenant à titre de principe actif cette protéine.

Il est bien connu que le système immunitaire comprend des éléments cellulaires et des substances solubles secrétées par ces derniers, appelées cytokines. Celles-ci sont des protéines qui assurent la communication entre une cellule émettrice et une cellule cible appartenant, soit au système immunitaire, soit à un autre système biologique de l'organisme. Les cytokines ont en général une activité biologique dite pléiotropique : elles peuvent avoir de multiples actions sur la cellule cible : prolifération, différenciation, cytolyse, activation, chimiotactisme, etc... Plusieurs de ces molécules ont déjà trouvé des applications en thérapeutique : par exemple, l'interleukine-2 ou l'interféron-$\alpha$ utilisés pour le traitement de certaines tumeurs par immunothérapie et les facteurs myélopeiëtiques tels que le GCSF (Granulocyte Colony Stimulating Factor) ou le GMCSF (Granulocyte Monocyte Colony Stimulating Factor) qui stimulent la croissance et la différenciation des cellules sanguines et permettent ainsi l'enrichissement en ces dernières du sang appauvri en celles-ci, suite, par exemple, à une chimiothérapie.

On a notamment les connaissances suivantes à propos des cytokines :

1) Celles-ci correspondent à des protéines sécrétées. La sécrétion d'une protéine, donc en particulier d'une cytokine, s'effectue le plus souvent par un mécanisme qui comprend le clivage d'une région aminoterminale hydrophobe de la protéine traduite, appelée séquence peptidique pré ou peptide signal, lors de l'excrétion de la protéine mature dans le milieu (Von Heijne G., 1986, Nucl. Ac. Res., 14, 4683-4690). La plupart des séquences peptidiques des cytokines connues comprennent effectivement un peptide signal.

2) les cytokines correspondent en général à des protéines inductibles, soit par d'autres cytokines (K. Matsushima et al, 1988, J. Exp. Med., 167, 1883-1893 - Shimizu H. et al, 1990, Mol. Cell. Biol., 10, 561-568), soit par des agents chimiques activateurs de la prolifération et de la différenciation cellulaire tels que les lipopolysaccharides, les ionophores calciques par exemple la calimicine, le dibutyryl AMP cyclique, le diméthylsulfoxyde, l'acide rétinoïque, la concanavaline A, la phytohémaglutinine (PHA-P) et les esters de phorbol, par exemple le phorbol myristate-2 acétate-3 (PMA) (Muegge K. et Durun S.K., 1990, Cytokine 2, 1-8 - C.B. Thompson et al, 1989, Proc. Natl. Acad. Sci., 86, 1333-1337), soit par des anticorps contre les molécules de surface, tels que les antigènes de surface CD2, CD3, CD28 et CD40 (Thomson C.B. et al, référence ci-dessus et Rousset F. et al, 1991, J. Exp. Med., 173, 703-710).

3) L'induction des cytokines étant transitoire, les ARN messagers des cytokines sont instables. Ils possèdent pour la plupart des cytokines des régions riches en A et U notamment la séquence AUUUA, séquence d'instabilité consensus mise en évidence par D. Caput et al, 1986, Proc. Ntl. Acad. Sci. USA, 83, 1670-1674 ; G. Shaw et al, 1986, Cell, 46, 659-667 ainsi que K. Peppel et al, 1991, J. Exp. Med., 173, 349-355.

4) La grande majorité des cytokines connues sont synthétisées par les cellules du système immunitaire, notamment par les monocytes et les lymphocytes T auxiliaires du sang périphérique (Ullman K.S. et al., 1989, Ann. Rev. Imm., 8, 421-452 et l'ouvrage "Macrophage derived cell regulatory factors" de C. Sorg, publié en 1989 par Karger-Bâle-Suisse). Leur synthèse est induite par les agents inducteurs mentionnés en 2).

Il est connu par ailleurs qu'on est capable de construire à partir de cellules mononucléaires du sang périphérique, constituées principalement de lymphocytes et de monocytes, cultivées et stimulées à l'aide de différents agents inducteurs tels que la phytohémaglutinine, le phorbol myristate-2 acétate-3 et l'anticorps monoclonal anti-CD2, une banque d'ADN complémentaire soustraite des séquences ubiquitaires d'ADN complémentaire dans les cellules animales, et donc enrichie en ADN complémentaire codant pour des cytokines (H.C. Chang et al, Eur. J. Imm., 1989, 19, 1045-1051, P.F. Zipfel et al, 1989, Mol. Cell. Biol, 9, 1041-1048).

On sait d'autre part (C. B. Thompson et al, 1989, Proc. Ntl. Acad. Sci., 86, 1333-1337 et Lindsten T. et al, 1989, Science, 244, 339) que la production de cytokines (appelées lymphokines) par les lymphocytes T, est plus importante, donc correspond à des quantités d'ARN messagers plus grandes, avec une stimulation à l'aide de la combinaison binaire : phorbol myristate-2 acétate-3 et phytohémaglutinine ou phorbol myristate-2 acétate-3 et l'anticorps anti -CD28, qu'avec le phorbol myristate-2 acétate-3 uniquement et avec la combinaison binaire : phorbol myristate-2 acétate-3 et phytohémaglutinine qu'avec la combinaison tertiaire : phorbol myristate-2 acétate-3, phytohémaglutinine et cyclosporine A, la cyclosporine A ayant apparemment un effet inhibiteur sur la transcription des ARN messagers des cytokines (Thompson et al, référence citée ci-dessus et Mattila et al., 1990, EMBO J., 9, 4425-4433).

Cette expression différentielle des ARN messagers des cytokines dans différentes conditions de stimulation peut servir à un criblage, appelé criblage différentiel, d'une banque d'ADN complémentaire contenant des séquences codant pour des cytokines dans le but de sélectionner ces dernières séquences (cf notamment Dworkin et al, 1980, Dev. Biol., 76, 449-464, Cochran B.M. et al, 1983, Cell, 33, 939-947 et Zipfel P.F. et al, 1989, Mol. Cell. Biol., 9, 1041-1048). Il est en effet possible de construire à partir de deux ARN messagers correspondant à deux états de stimulation des cellules, appelés ici état de stimulation 1 et état de stimulation 2, l'état de stimulation 2 étant supposé plus riche en ARN messagers codant pour une cytokine que l'état 1, deux sondes radioactives appelées respectivement sonde 1 et sonde 2, qui sont des transcripts obtenus à l'aide de la transcriptase inverse de ces deux ARN messagers. On hybride ces deux sondes avec des colonies bactériennes contenant des séquences d'ADN complémentaire de la banque. Les colonies bactériennes contenant un ADN complémentaire dont l'ARN messager change d'abondance entre les deux états d'activation vont donner des signaux différentiels avec les deux sondes, l'hybridation étant plus importante pour les clones retenus (qui correspondent à des protéines inductibles) pour la sonde 2 que pour la sonde 1.

Il est connu par ailleurs que les cellules COS, cellules de reins de singe exprimant l'antigène T du virus SV40 (Gluzman Y., Cell, 23, 1981, 175-182), qui permettent la réplication des vecteurs contenant l'origine de réplication de l'ADN du virus SV40, constituent des hôtes de choix pour l'étude de l'expression de gènes dans les cellules animales et de la sécrétion des protéines exprimées. La sécrétion d'une protéine dans ces cellules indique que celle-ci est sécrétée par la cellule qui la produit naturellement (H.C. Chang et al., Eur. J. Imm., 1989, 19, 1045-1051 et W.Y. Weiser et al., 1989, Proc. Ntl. Acad. Sci. USA, 86, 7522-7526)

La présente invention a pour objet une protéine à activité de type cytokine, ou un précurseur de cette protéine qui comprend la séquence d'acides aminés (a1) suivante :

$$
\begin{array}{cccc}
\text{Gly Pro Val Pro Pro Ser Thr Ala Leu Arg Glu Leu Ile Glu Glu Leu} \\
1 \qquad\qquad 5 \qquad\qquad 10 \qquad\qquad 15 \\
\text{Val Asn Ile Thr Gln Asn Gln Lys Ala Pro Leu Cys Asn Gly Ser Met} \\
20 \qquad\qquad 25 \qquad\qquad 30 \\
\text{Val Trp Ser Ile Asn Leu Thr Ala Xaa Met Tyr Cys Ala Ala Leu Glu} \\
35 \qquad\qquad 40 \qquad\qquad 45 \\
\text{Ser Leu Ile Asn Val Ser Gly Cys Ser Ala Ile Glu Lys Thr Gln Arg} \\
50 \qquad\qquad 55 \qquad\qquad 60 \\
\text{Met Leu Ser Gly Phe Cys Pro His Lys Val Ser Ala Gly Gln Phe Ser} \\
65 \qquad\qquad 70 \qquad\qquad 75 \qquad\qquad 80 \\
\text{Ser Leu His Val Arg Asp Thr Lys Ile Glu Val Ala Gln Phe Val Lys} \\
85 \qquad\qquad 90 \qquad\qquad 95 \\
\text{Asp Leu Leu Leu His Leu Lys Lys Leu Phe Arg Glu Gly Arg Phe Asn} \\
100 \qquad\qquad 105 \qquad\qquad 110
\end{array}
$$

dans laquelle Xaa représente Asp ou Gly,
ou une séquence présentant un degré d'homologie élevé avec la séquence (a$_1$)

Un degré d'homologie élevée signifie ici une homologie d'identité (rapport entre les acides aminés identiques et le nombre total d'acides aminés) d'au moins 80 %, et de préférence d'au moins 90 %, des séquences d'acides aminés, lorsqu'elles sont alignées d'après l'homologie maximale, selon la méthode d'alignement optimal des séquences de Needleman et Wunsch, 1970, J. Mol. Biol., 48, 443-453. Cette méthode algorithmique, qui considère tous les alignements possibles et crée un alignement dans lequel le plus possible d'acides aminés identiques sont appariés et le nombre de trous dans les séquences alignées est minimal, est notamment utilisée dans le logiciel UWGCG de l'Université du Wisconsin : Devereux et al, 1984, Nucl. Ac. Res., 12, 8711-8722- option GAP.

La séquence peptidique déjà connue la plus proche de celle de la séquence ($a_1$) de 112 acides aminés est celle de 132 acides aminés déduite de l'ADN complémentaire de la protéine P600 de souris, décrite par K.D. Brown et al., 1989, J. Imm., 142, 679-687 (protéine exprimée par les lymphocytes TH2 de souris, dont la fonction n'a pas été élucidée). Une comparaison de ces séquences peptidiques à l'aide de la méthode de Needleman et Wunsch, 1970, J. Mol. Biol., 48, 443-453 montre que 63 acides aminés sont identiques sur 112, soit une homologie d'identité d'environ 56 %.

La protéine de la présente invention est une protéine sécrétée notamment par les lymphocytes T et inductible dans ces derniers par le phorbol myristate-2 acétate-3, cette induction étant - comme attendu pour une cytokine (v. texte ci-dessus) - amplifiée par la phytohémaglutinine ou l'anticorps monoclonal anti-CD28 avec un effet inhibiteur de cette augmentation dans le cas de la stimulation par la phytohémaglutinine en présence supplémentaire de cyclosporine A. La protéine selon l'invention, dénommée protéine NC30, est donc une nouvelle lymphokine humaine possédant des activités immunomodulatrices de type cytokine (prolifération cellulaire, activation cellulaire, chimiotactisme et régulation de la synthèse d'autres cytokines). Elle agit sur au moins deux cellules clés du système immunitaire : les monocytes et les lymphocytes B. Il s'agit donc d'une nouvelle interleukine. Certaines de ses propriétés sont en commun avec l'interleukine-4 : inhibition de la synthèse de l'interleukine-1$\beta$ et de l'interleukine-6 par les monocytes du sang périphérique activés au LPS et modulation de l'expression de l'antigène CD23 sur les lymphocytes B des amygdales. Ces deux propriétés sont également celles de l'interleukine-4 (W. Paul, 1991, Blood, 77, 1959 et Waal Malefyt et al, 1991, J. Exp. Med., 174, 1199-1220).

La protéine de l'invention est une nouvelle interleukine, de type lymphokine. Elle présente un intérêt comme principe d'un médicament utile pour le traitement par immunomodulation des tumeurs et de certains états infectieux ou inflammatoires.

La protéine de l'invention peut comprendre, immédiatement en amont de la séquence (a1) un ou plusieurs acides aminés, en particulier la séquence : Ser-Pro.

Cette protéine est de préférence sous une forme qui a une masse moléculaire apparente, déterminée par électrophorèse sur gel de polyacrylamide en présence de SDS de 9,0 ±2 ou 16,0 ± 2 kDa. Elle est avantageusement N-glycosylée, en particulier lorsqu'elle a une masse moléculaire apparente de 16,0 ± 2 kDa.

Il est intéressant que cette protéine présente un degré de pureté, déterminé par électrophorèse sur gel de polyacrylamide en présence de SDS et révélation à l'argent, supérieur à 70 %, de préférence supérieur à 90%.

L'invention a aussi pour objet un ADN recombinant caractérisé en ce qu'il code pour la protéine précédente, laquelle peut être alors obtenue à partir du lysat cellulaire ou, avantageusement, pour un précurseur de la protéine précédente. Ce précurseur comprend de préférence une séquence signal.

Cette séquence signal, choisie en fonction de la cellule hôte, a pour fonction de permettre l'exportation de la protéine recombinante en dehors du cytoplasme, ce qui permet à la protéine recombinante de prendre une conformation proche de celle de la protéine naturelle et facilite considérablement sa purification. Cette séquence signal peut être clivée, soit en plusieurs étapes par un signal-peptidase que libère la protéine mature, soit en plusieurs étapes lorsque cette séquence signal comprend en plus de la séquence éliminée par la signal-peptidase, appelée peptide signal ou séquence pré, une séquence éliminée plus tardivement au cours de un ou plusieurs évènements protéolytiques, appelée séquence pro.

La séquence codant pour la protéine mature est par exemple l'une des séquences ($Na_1$) ou ($Na'_1$) suivantes :

(Na$_1$)

```
GGCCCTGTGC  CTCCCTCTAC  AGCCCTCAGG  GAGCTCATTG
AGGAGCTGGT  CAACATCACC  CAGAACCAGA  AGGCTCCGCT
CTGCAATGGC  AGCATGGTAT  GGAGCATCAA  CCTGACAGCT
GACATGTACT  GTGCAGCCCT  GGAATCCCTG  ATCAACGTGT
CAGGCTGCAG  TGCCATCGAG  AAGACCCAGA  GGATGCTGAG
CGGATTCTGC  CCGCACAAGG  TCTCAGCTGG  GCAGTTTCC
AGCTTGCATG  TCCGAGACAC  CAAAATCGAG  GTGGCCCAGT
TTGTAAAGGA  CCTGCTCTTA  CATTTAAAGA  AACTTTTTCG
CGAGGGACGG  TTCAAC
```

(Na'$_1$)

```
GGCCCTGTGC  CTCCCTCTAC  AGCCCTCAGG  GAGCTCATTG
AGGAGCTGGT  CAACATCACC  CAGAACCAGA  AGGCTCCGCT
CTGCAATGGC  AGCATGGTAT  GGAGCATCAA  CCTGACAGCT
GGCATGTACT  GTGCAGCCCT  GGAATCCCTG  ATCAACGTGT
CAGGCTGCAG  TGCCATCGAG  AAGACCCAGA  GGATGCTGAG
CGGATTCTGC  CCGCACAAGG  TCTCAGCTGG  GCAGTTTCC
AGCTTGCATG  TCCGAGACAC  CAAAATCGAG  GTGGCCCAGT
TTGTAAAGGA  CCTGCTCTTA  CATTTAAAGA  AACTTTTTCG
CGAGGGACGG  TTCAAC
```

Pour une expression dans les microorganismes procaryotes, tels que par exemple Escherichia coli, cette séquence signal peut être, soit une séquence dérivée de la séquence codant pour un précurseur naturel d'une protéine exportée par un microorganisme procaryote (par exemple le peptide signal OmPA [Ghrayeb et al., 1984, EMBO Journal, 3, 2437-2442] ou le peptide signal de la phosphatase alcaline [Michaelis et al., J. Bact. 1983, 154, 366-374]), soit une séquence non endogène provenant d'une séquence codant pour un précurseur eucaryote (par exemple le peptide signal de l'un des précurseurs naturels de l'hormone de croissance humaine), soit une séquence codant pour un peptide signal synthétique (par exemple celui décrit dans la demande de brevet française n° 2 636 643, de séquence :

```
Met Ala Pro Ser Gly Lys Ser Thr Leu Leu Leu Leu Phe Leu Leu Leu
 1               5                  10                    15
Cys Leu Pro Ser Trp Asn Ala Gly Ala
           20                  25
```

Pour une expression dans les cellules eucaryotes telles que les ascomycètes, par exemple la levure Saccharomyces cerevisiae ou le champignon filamenteux Cryphonectria parasitica, cette séquence signal est de préférence une séquence dérivée d'une séquence codant pour un précurseur naturel d'une protéine sécrétée par ces cellules, par exemple pour la levure le précurseur de l'invertase (demande de brevet EP O123289) ou le précurseur de la séquence prépro de la phéromone alpha (demande de brevet DK 2484/84), ou pour Cryphonectria parasitica, le précurseur de la séquence prépro de l'endothiapepsine, de séquence :

Met Ser Ser Pro Leu Lys Asn Ala Leu Val Thr Ala Met Leu Ala Gly
1           5             10            15

Gly Ala Leu Ser Ser Pro Thr Lys Gln His Val Gly Ile Pro Val Asn
20          25           30

Ala Ser Pro Glu Val Gly Pro Gly Lys Tyr Ser Phe Lys Gln Val Arg
35          40           45

Asn Pro Asn Tyr Lys Phe Asn Gly Pro Leu Ser Val Lys Lys Thr Tyr
50          55           60

Leu Lys Tyr Gly Val Pro Ile Pro Ala Trp Leu Glu Asp Ala Val Gln
65          70           75           80

Asn Ser Thr Ser Gly Leu Ala Glu Arg
85

Pour une expression dans les cellules animales, on utilise comme séquence signal, soit une séquence signal d'une protéine de cellule animale connue pour être exportée -par exemple une séquence codant pour le peptide signal de l'un des précurseurs naturels de l'hormone de croissance humaine, déjà connue pour permettre la sécrétion de l'interleukine-2 (cf. la demande de brevet française 2 619 711), soit l'une des quatre séquences signal (b1), (b2), (b3) et (b4) suivantes :

**(b1)**

Met His Pro Leu Leu Asn Pro Leu Leu Leu Ala Leu Gly Leu Met Ala
1           5             10            15

Leu Leu Leu Thr Thr Val Ile Ala Leu Thr Cys Leu Gly Gly Phe Ala
20          25           30

**(b2)**

Met His Pro Leu Leu Asn Pro Leu Leu Leu Ala Leu Gly Leu Met Ala
1           5             10            15

Leu Leu Leu Thr Thr Val Ile Ala Leu Thr Cys Leu Gly Gly Phe Ala
20          25           30

Ser Pro

**(b3)**

Met Ala Leu Leu Leu Thr Thr Val Ile Ala Leu Thr Cys Leu Gly Gly
1           5             10            15

Phe Ala

**(b4)**

Met Ala Leu Leu Leu Thr Thr Val Ile Ala Leu Thr Cys Leu Gly Gly
1           5             10            15

Phe Ala Ser Pro
20

avantageusement codées respectivement par les séquences (Nb1), (Nb2), (Nb3) et (Nb4) suivantes :

**(Nb1)**

ATGCATCCGC  TCCTCAATCC  TCTCCTGTTG GCACTGGGCC
TCATGGCGCT  TTTGTTGACC  ACGGTCATTG CTCTCACTTG
CCTTGGCGGC  TTTGCC

**(Nb2)**

ATGCATCCGC  TCCTCAATCC  TCTCCTGTTG GCACTGGGCC
TCATGGCGCT  TTTGTTGACC  ACGGTCATTG CTCTCACTTG
CCTTGGCGGC  TTTGCCTCCC  CA

**(Nb3)**

ATGGCGCTTT  TGTTGACCAC  GGTCATTGCT CTCACTTGCC
TTGGCGGCTT  TGCC

**(Nb4)**

ATGGCGCTTT  TGTTGACCAC  GGTCATTGCT CTCACTTGCC
TTGGCGGCTT  TGCCTCCCCA

L'invention concerne également un vecteur d'expression qui porte, avec les moyens nécessaires à son expression, l'ADN recombinant défini précédemment.

Pour une expression dans les micro-organismes procaryotes, en particulier dans Escherichia coli, l'ADN recombinant doit être inséré dans un vecteur d'expression comportant notamment un promoteur efficace, suivi d'un site de fixation des ribosomes en amont du gène à exprimer, ainsi qu'une séquence d'arrêt de transcription efficace en aval du gène à exprimer. Ce plasmide doit également comporter une origine de réplication et un marqueur de sélection. Toutes ces séquences doivent être choisies en fonction de la cellule hôte.

Pour une expression dans les cellules eucaryotes, le vecteur d'expression selon l'invention porte l'ADN recombinant défini précédemment avec les moyens nécessaires à son expression, à sa réplication dans les cellules eucaryotes et à la sélection des cellules transformées. De préférence, ce vecteur porte un marqueur de sélection, choisi par exemple pour complémenter une mutation des cellules eucaryotes réceptrices, qui permet la sélection des cellules qui ont intégré l'ADN recombinant en un nombre de copies élevé soit dans leur génome, soit dans un vecteur multicopie.

Pour une expression dans les cellules eucaryotes telles que la levure, par exemple Saccharomyces cerevisiae, il convient d'insérer l'ADN recombinant entre, d'une part, des séquences reconnues comme promoteur efficace, d'autre part, un terminateur de transcription. L'ensemble promoteur-séquence codante-terminateur, appelé cassette d'expression, est cloné soit dans un vecteur plasmidique monocopie ou polycopie pour la levure, soit intégré en multicopie dans le génome de la levure.

Pour une expression dans les cellules eucaryotes telles que celles des champignons filamenteux, du groupe des ascomycètes, par exemple ceux des genres Aspergillus, Neurospora, Podospora, Trichoderma ou Cryphonectria, le vecteur d'expression selon l'invention porte l'ADN recombinant défini précédemment avec les moyens nécessaires à son expression, et éventuellement un marqueur de sélection et/ou des séquences télomériques. Il est en effet possible de sélectionner les transformants ayant intégré un ADN d'intérêt à l'aide d'un marqueur de sélection situé soit sur le même vecteur que l'ADN d'intérêt, soit sur un autre vecteur, ces deux vecteurs étant alors introduits par cotransformation. L'ADN recombinant de l'invention peut être soit intégré au génome des champignons filamenteux, soit conservé sous forme extrachromosomique grâce à des séquences permettant la réplication et la partition de cet ADN.

Pour une expression dans les cellules animales, notamment dans les cellules d'ovaires de hamster chinois CHO, l'ADN recombinant est de préférence inséré dans un plasmide (par exemple dérivé du

EP 0 506 574 B1

pBR322) comportant, soit une seule unité d'expression dans laquelle est inséré l'ADN recombinant de l'invention et éventuellement un marqueur de sélection, derrière un promoteur efficace, soit deux unités d'expression. La première unité d'expression comporte l'ADN recombinant ci-dessus, précédé d'un promoteur efficace (par exemple le promoteur précoce de SV40). La séquence autour de l'ATG d'initiation est de préférence choisie en fonction de la séquence consensus décrite par Kozak (M. Kozak (1978), Cell., 15, 1109-1123). Une séquence intronique, par exemple de l'intron de l'α-globine de souris, peut être insérée en amont de l'ADN recombinant ainsi qu'une séquence comportant un site de polyadénylation, par exemple une séquence de polyadénylation du SV40, en aval du gène recombinant. La deuxième unité d'expression comporte un marqueur de sélection, par exemple une séquence d'ADN codant pour la dihydrofolate réductase (enzyme ci-après abrégée DHFR). Le plasmide est transfecté dans les cellules animales, par exemple les cellules CHO dhfr⁻ (incapables d'exprimer la DHFR). Une lignée est sélectionnée pour sa résistance au méthotréxate : elle a intégré dans son génome un nombre élevé de copies de l'ADN recombinant et exprime ce dernier à un niveau suffisant.

L'invention a également trait aux microorganismes procaryotes transformés par le vecteur d'expression défini précédemment, en particulier ceux de l'espèce Escherichia coli, ainsi qu'aux cellules eucaryotes qui contiennent, avec les moyens nécessaires à son expression, l'ADN recombinant défini précédemment. Celui-ci peut avoir été introduit par transformation par le vecteur d'expression défini précédemment ou par l'ADN recombinant de l'invention lui-même, lequel peut parfois être intégré directement au génome en un locus qui permet son expression.

Des cellules eucaryotes intéressantes sont les cellules animales.

L'ADN recombinant peut, par exemple, avoir été introduit dans ces cellules par transfection par le vecteur d'expression ci-dessus, par infection au moyen d'un virus ou d'un rétrovirus le portant, ou par microinjection. Des cellules animales préférées sont les cellules CHO, en particulier les cellules CHO dhfr⁻ à partir desquelles il est possible d'obtenir des lignées hautement productrices en la protéine de l'invention. Les cellules COS constituent également un hôte avantageux pour l'obtention de cette protéine.

D'autres cellules eucaryotes intéressantes sont les cellules de levure, en particulier de Saccharomyces cerevisiae.

L'invention concerne aussi un procédé de préparation de la protéine définie précédemment qui comprend une étape de culture des cellules animales ci-dessus, ou de cette levure, suivie de l'isolement et de la purification de la protéine recombinante.

L'invention se rapporte également à la protéine recombinante susceptible d'être obtenue par un procédé qui comprend une étape de culture de ces cellules animales, ou de cette levure, suivie de l'isolement et de la purification de la protéine recombinante.

L'invention a donc également pour objet le médicament, utile notamment en cancérologie et dans le traitement par immunomodulation de certains états infectieux et de certains états inflammatoires qui contient comme principe actif la protéine défuiie précédemment dans un excipient pharmaceutiquement acceptable. Ceux-ci peuvent être utilisés seuls ou en association avec d'autres agents actifs : par exemple une ou plusieurs autres cytokines.

L'invention sera mieux comprise à l'aide de l'exposé ci-après, divisé en sections, qui comprend des résultats expérimentaux et une discussion de ceux-ci. Certaines de ces sections concernent des expériences effectuées dans le but de réaliser l'invention, d'autres des exemples de réalisation de l'invention donnés bien sûr à titre purement illustratif.

Une grande partie de l'ensemble des techniques décrites dans ces sections, bien connues de l'homme de l'art, est exposée en détail dans l'ouvrage de Sambrook, Fritsch et Maniatis : "Molecular cloning : a Laboratory manual" publié en 1989 par les éditions Cold Spring Harbor Press à New-York (2ème édition).

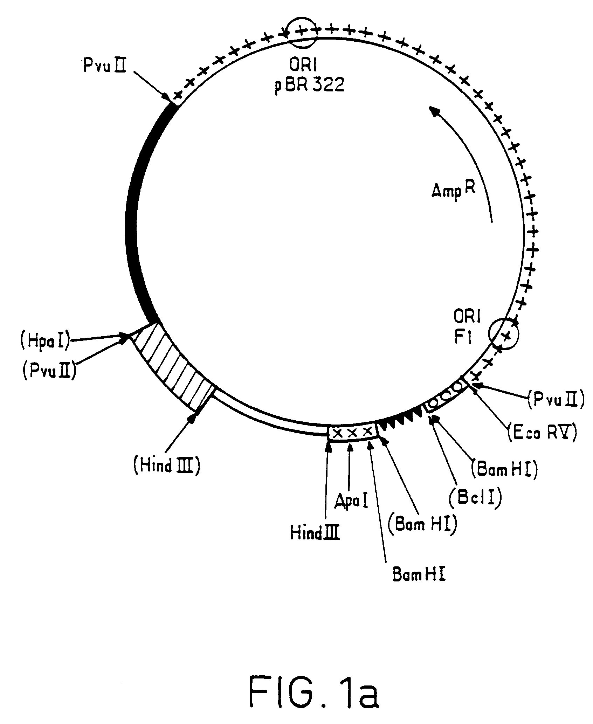

La description ci-après sera mieux comprise à l'aide des figures 1a, 1b, 1c, 2, 3, 4, 5, 6 et 7.

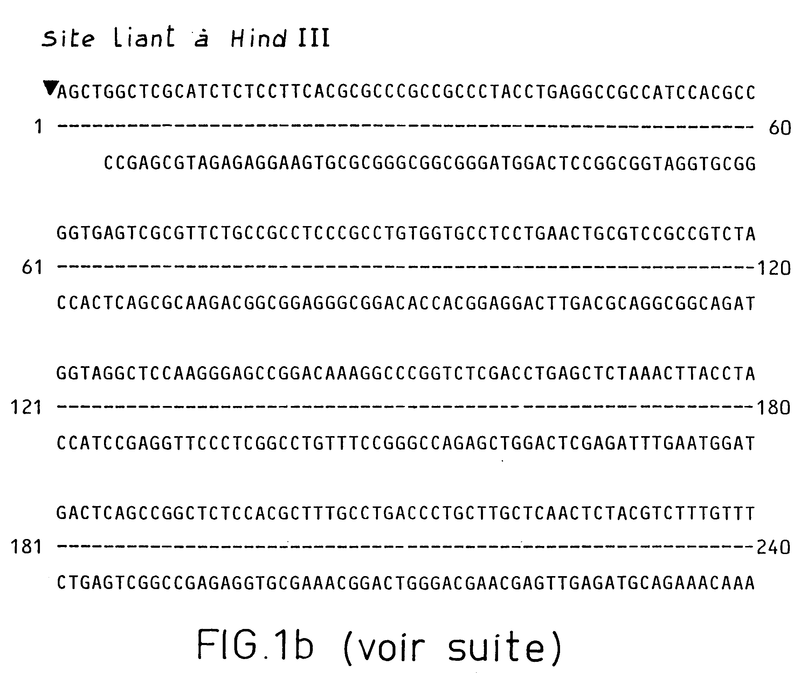

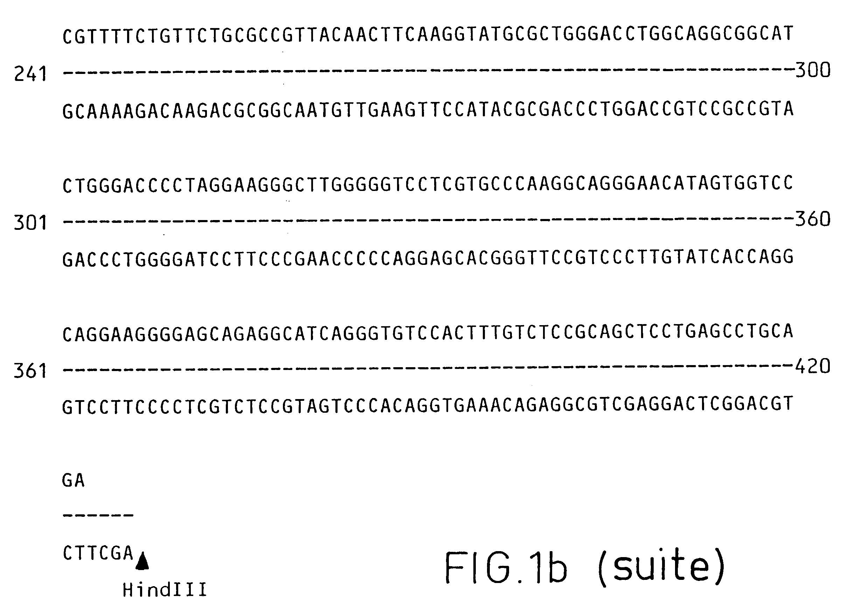

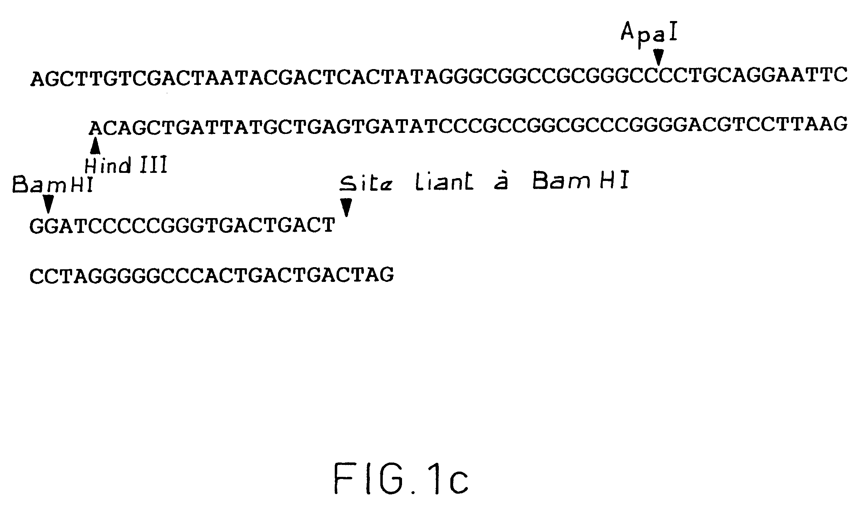

La figure 1$_a$, représente une carte d'assemblage du plasmide pSE1, plasmide de clonage dans E. coli et d'expression dans les cellules animales, les sites ayant disparu par ligation étant notés entre parenthèses. Les symboles utilisés dans cette figure seront précisés lors de la description de ce plasmide (section 2).

La figure 1$_b$ représente la séquence du fragment synthétique "site liant à HindIII" - HindIII, utilisé pour l'assemblage du plasmide pSE1.

La figure 1c représente la séquence du fragment synthétique HindIII-" site liant à BamHI".

La figure 2 représente la séquence nucléotidique de l'ADNc NC30 et en vis-à-vis la séquence d'acides aminés déduite, les deux Met susceptibles d'initier la traduction étant soulignés, les sites de clivage probables du peptide signal étant indiqué par des flèches verticales et les quatre sites possibles de N-glycosylation étant soulignés en pointillés.

9

La figure 3 et la figure 4 représentent respectivement l'alignement d'après l'homologie maximale selon la méthode de Needleman et Wunsch, 1970, J. Mol. Biol., 48, 443-453 de la séquence d'acides aminés déduite de l'ADNc NC30 (ligne supérieure) et de la séquence déduite de l'ADNc de la protéine P600 de souris (ligne inférieure) et l'alignement selon cette méthode de la partie codante de l'ADNc NC30 (ligne supérieure) et de la partie codante de l'ADNc de la protéine P600 (ligne inférieure).

La figure 5 représente la séquence du fragment B, le site de mutation silencieuse par rapport à l'ADNc NC30 étant indiqué par un astérisque.

Les figures 6 et 7 représentent la variation de la densité optique et/ou de la densité cellulaire de la lignée B9 en fonction de la concentration en protéine NC30 purifiée issue du surnageant de cellules COS (figure 6) et issue de levure (figure 7).

**SECTION 1** : Culture et stimulation à l'aide de PMA et PHA-P des cellules mononucléaires du sang périphérique. Préparation de l'ARN messager utilisé pour faire la banque d'ADN complémentaire

1) Culture et stimulation des cellules mononucléaires du sang périphérique :

A partir de cellules de sang périphérique (prélevées sur trois volontaires sains dans un centre de transfusion sanguine), ayant préalablement subi une cytaphérèse et été purifiées sur un gradient de Ficoll (Pharmacia, Boyum A., 1968, Scand. J. Clin. Lab. Invest., 21, p. 77-89) on prélève une fraction de cellules enrichie en cellules mononucléaires du sang périphérique PBMNC (peripheral blood mononuclear cells) de composition approximative suivante : 70 % de lymphocytes, 25 % de monocytes et 5 % de granulocytes (comptage des cellules à l'aide du compteur de cellules Coulter-Modèle S-Plus IV)

Les cellules sont recueillies dans un flacon de 250 ml, puis centrifugées à 300 g pendant 10 min à 37°C. Le surnageant est éliminé et le culot de cellules est rincé avec 50 ml de milieu à base de glucose, sels minéraux, acides aminés et vitamines, appelé milieu RPMI (milieu RPMI 1640 de Gibco BRL), puis de nouveau centrifugé dans les mêmes conditions.

Le culot de cellules est alors repris avec 500 ml du milieu RPMI complété avec 10 % de sérum de veau foetal (Gibco BRL- réf. 013-06290H), additionné de 10 unités de pénicilline et de 10 $\mu$g de streptomycine (solution de pénicilline/streptomycine de Gibco réf. 043-05140D) par ml de milieu ainsi que de L-glutamine (Gibco BRL-réf. 043-05030D) jusqu'à 2mM final.

Une partie de la suspension cellulaire est répartie en vue de la séparation des cellules adhérentes et des cellules non adhérentes, à raison d'environ 100 ml par boîte, dans quatre grandes boîtes de culture carrées (245 x 245 x 20 mm-Nunc - réf. 166508) et incubée pendant 1 h à 37°C. On sait en effet que la plupart des cellules monocytaires adhérent à la boîte de culture tandis que la plupart des cellules lymphocytaires restent en suspension.

Les cellules non adhérentes sont aspirées à l'aide d'une pipette et cultivées dans des flacons de culture du type Falcon de surface 175 cm$^2$ en présence de milieu RPMI complété comme décrit ci-dessus, additionné de 10 ng/ml de phorbol myristate-2 acétate-3 (PMA) (Sigma-réf. P8139) et de 5 $\mu$g/ml de phytohémaglutinine (PHA-P) (Sigma-réf. L8754), à 37°C en présence de 5 % de $CO_2$ pendant 24 h.

Sur les cellules adhérentes on rajoute 100 ml de milieu RPMI complété comme décrit ci-dessus additionné de 10 ng/ml de PMA et de 5 $\mu$g/ml de PHA-P. Les cellules sont incubées à 37°C en présence de 5 % (v/v) de $CO_2$ pendant 5 h.

Le reste de la suspension cellulaire, appelé ci-après les cellules totales, est réparti dans 4 grandes boîtes de culture carrées et incubé en présence de milieu RPMI complété comme décrit ci-dessus, additionné de 10 ng/ml de PMA et 5 $\mu$g/ml de PHA-P à 37°C en présence de 5 % (v/v) de $CO_2$ pendant 5 h pour les deux premières boîtes et 24 h pour les deux autres.

Environ 2 h avant la fin de l'incubation on ajoute dans le milieu de culture de ces différentes cellules 10 $\mu$g/ml de cycloheximide (Sigma réf. C6255) (inhibiteur de traduction qui augmente la stabilité des ARN des cytokines : cf. Lindsten et al, 1989, Science, 244, 339-344) et l'incubation est poursuivie pendant 2 h à 37°C.

2) Préparation de l'ARN messager :

a) Extraction de l'ARN messager

Les cellules sont récupérées de la façon suivante :
- les cellules adhérentes sont lavées 2 fois avec du tampon PBS (Phosphate buffered saline réf. 04104040-Gibco BRL) puis grattées avec un grattoir en caoutchouc et centrifugées. On obtient ainsi un culot cellulaire, appelé culot A.

- pour les cellules non adhérentes, après agitation du flacon contenant la suspension des cellules, la suspension cellulaire est prélevée et centrifugée. On obtient ainsi un culot cellulaire, appelé culot cellulaire NA.

- pour les cellules totales, la fraction adhérente est lavée 2 fois avec du tampon PBS et grattée comme précédemment puis centrifugée. La fraction non adhérente est centrifugée. Les deux culots cellulaires obtenus seront réunis par la suite. Leur réunion est appelée culot cellulaire T(5 h) pour les cellules totales incubées pendant 5 h et T(24 h) pour les cellules totales incubées pendant 24 h.

Les culots cellulaires A, NA, T(5 h) et T(24 h) sont congelés et conservés à -80°C.

Chaque culot cellulaire congelé est mis en suspension dans le tampon de lyse de composition suivante : guanidine- thiocyanate 5M ; Tris-(hydroxy-méthyl)-aminométhane 50mM pH 7,5 EDTA 10mM. La suspension est broyée à l'aide d'un broyeur Ultra Turax n° 231 256 (Janke et Kunkel) à puissance maximale pendant 4 cycles de 20 s. On ajoute du $\beta$-mercaptoéthanol jusqu'à 0,2M et on refait un cycle de 30 s. On ajoute du chlorure de lithium jusqu'à 3M. On refroidit la suspension à 4°C et on la laisse reposer à cette température pendant 48 h. L'ARN est ensuite isolé par centrifugation pendant 60 min. Le culot d'ARN est lavé une fois avec une solution de chlorure de lithium 3M, recentrifugé, puis repris dans un tampon de composition suivante : SDS 1 %, EDTA 5mM et Tris HCl 10mM pH 7,5, additionné de 1 mg/ml de protéinase K (Boehringer Mannheim, GmbH). Après incubation à 40°C pendant 1 h, la solution d'ARN est extraite avec un mélange phénol/chloroforme. On précipite à -20°C l'ARN contenu dans la phase aqueuse à l'aide d'une solution d'acétate d'ammonium 0,3M final et 2,5 volumes d'éthanol. On centrifuge à 15 000 g pendant 30 min et on garde le culot.

b) Purification de la fraction poly A+ de l'ARN

Le culot est repris dans 1 ml de tampon de composition Tris-HCl 10mM pH 7,5 EDTA 1mM, appelé tampon TE et mis en suspension par agitation au vortex. L'oligo dT-cellulose type 3 (commercialisé par Collaborative Research Inc, Biomedicals Product Division) est préparé suivant les recommandations du fabricant. L'ARN est déposé sur l'oligo dT-cellulose, agité doucement pour mettre en suspension les billes, puis chauffé pendant 1 min à 65°C.

La suspension est ajustée à 0,5 M NaCl, puis mise à agiter doucement pendant 10 min. La suspension est alors centrifugée pendant 1 min à 1000 g, le surnageant est éliminé, le culot est lavé 2 fois avec 1 ml de tampon TE contenant 0,5 M NaCl. Les surnageants sont éliminés. L'élution de la fraction polyadénylée des ARN (constituée des ARN messagers) est obtenue par suspension des billes dans 1 ml de tampon TE, puis chauffage de cette suspension à 60°C pendant 1 min, suivie d'une agitation pendant 10 min sur plaque basculante. On centrifuge ensuite pendant 1 min à 1000 g, ce qui permet de récupérer le surnageant contenant des ARN messagers libres en solution. L'ensemble des opérations ci-dessus (à partir de l'élution) est répété 2 fois. Les surnageants ainsi obtenus sont rassemblés, on élimine les billes résiduelles par centrifugation et on précipite le surnageant avec 3 volumes d'éthanol et une solution de NaCl à 0,3M final.

On obtient ainsi à partir des culots cellulaires A, NA, T(5 h) et T(24 h), quatre échantillons d'ARN-poly A+, nommés par la suite ARN-poly A+-A, ARN-poly A+-NA; ARN-polyA+-T(5 h) et ARN-poly A+-T(24 h).

**SECTION 2** : Préparation d'une banque d'ADN complémentaire enrichie en séquences spécifiques des cellules mononucléaires du sang périphérique

1) Construction du vecteur de clonage pSE1 :

La stratégie mise en oeuvre fait appel à des fragments obtenus à partir de plasmides préexistants accessibles au public et à des fragments préparés par voie de synthèse selon les techniques maintenant couramment utilisées. Les techniques de clonage employées sont celles décrites par Sambrook et al, dans "Molecular Cloning, a Laboratory manual" (Cold Spring Harbor Laboratory, 1989). La synthèse des oligonucléotides est réalisée à l'aide d'un synthétiseur d'ADN Biosearch 8700.

La description ci-après sera mieux comprise en référence à la figure 1$_a$.

Ce plasmide a été construit par ligations successives des éléments suivants :

a)- un fragment PvuII-PvuII - symbolisé par + + + + + + + sur la figure 1$_a$ - de 2525pb, obtenu par digestion complète du plasmide pTZ18R (Pharmacia) à l'aide de l'enzyme de restriction PvuII. Ce fragment contient l'origine de réplication du phage f1 (notée ORI F1 sur la figure 1$_a$), un gène (noté Amp$^R$ sur la figure 1$_a$) portant la résistance à l'ampicilline et l'origine de réplication (notée ORI pBR322

sur la figure 1$_a$) permettant la réplication de ce plasmide dans E. coli. Le premier site franc PvuII disparaît par ligation avec le site franc EcoRV (qui disparaît également) du fragment décrit en g).

b)- un fragment PvuII-HpaI - symbolisé par ▬ sur la figure 1$_a$ - de 1060 pb de l'ADN d'adénovirus type 5 entre les positions 11299 (site de restriction PvuII) et 10239 (site de restriction HpaI) (Dekker et Van Ormondt, Gene, 27, 1984, 115-120) contenant l'information pour les ARN VA-I et VA-II. Le site franc HpaI disparaît par ligation avec le site franc PvuII (qui disparaît également) du fragment décrit en c). Le site ApaI en position 11 218 a été enlevé par clivage à l'aide de l'enzyme ApaI, traitement à l'aide de l'exonucléase : ADN polymérase du phageT4 et religation.

c)- un fragment PvuII-HindIII - symbolisé par //////// sur la figure 1$_a$ - de 344 pb, issu de l'ADN du virus SV40 obtenu par digestion complète à l'aide des enzymes de restriction PvuII et HindIII. Ce fragment comporte l'origine de réplication et le promoteur précoce de l'ADN du virus SV40 (réf. B.J. Byrne et al., Proc. Ntl. Acad. Sci. USA (1983), 80, 721-725).

Le site HindIII disparaît par ligation avec le site liant à HindIII du fragment décrit en d).

d)- un fragment synthétique "site liant à HindIII" - HindIII - symbolisé par ══ sur la figure 1$_a$ - de 419 pb dont la séquence, donnée sur la figure 1$_b$, contient une séquence proche de la séquence 5' non traduite du virus HTLV1 (R. Weiss et al, "Molecular Biology of Tumor Viruses - part 2-2$^e$ ed - 1985 - Cold Spring Harbor Laboratory - p. 1057) et l'intron distal du gène de l'α-globine de souris (Y. Nishioka et al, 1979, Cell, 18, 875-882).

e)- un fragment synthétique HindIII-"site liant à BamHI" - symbolisé par XXXXXXX sur la figure 1$_a$ - contenant le promoteur de l'ARN-polymérase du phage T7 ainsi qu'un polylinker contenant notamment les sites de clonage ApaI et BamHI, dont la séquence est représentée sur la figure 1c.

f)- un fragment BamHI-BclI de 240 pb - représenté par ▲▲▲ sur la figure 1$_a$ -, petit fragment obtenu par digestion complète à l'aide des enzymes BclI et BamHI du virus SV40 qui contient le site de polyadénylation tardif de ce dernier. (M. Fitzgerald et al., Cell, 24, 1981, 251-260). Les sites BamHI et BclI disparaissent par ligations avec respectivement le "site liant à BamHI" du fragment décrit en e) et le site BamHI (qui disparaît également) du fragment décrit en g).

g)- un fragment BamHI-EcoRV - symbolisé par OOOOOO sur la figure 1$_a$ -de 190 pb, petit fragment issu du plasmide pBR322 après digestion complète à l'aide des enzymes EcoRV et BamHI.

Le plasmide pSE1 comporte donc les éléments nécessaires pour son utilisation comme vecteur de clonage dans E. coli (origine de replication dans E. coli et gène de résistance à l'ampicilline, provenant du plasmide pTZ18R) ainsi que comme vecteur d'expression dans les cellules animales (promoteur, intron, site de polyadénylation, origine de replication du virus SV40), et pour sa copie en simple brin dans un but de séquençage (origine de réplication du phage f1).

2) Constitution d'une banque d'ADN complémentaire enrichie en séquences spécifiques des cellules mononucléaires du sang périphérique :

La technique de clonage utilisée est celle décrite par Caput et al, (technique de l'amorce-adaptateur (Primer-adapter) : Caput et al, Proc. Natl. Acad. Sci. U.S.A., 1986, 83, 1670-1674.

Elle consiste d'une part à digérer le vecteur pSE1 par ApaI, ajouter une queue de polydC sur l'extrémité 3' protubérante, puis à digérer les plasmides ainsi obtenus par l'endonucléase BamHI. Le fragment correspondant au vecteur est purifié sur colonne de Sépharose CL4B (Pharmacia). Il comprend donc une queue polydC à une extrémité, l'autre extrémité étant cohésive, du type BamHI.

D'autre part, les ARN polyA$^+$ obtenus à l'issue de la section 1 sont soumis à la transcription inverse à partir d'une amorce dont la séquence est la suivante

5'<GATCCGGGCC CTTTTTTTTT TTT<3'

Ainsi, les ADNc présentent à leur extrémité 5' la séquence GATCC complémentaire de l'extrémité cohésive BamHI.

Les hybrides ARN-ADN obtenus par action de la transcriptase inverse sont soumis à une hydrolyse alcaline qui permet de se débarrasser de l'ARN. Les ADNc simple brin sont alors soumis à un traitement à la terminale transférase, de façon à ajouter des polydG en 3', et purifiés par 2 cycles sur colonne de sépharose CL4B.

Ces ADNc sont hybridés avec de l'ARN-polyA$^+$ provenant de cellules de la lignée COS3 (lignée de cellules de reins de singe exprimant l'antigène T du virus SV40 : cf. Y. Gluzman, 1981, Cell, 23, 175-182), préparées comme décrit dans la section 1 2).

Les ADNc non hybridés sont isolés (fraction enrichie en ADN complémentaire aux ARN messagers spécifiques des cellules mononucléaires du sang périphérique).

Ces ADNc sont insérés sous forme simple brin dans le vecteur pSE1. Un second oligonucléotide (l'adaptateur) complémentaire de l'amorce est nécessaire pour générer un site BamHI à l'extrémité 5' des ADNc. Après hybridation du vecteur, de l'ADNc et de l'adaptateur, les molécules recombinantes sont circularisées par l'action de la ligase du phage T4. les régions simple brin sont alors réparées grâce à l'ADN polymérase du phage T4. Le pool de plasmides ainsi obtenu sert à transformer la souche E. coli MC 1061 (Casabadan et S. Cohen, J. Bact. (1980), 143, 971-980) par électroporation.

Protocole de préparation de la banque d'ADN complémentaire

a) Préparation de l'ADN complémentaire

A partir de 5 $\mu$g des ARN-poly A$^+$ de cellules mononucléaires de sang périphérique obtenus à l'issue de la section 1 de composition suivante : ARN-poly A$^+$ A : 0,5 $\mu$g, ARN-poly A$^+$ NA : 2 $\mu$g, ARN-poly A$^+$ T- (5 h) : 2 $\mu$g et ARN-poly A$^+$ T(24 h) : 0,5 $\mu$g, on prépare l'ADN complémentaire simple-brin marqué au $^{32}$P-dCTP (l'ADN complémentaire obtenu présente une activité spécifique de 3000 dpm/ng) avec l'amorce synthétique de séquence suivante (comprenant un site BamHI) :

5' < GATCCGGGCC CTTTTTTTTT TTT < 3'

dans un volume de 100 $\mu$l de tampon de composition : Tris HCl 50mM pH8,3, MgCl$_2$ 5mM, DTT 10mM, contenant 0,5 mM de chacun des désoxynucléotides trisphosphates, 100 $\mu$Ci de dCTP $\alpha^{32}$P et 100 U de RNasin (Promega). Après 30 min d'incubation à 46°C avec 100 unités de l'enzyme transcriptase inverse (Genofit-E1 022) on ajoute 4 ml d'EDTA 0,5M. On extrait une première fois avec du phénol (saturé en tampon TE) puis une seconde fois avec du chloroforme. On ajoute 10 $\mu$g d'ARN de transfert de foie de veau, 1/10 de volume d'une solution d'acétate d'ammonium 10M et 2,5 volumes d'éthanol pour précipiter l'ADN complémentaire. On centrifuge, on dissout le culot dans 30 $\mu$l de tampon TE puis on retire les petites molécules telles que sels, phénol et chloroforme par chromatographie d'exclusion sur une colonne de polyacrylamide P10 (Biogel P10-200-400mesh, réf. 1501050-Biorad).

b) Hydrolyse alcaline de la matrice ARN

On ajoute 4,6 $\mu$l d'une solution de NaOH 2N, on incube pendant 30 min à 68°C, puis on ajoute 4,6 $\mu$l d'acide acétique 2N et on fait passer la solution obtenue sur une colonne de polyacrylamide P10.

c) Addition homopolymérique de dG

On allonge l'ADN complémentaire en 3' avec une "queue" de dG avec 66 unités de l'enzyme terminale transférase (Pharmacia 27073001). On incube dans 60 $\mu$l de tampon de composition : Tris HCl 30mM pH7,6 ; chlorure de cobalt 1mM, acide cacodylique 140mM, DTT 0,1mM, dGTP 1 mM, pendant 30 min à 37°C, puis on ajoute 4 $\mu$l d'EDTA 0,5M.

d) Purification sur colonne de sépharose CL4B

Afin d'éliminer l'amorce synthétique, on purifie l'ADN complémentaire sur deux colonnes successives de 1 ml de sépharose CL4B (Pharmacia), équilibrées avec une solution NaOH 30mM/EDTA 2mM.

Les trois premières fractions radioactives (d'environ 80 $\mu$l chacune) sont regroupées et précipitées avec 1/10 de volume d'une solution d'acétate d'ammonium 10 M et 2,5 volumes d'éthanol. La quantité d'ADN complémentaire est de 1 $\mu$g.

e) Hybridation

Le culot d'ADN complémentaire est mis en suspension dans 25 $\mu$l de tampon TE, on ajoute 15 $\mu$g d'ARN-polyA$^+$ extrait de cellules de la lignée COS, puis 1/10$^{ème}$ de volume d'une solution de NaCl 3M, 2,5 volumes d'éthanol et on laisse précipiter à -20°C.

On centrifuge, on lave à l'éthanol 70 %, on sèche, on dissout le culot dans 5 $\mu$l de tampon de composition suivante : Tris-HCl 0,1M pH 7,5 ; NaCl 0,3M, EDTA 1mM, on met la solution obtenue dans un tube capillaire que l'on scelle, puis on incube à 65°C pendant 40 h.

On dilue le contenu du capillaire dans 100 $\mu$l de tampon TE auquel on ajoute 300 $\mu$l de tampon phosphate de sodium 50mM pH6,8. On fait passer la solution obtenue sur une colonne d'hydroxyapatite (Biorad réf. 130.0520) à 60°C, équilibrée avec ce tampon phosphate. On sépare le simple brin (l'ADN complémentaire non-hybridé) et le double brin (ARN messager de COS hybridé à l'ADN complémentaire) par un gradient de tampon phosphate de 0,1M à 0,2M à travers la colonne d'hydroxyapatite. On regroupe les fractions correspondant à l'ADN complémentaire simple brin (25 % en poids de l'ADNc élué, ce qui correspond à un enrichissement d'environ 4 fois en séquences spécifiques de cellules mononucléaires du sang périphérique), on ajoute 20 $\mu$g d'ARN de transfert, on précipite le volume total avec $1/10^{\text{ème}}$ de volume d'une solution d'acétate d'ammonium 10M et 2,5 volumes d'éthanol. On centrifuge, le culot est dissout dans 200 $\mu$l de TE, on retire le phosphate résiduel sur polyacrylamide P10, on précipite de nouveau avec $1/10^{\text{ème}}$ de volume d'une solution d'acétate d'ammonium 10M et 2,5 volumes d'éthanol.

Le culot est dissout dans 30 $\mu$l d'une solution de NaOH 30mM ; EDTA 2mM. On charge l'ADN complémentaire sur une colonne de sépharose CL4B (Pharmacia) de 1 ml, équilibrée avec une solution de NaOH 30mM ; EDTA 2mM, afin d'éliminer le reste d'amorce synthétique. On regroupe les 3 premières fractions radioactives d'environ 80 $\mu$l chacune. On précipite l'ADNc contenu dans ces fractions avec $1/10^{\text{ème}}$ de volume d'une solution d'acétate d'ammonium 10 M et 2,5 volume d'éthanol. La quantité d'ADN complémentaire ainsi récupérée est de 20 ng.

f) Appariement du vecteur de clonage PSE1 et de l'ADN complémentaire en présence de l'adaptateur

On centrifuge, le culot est dissout dans 33 $\mu$l de tampon TE, on ajoute 5 $\mu$l (125 ng) de vecteur de clonage pSE1, 1 $\mu$l (120 ng) de l'adaptateur de séquence suivante (comprenant un site ApaI),

5' AAAAAAAAAA AAAGGGCCCG 3'

10 $\mu$l d'une solution de NaCl 200mM, on incube pendant 5 min à 65°C puis on laisse refroidir le mélange réactionnel jusqu'à température ambiante.

g) Ligation

On ligue le vecteur de clonage et l'ADNc simple brin dans un volume de 100 $\mu$l avec 32,5 unités de l'enzyme ADN ligase du phage T4 (Pharmacia ref : 270 87002) pendant une nuit à 15°C dans un tampon de composition : Tris HCl 50mM pH7,5, MgCl$_2$ 10mM, ATP 1mM.

h) Synthèse du deuxième brin de l'ADNc

On élimine les protéines par extraction au phénol suivie d'une extraction au chloroforme, puis on ajoute $1/10^{\text{ème}}$ de volume d'une solution d'acétate d'ammonium 10 mM, puis 2,5 volumes d'éthanol. On centrifuge, le culot est dissous dans le tampon de composition Tris acétate pH 7,9 33 mM, acétate de potassium 62,5 mM, acétate de magnésium 1 mM et dithiothréitol (DTT) 1 mM, le deuxième brin d'ADN complémentaire est synthétisé dans un volume de 30 $\mu$l avec 30 unités de l'enzyme ADN polymérase du phage T4 (Pharmacia, réf. 27-0718) et un mélange de 1mM des quatre désoxynucléotides triphosphates de dATP, dCTP, dGTP et dTTP, ainsi que deux unités de la protéine du gène 32 du phage T4 (Pharmacia - réf. 27-0213), pendant 1 h à 37°C. On extrait au phénol et on retire les traces de phénol par une colonne de polyacrylamide P10 (Biogel P10-200-400 mesh- Réf 15011050 - Biorad).

i) Transformation par électroporation

On transforme des cellules E. coli MC1061 (Clontech) avec l'ADN recombinant obtenu précédemment par électroporation à l'aide de l'appareil Biorad Gene Pulser (Biorad) utilisé à 2,5 kV dans les conditions prescrites par le fabricant, puis on fait pousser les bactéries pendant 1 heure dans du milieu dit milieu LB (Sambrook, op cité) de composition : bactotryptone 10 g/l ; extrait de levure 5 g/l ; NaCl 10 g/l, puis pendant 6 h 30 dans le milieu LB additionné de 100 $\mu$g/ml d'ampicilline.

On détermine le nombre de clones indépendants en étalant une dilution au $1/1000^{\text{ème}}$ de la transformation après la 1ère heure d'incabution sur une boîte de milieu LB additionné de 1,5 % d'agar (p/v) et de 100 $\mu$g/ml d'ampicilline, appelé par la suite milieu LB gélosé. Le nombre de clones indépendants est de 500 000.

**SECTION 3** : Criblage de la banque d'ADN complémentaire soustraite et sélection du clone NC30

1) Réalisation des répliques des colonies bactériennes de la banque d'ADNc sur filtre de nylon :

On distribue environ 40 000 bactéries recombinantes de la banque d'ADNc sur des boîtes de Pétri de (245 x 245 mm) contenant du milieu LB gélosé (environ 2000 colonies/boîte).

A partir de chacune de ces boîtes, on réalise un transfert des colonies sur une membrane de nylon (Hybond N-Amersham) par dépôt de la membrane sur la surface de la boîte et mise de repères par perçage de la membrane à l'aide d'une aiguille. La membrane est ensuite retirée et déposée sur la surface d'une nouvelle boîte de Pétri contenant du milieu LB gélosé. On laisse pendant quelques heures à 37°C pour obtenir la repousse des colonies. A partir de cette première membrane on réalise quatre répliques sur de nouvelles membranes (préalablement déposées sur du milieu LB gélosé pour les humidifier) par contacts successifs avec la première membrane. Les répliques sur membrane obtenues sont finalement déposées sur des boîtes de milieu LB gélosé et mises à incuber pendant une nuit à 30°C.

Les répliques sur membrane sont déposées avec les colonies vers le haut, sur une feuille de Whatman 3MM saturée avec une solution de composition : NaOH 0,5M ; NaCl 1,5M, pendant 5 min, ce qui permet de lyser les bactéries et de fixer l'ADN. Les répliques sur membrane sont ensuite posées sur une deuxième feuille de Whatman 3MM ,saturée cette fois avec une solution neutralisante de composition : NaCl 1,5M ; Tris-HCl pH8, 0,5M pendant 5 min. Les répliques sur membrane sont ensuite plongées dans une solution de 2 x SSC (composition de la solution SSC : NaCl 0,15M ; citrate de sodium 0,015M) et les débris bactériens sont partiellement éliminés en frottant doucement à l'aide d'ouate de nettoyage.

On traite ensuite les répliques sur membrane avec de la protéinase K (Boehringer Mannheim GmbH) à 100 μg/ml dans une solution de composition : Tris-HCl 10mM pH8 ; EDTA 10mM ; NaCl 50mM ; SDS 0,1 % à raison de 20 ml par membrane. On incube pendant 30 min à 37°C avec agitation. Les répliques sur membrane sont de nouveau plongées dans une solution de 2 x SSC pour éliminer définitivement toute trace de débris bactériens. Elles sont finalement mises à sécher sur du papier filtre pendant quelques minutes puis pendant 30 min sous vide à + 80°C. On obtient ainsi, pour chaque boîte, quatre répliques sur membrane, appelées par la suite réplique 1, réplique 2, réplique 3 et réplique 4.

2) Préparation de l'ARN utilisé pour la fabrication des sondes ADNc :

a) Culture et stimulation des cellules mononucléaires du sang périphérique à l'aide de PMA, (PMA et anti-CD28), (PMA, PHA-P et cyclosporine A) ou (PMA et PHA-P) :

Les cellules non adhérentes sont préparées comme décrit précédemment (section 1,1). Elles sont cultivées pendant 5 h en flacons de type Falcon de surface 175 cm$^2$ en présence du milieu RPMI complété avec 10 % de sérum de veau foetal (Gibco BRL-Réf. 013-06290H), additionné de 10 unités de pénicilline et de 10 μg de streptomycine (solution de pénicilline/streptomycine de Gibco-Réf. 043-05140D) par ml de milieu ainsi que de L-glutamine (Gibco BRL-Réf. 043-05030D) jusqu'à 2 mM final. Les cellules sont stimulées pendant 5 h dans l'une des conditions de stimulation 1) 2) 3) et 4) suivantes :
    1) en présence de 10 ng/ml de phorbol myristate-2 acétate-3 (PMA) (Sigma, réf. P8139)
    2) en présence de 10 ng/ml de PMA et de 1 μg/ml de l'anticorps monoclonal anti-CD28 (Réactifs et Systèmes S.A. réf. ACM0280NC050)
    3) en présence de 10 ng/ml de PMA, de 5 μg/ml de phytohémaglutinine (PHA-P) (Sigma, réf. L8754) et de 1 μg/ml de cyclosporineA (Sandoz)
    4) en présence de 10 ng/ml de PMA et de 5 μg/ml de PHA-P
    Il est en effet connu que l'addition de l'anticorps anti-CD28 augmente la quantité des ARN messagers de plusieurs cytokines, notamment l'IL-2, l'IFNγ le TNFα et le GMCSF par une augmentation de la stabilité de leurs ARN messagers (Lindsten T. et al, (1989), Science, 244, 339) dans les lymphocytes T, et que l'immunosuppresseur cyclosporineA inhibe l'augmentation des ARN messagers des cytokines induites par l'activation avec PMA et PHA-P dans les lymphocytes T (Thompson C.B. et al, 1989, Proc. Natl. Acad. Sci. USA, 86, 1333-1337).

Les cellules sont recueillies dans les conditions de stimulation ci-dessus et on retient les culots cellulaires, appelés respectivement, culot cellulaire 1, culot cellulaire 2, culot cellulaire 3 et culot cellulaire 4.

b) Préparation de l'ARN poly A$^+$

A partir des culots cellulaires ci-dessus, l'ARN est extrait et la fraction polyA$^+$ est purifiée comme décrit dans la section 1-2) a) et b). On obtient ainsi quatre fractions ARN-polyA$^+$, appelées respectivement fraction polyA$^+$1, fraction polyA$^+$2, fraction polyA$^+$3 et fraction polyA$^+$4.

3) Préparation des sondes ADNc radiomarquées :

Les sondes ADNc radiomarquées, appelées respectivement sonde 1, sonde 2, sonde 3 et sonde 4, sont synthétisées à partir des quatre fractions ARN-polyA$^+$ ci-dessus, préparées comme décrit ci-après.

1 $\mu$g d'ARN polyA$^+$ est hybridé avec 200 ng d'oligo dT$_{12-18}$ (Pharmacia) dans 2 à 3 $\mu$l de tampon de composition Tris-HCl pH 7,5 50 mM et EDTA 1 mM, par incubation pendant 2 min à 65°C et refroidissement jusqu'à température ambiante. La synthèse de l'ADNc radiomarqué est réalisée dans un volume réactionnel de 10 $\mu$l en tampon de composition : Tris-HCl 50mM pH8,3 ; MgCl$_2$ 5mM ; dithiothreitol 10mM, contenant 500 $\mu$M de chacun des 3 désoxynucléotides triphosphates dATP, dGTP et dTTP (Pharmacia), 10 $\mu$M de dCTP et 150 $\mu$Ci de dCTP $\alpha$32P (3000 Ci/ mmol-Amersham) et 40 unités de RNasine (inhibiteur de RNAse-Genofit). La réaction s'effectue à 46°C pendant 30 min en présence de 10 à 20 unités de transcriptase inverse (Genofit) . Cette synthèse est suivie de l'hydrolyse alcaline de l'ARN par une solution de NaOH 0,3M dans un volume final de 20 $\mu$l pendant 30 min à 65°C. On neutralise par ajout d'acide acétique 3M. On ajuste le volume à 50 $\mu$l avec du milieu TE. On fait une extraction avec un même volume de phénol suivie d'une deuxième extraction avec un même volume d'un mélange chloroforme/ isoamylalcool (dans les proportions respectives 24/1). On élimine le dCTP $\alpha$32P non incorporé lors de la synthèse du brin ADNc par chromatographie d'exclusion sur une colonne de polyacrylamide P10 (Biogel-200-400 mesh-Biorad).

La quantité d'ADNc est de 60 à 100 ng possédant une activité spécifique de 1 x 10$^9$ dpm/$\mu$g.

4) Hybridation des répliques des colonies bactériennes avec les sondes ADNc :

Les répliques sur membrane sont préhybridées pendant 2 h à 42°C dans un tampon de composition : 50 % formamide ; 6 x SSC ; 5 x solution de Denhardt ; 0,1 % SDS et 100 $\mu$g/ml d'ADN de sperme de saumon soniqué, rajouté après dénaturation pendant 10 min à 100°C. Les répliques sur membrane sont hybridées pendant deux jours : la réplique 1 avec la sonde 1, la réplique 2 avec la sonde 2, la réplique 3 avec la sonde 3 et la réplique 4 avec la sonde 4, ces sondes étant utilisées à une concentration de 4 ng/ml dans le tampon ci-dessus. La 5 x solution de Denhardt (cf. Sambrook , op. cité) a la composition : Ficoll (type 400-Pharmacia) 1 g/l, polyvinylpyrrolidone 1 g/l et la sérumalbumine bovine (BSA) 1 g/l.

Les préhybridation et hybridation s'effectuent en tubes dans un four à hybrider (Hybaid) avec respectivement 25 ml et 10 ml de tampon par membrane.

Ensuite les répliques sur membrane sont successivement lavées plusieurs fois pendant 15 min à 20°C dans le tampon de composition 2 x SSC ; 0,1 % SDS, puis deux fois pendant 15 min dans une solution 0,1 x SSC, 0,1 % SDS à 55°C, séchées sur papier Whatman 3MM et autoradiographiées sur films Kodak XAR5.

5) Hybridation avec un mélange d'oligonucléotides correspondant à la plupart des cytokines connues :

Pour identifier les clones qui contiennent les ADN complémentaires aux ARN messagers des cytokines connues, une autre série de répliques sur membrane, préparées comme décrit ci-dessus, est hybridée avec un mélange - appelé mélange C - de 28 oligonucléotides comportant chacun 20 nucléotides, correspondant aux ADN complémentaires des cytokines suivantes :
Interleukine-1 $\alpha$(Furutani Y. et al, 1985, Nucl. Ac. Res., 13, 5869-5882), Interleukine-1 $\beta$(Auron P. et al, 1984, Proc. Natl. Acad. Sci. USA, 81, 7907-7911), Interleukine-2 (Degrave W. et al, 1983, EMBO J., 2, 3249-3253), Interleukine-3 (Yang Y.C. et al, Cell, 1986, 47, 3-10), Interleukine-4 (Yohoto T. et al, 1986, Proc. Ntl. Acad. Sci., 83, 5894-5898), Interleukine-5 (Hirano T. et al, 1986, Nature, 324, 73-75), Interleukine- 6 (May L. et al, 1986, Proc. Natl. Acad. Sci. USA, 83, 8957-8961), Interleukine-7 (Namen A. et al, 1988, Nature, 333, 571-573), Interleukine-8 (Matsushima K. et al, 1988, J. Exp. Med., 167, 1883-1893), Interleukine-9 (Yang Y.C. et al, 1989, Blood, 74, 1880-1884), TNF$\alpha$ (Pennica D. et al, 1984, Nature, 312, 724-729), TNF$\beta$ (Gray P. et al, 1984, Nature, 312, 721-724), GCSF (Nagata S. et al, Nature, 1986, 319, 415-418), MCSF (Kawasaki E. et al, 1985, Science, 230, 291-296), GMCSF (Wong G. et al, 1985, Science, 228, 810-815), LIF (Gough N. et al, 1988, Proc. Natl. Acad. Sci. USA, 85, 2623-2627), Interféron al, 1981, Nature, 290, 20-26), Interféron $\beta$1

(Taniguchi T. et al, 1980, Gene, 10, 11-15), Interféron γ (Gray P. et al, 1982, Nature, 295, 503-508), TGFα-(Derynck R. et al, 1984, Cell, 38, 287-297), TGFβ1 (Derynck R. et al, 1985, Nature, 316, 701-705), bFGF (Prats H. et al, 1989, Proc. Natl. Acad. Sci. USA, 86, 1836-1840), Erythropoïétine (Jacobs K. et al, 1985, Nature, 313, 806-810), BCGF (Sharma S. et al, 1987, Science, 235, 1489-1492), MIF (Weiser W. et al, 1989, Proc. Natl. Acad. Sci USA, 86, 7522-7526), MCP-1 (Yoshimura T. et al, FEBS Lett., 244, 487-493), Oncostatine-M (Malik N. et al, 1989, Mol. Cell. Biol., 9, 2847-2853) et EDF (Murata M. et al, 1988, Proc. Natl. Acad. Sci. USA, 85, 2434-2438).

Ces oligonucléotides, fabriqués à l'aide du synthétiseur d'ADN Biosearch 8700, sont couplés avec de la peroxydase de raifort EC 1.11.17 (Boehringer Mannheim-Réf. 814-407) selon le protocole suivant :

- on fait réagir sur la colonne de synthèse les oligonucléotides avec du carbonyl-diimidazole (Aldrich - 11, 553-3) et du diamino-1,6-hexane (Aldrich - H1.169-6) selon la méthode de Wachter et al, 1986, Nucl. Ac. Res., 14, 7985-7994.
- après déprotection des bases et clivage du support par traitement ammoniacal les oligonucléotides sont purifiés sur une résine d'échange d'ions (Quiagen - Diagen-500051) avec changement du contre-ion ammonium en ion lithium.
- les 5'-amino-oligonucléotides sont couplés à la peroxydase de raifort (Boehringer Mannheim-814407) selon la méthode de M. Urdea et al, Nucl. Ac. Res., 1988, 16, 4937-4956.

Le mélange d'oligonucléotides s'hybride avec environ 10 % des clones de la banque.

Les clones donnant un signal autoradiographique plus fort avec la sonde 2 qu'avec la sonde 1 , plus fort avec la sonde 4 qu'avec la sonde 1 et plus fort avec la sonde 4 qu'avec la sonde 3 et ne s'hybridant pas avec le mélange C ont été partiellement séquencés comme décrit dans la section 4 ci-après. Deux de ces clones, appelés par la suite clones NC30 et NC30-bis, ont ainsi été retenus. Ces clones contiennent respectivement un plasmide appelé pSE1-NC30 et un plasmide appelé pSE1-NC30-bis.

**SECTION 4** : Expression de l'ARN messager du clone NC-30 dans les cellules mononucléaires du sang périphérique

Les cellules non adhérentes (constituées principalement de lymphocytes) sont préparées comme décrit dans la section 1-1) et stimulées comme décrit dans la section 3-2)-a) avec, en plus, des cellules témoins non stimulées (conditions de stimulation 0)). Les ARN messagers de ces cellules dans 5 conditions de stimulation sont préparés comme décrit dans l'exemple 1-2)-a) et analysés par électrophorèse sur gel d'agarose 1 % en présence de formaldéhyde (Sambrook, op cité), suivie d'un transfert sur membrane de nylon (Hybond N + - Amersham) et hybridation selon le protocole décrit ci-après.

Cette membrane est hybridée avec une sonde radiomarquée avec du dCTPα32P fabriquée à partir de l'ADNc NC30 (Amersham) par coupure partielle de ce dernier à l'aide de la DNAse I, suivie d'une polymérisation à l'aide de l'enzyme ADN polymérase I (technique dite de "nick-translation" ou déplacement de césure), comme décrit par Sambrook et al, op cité. L'hybridation se fait à 42°C pendant 16 h en milieu aqueux contenant 50 % de formamide, 1M de NaCl, une solution de 5 x Denhardt et 0,1 % de SDS. Les membranes sont lavées plusieurs fois à température ambiante avec une solution 2 x SSC contenant 0,1 % de SDS, puis lavées deux fois à 50°C pendant 15 min avec une solution 0,1 x SSC contenant 0,1 % de SDS. La solution de 5 x Denhardt a la composition suivante : Ficoll (type 400 - Pharmacia) 1 g/l, polyvinylpyrrolidone 1 g/l et BSA 1 g/l. La solution 1 x SSC contient 0,15M de NaCl et 0,015M de citrate de sodium.

On constate pour les cellules non stimulées et pour les cellules stimulées dans les conditions 1), 2), 3) et 4) une bande autoradiographique correspondant à un ARN d'environ 1,4 kb. L'expression de cet ARN est augmentée en présence de PMA (bande d'intensité au moins 5 fois plus forte pour la condition de stimulation 1) que pour la condition de stimulation 0)), cette augmentation étant amplifiée avec la présence supplémentaire de PHA-P ou d'anti-CD28 (bandes d'intensité environ 5 fois plus forte pour les conditions de stimulation 2) et 4) que pour la condition de stimulation 1)) et non changée avec la présence supplémentaire de PHA-P et de cyclosporine (bandes d'intensité semblable pour les conditions de stimulation 1) et 3)).

On constate également dans une autre expérience effectuée avec une population de lymphocytes T purifiés (à plus de 95 %) l'expression de l'ARN messager NC30 après costimulation avec le PMA et l'anti-CD28.

**SECTION 5** : Séquençage et analyse de la séquence de l'ADNc du clone NC30 :

1) Séquençage de l'ADNc du clone NC30 :

a) préparation de l'ADN simple brin

Le clone NC30 contient le vecteur pSE1, lequel porte un ADNc entre les sites ApaI et BamHI, appelé par la suite ADNc NC30.

Le vecteur pSE1, qui contient l'origine de replication du phage f1 permet de produire de l'ADN simple brin par culture du clone NC30 en présence du bactériophage M13K07 (Pharmacia- réf : 27-1524) de la manière suivante :

Le clone NC30 est cultivé, dans un tube de 15 ml, sous agitation à 37°C dans 2 ml de milieu 2 x YT de composition : bactotryptone 16 g/l, extrait de levure 10 g/l, NaCl 5 g/l (décrit dans Sambrook et al, op cité), complémenté avec 0,001 % de thiamine et 100 $\mu$g/ml d'ampicilline jusqu'à une densité optique à 660 nm d'environ 0,60.

- 100 $\mu$l de cette culture sont infectés avec du bactériophage M13K07 (Pharmacia- réf : 27-1524) à une multiplicité d'infection de l'ordre de 10 dans un tube de 15 ml. La culture est mise à agiter à 37°C.
- Au bout de 1 h, 2 ml de milieu sont ajoutés. La culture est alors mise à incuber pendant environ 16 h à 37 °C sous agitation.
- 1,5 ml de la culture est centrifugé, dans un microtube, à 15 000 g pendant 2 min.
- 1 ml de surnageant est transféré dans un microtube et additionné de 250 $\mu$l d'une solution de polyéthylène glycol de masse moléculaire 6000, 20 % contenant 2,5M de NaCl. Le mélange est incubé pendant 5 min à 4°C pour faciliter la précipitation du phage puis centrifugé pendant 5 min à 15 000 g. Le surnageant est éliminé et le culot phagique est remis en suspension dans 500 $\mu$l de tampon de composition Tris-HCl 10mM pH8, EDTA 1mM.
- La suspension est extraite une fois au phénol saturé avec du Tris-HCl 100mM pH8, puis deux fois au chloroforme.
- La préparation est ensuite précipitée par adjonction de 1/10 de volume d'une solution d'acétate de sodium 3M pH4,8 et de 2,5 volumes d'éthanol. La précipitation est réalisée à -20°C pendant un minimum de 20 min. L'ADN est centrifugé pendant 10 min à 15 000 g, le culot est lavé avec une solution d'éthanol à 70 % puis remis en suspension dans 30 $\mu$l de tampon de composition : Tris-HCl 10mM pH8, EDTA 1mM.

b) séquençage

Les réactions de séquençage sont réalisées à l'aide du kit de séquençage United States Biochemical (réf : 70770), qui utilise la méthode de Sanger et al , Proc. Ntl. Acad. Sci. USA, 1977, 14, 5463-5467. Les amorces utilisées sont des oligonucléotides de 18 nucléotides, complémentaires soit au vecteur pSE1 dans la région immédiatement en 5' de l'ADNc NC30, soit à la séquence de l'ADNc NC30.

2) Analyse de la séquence de l'ADNc NC30 :

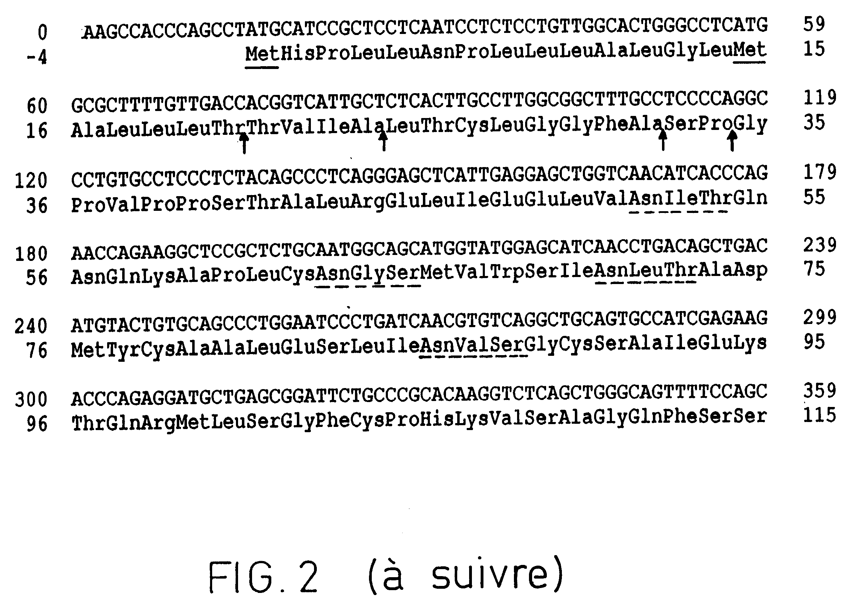

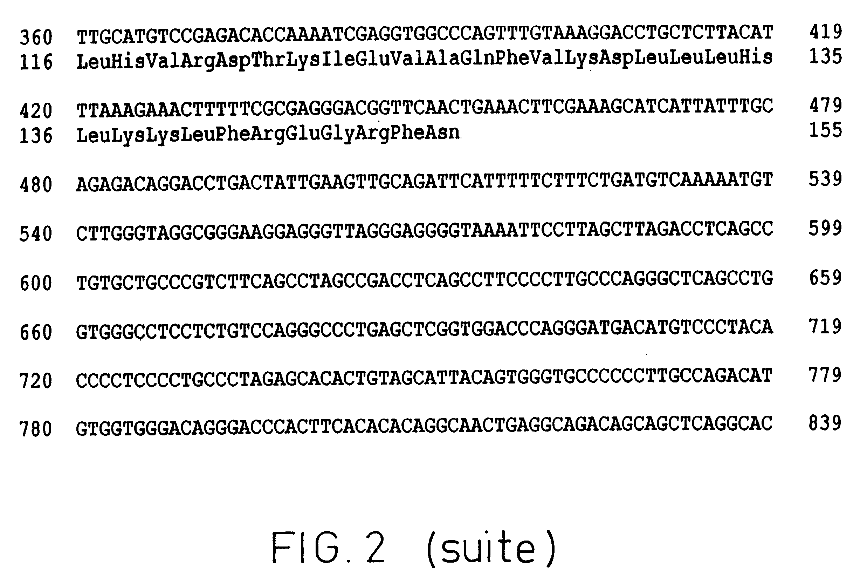

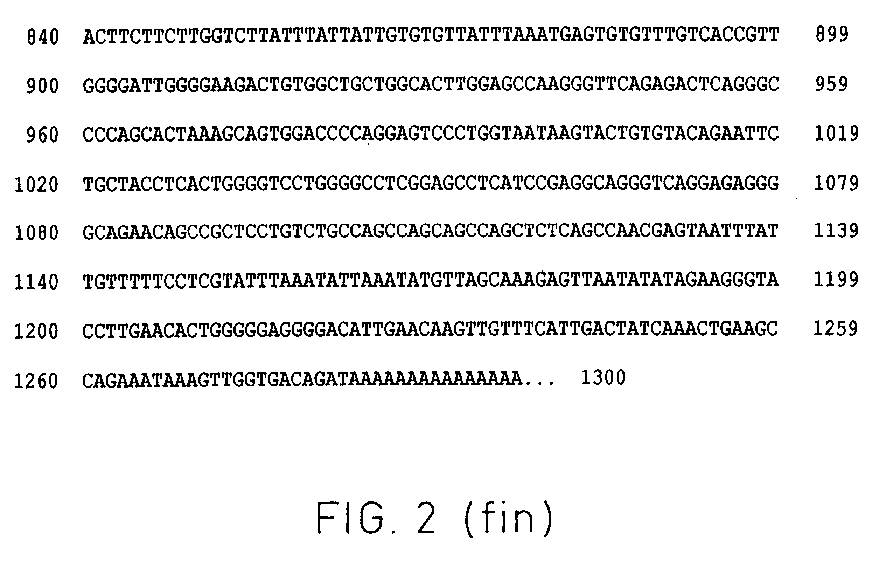

La description ci-après sera mieux comprise à l'aide des figures 2, 3 et 4.
Analyse de la séquence de l'ADNc NC30

(1) L'ADNc NC30 comporte 1282 nucléotides et se termine par une séquence polyA

(2) Ce nombre de nucléotides est en accord avec la taille de l'ARN messager correspondant (environ 1,4 kb) (cf. section 4).

(3) A la position 1264-1269 la séquence AATAAA, qui correspond à la séquence consensus décrite par M. Birnstiel et al, 1985, Cell, 41, 349 est un signal de polyadénylation. Aux positions 855-861, 872-878, 1133-1139 et 1152-1158 on trouve des séquences de 7 nucléotides : TATTTAT, TATTTAA, AATTTAT et TATTTAA, contenant la séquence ATTTA correspondant au motif consensus d'instabilité AUUUA décrit par G. Shaw et al, 1986, Cell, 46, 659-667. L'ADN complémentaire de la plupart des cytokines connues possèdent une séquence correspondant à ce motif consensus d'instabilité.

(4) La séquence d'ADN comporte une phase de lecture ouverte pour la traduction d'une protéine à partir de l'ATG en position 15-17 jusqu'au TGA à la position 453-455 qui correspond à un codon d'arrêt de la traduction. Dans cette phase de lecture il y a deux codons ATG aux positions 15-17 et 57-59, susceptibles d'initier la traduction, correspondant respectivement à des protéines traduites de 146 et 132 acides aminés. Parmi ceux-ci l'environnement nucléotidique de l'ATG aux positions 57-59 est celui qui se rapproche le plus de la séquence consensus décrite par Kozak M., 1978,Cell, 15, 1109-1123, pour l'initiation de la traduction dans les cellules eucaryotes

(5) Un logiciel de recherche de peptide signal, appelé ci-après logiciel PS, a été développé par la Demanderesse d'après la méthode et les informations décrites par Von Heijne, 1986, Nucl. Ac. Res. 14, 483-490. Ce logiciel prévoit dans cette phase de lecture une région hydrophobe ressemblant à un peptide signal et quatre sites vraisemblables de clivage protéique, aux positions 74-75 (entre Thr et Thr), 86-87 (entre Ala et Leu), 110-111 (entre Ala et Ser) et 116-117 (entre Pro et Gly). Le peptide signal prévu est compris entre l'une des deux Met susceptibles d'initier la traduction et l'un de ces quatre sites de clivage. La protéine mature prévue (protéine traduite clivée de son peptide signal) comprend donc une séquence de 126, 122, 114 ou 112 acides aminés.

Les mêmes prévisions sont obtenues à l'aide du Logiciel UWGCG de l'Université du Wisconsin : Devereux et al., 1984, Nucl. Ac. Res., 12, 8711-8721-Option : Recherche de peptide-signal d'après la méthode de G. von Heijne (référence ci-dessus)

Comparaison aux autres séquences connues

La séquence peptidique déjà connue la plus proche de celle de la séquence de 112 acides aminés de la protéine mature, est celle de la protéine de 132 acides aminés déduite de l'ADNc de la protéine P 600 de souris décrite par K.D. Brown et al, 1989, J. Imm., 142, 679-687. Cette protéine est exprimée dans une sous classe de lymphocytes T de souris : les cellules Th2, activées à l'aide de la concanavaline A.

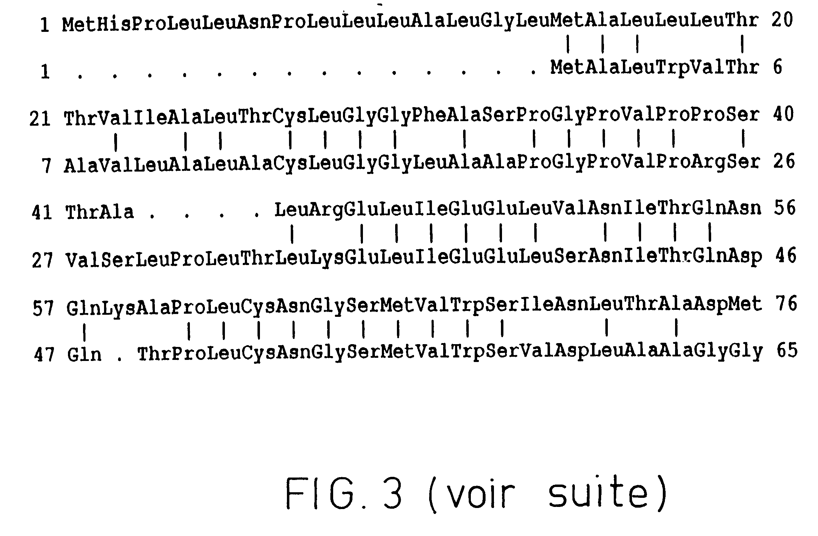

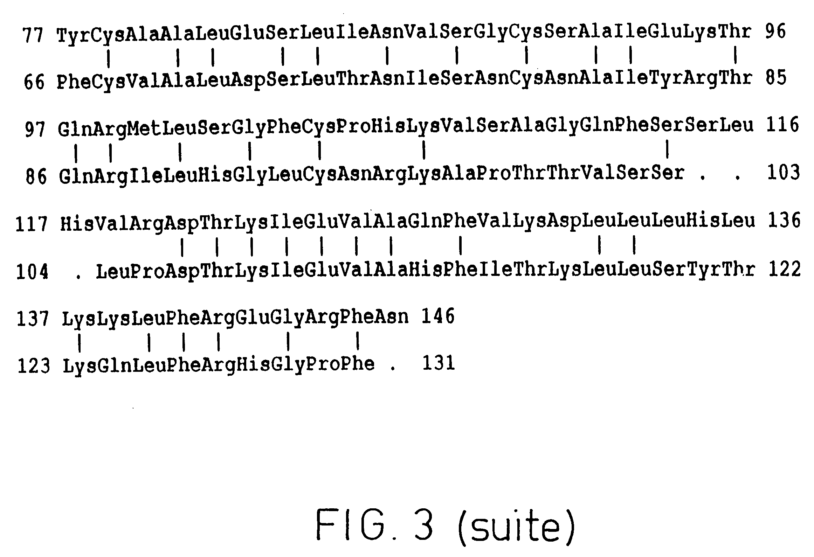

Une comparaison de ces séquences peptidiques à l'aide de la méthode de Needleman et Wunsch, 1970, J. Mol. Biol, 48, 443-453, mise en oeuvre dans le logiciel UWGCG de l'Université du Wisconsin : Devereux et al, 1984, Nucl. Ac. Res. 12, 8711-8721, option GAP, montre que 63 acides aminés sont identiques sur 112, soit une homologie d'identité d'environ 56 %. Cette méthode algorithmique considère tous les alignements possibles et crée un alignement, représenté sur la figure 3, dans lequel le plus possible d'acides aminés identiques sont appariés et le nombre de trous dans les séquences alignées est minimal.

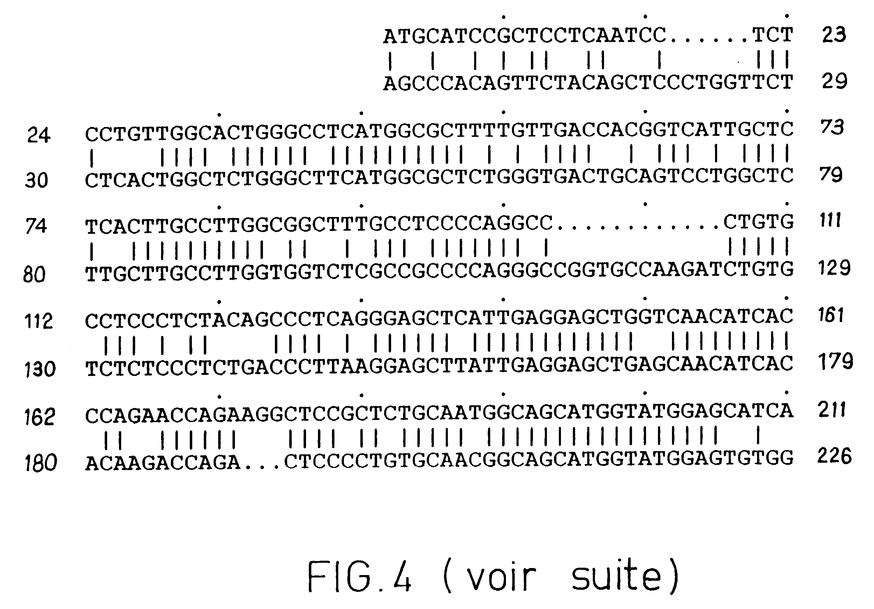

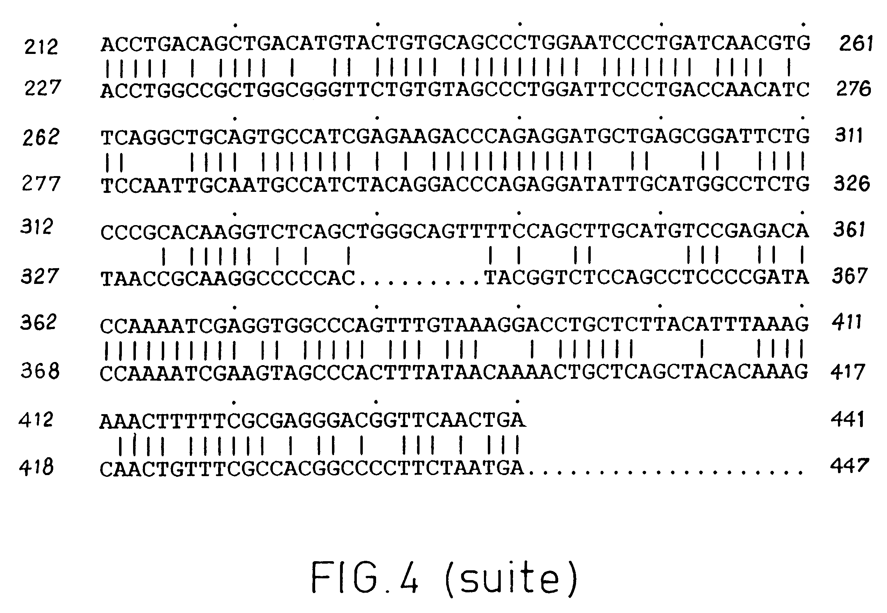

Une comparaison des séquences nucléotidiques par cette méthode montre une homologie d'identité d'environ 70 % entre la séquence codante de l'ADNc NC30 et l'ADNc de la protéine P600 de souris (cf. figure 4).

3) Séquençage de l'ADNc du clone NC30-bis

Le séquençage effectué comme en 1) ci-dessus a permis de retrouver pour l'ADNc du clone NC30-bis la même séquence protéique que pour le clone NC30, à l'exception du 75ème acide aminé traduit, qui est Asp codé par GAC pour le clone NC30 et Gly codé par GGC pour le clone NC30-bis.

**SECTION 6** : Analyse de la sécrétion dans les cellules COS de la protéine codée par l'ADNc NC30

Les cellules COS sont des cellules de reins de singe exprimant l'antigène T du virus SV40 (Gluzman Y., Cell, 23, 1981, 175-182). Ces cellules, qui permettent la réplication des vecteurs contenant l'origine de réplication de l'ADN du virus SV40 (cas du vecteur pSE1) constituent des hôtes de choix pour l'étude de l'expression de gènes dans les cellules animales.

1) Transfection des cellules COS et expression transitoire de la protéine codée par l'ADNc NC30 :

$5 \times 10^5$ cellules COS sont ensemencées dans une boîte de Pétri de 6 cm de diamètre (Corning) dans 5 ml de milieu modifié d'Eagle selon Dubelcco, ci-après appelé DMEM (le Dulbecco's Modified Eagle medium de Gibco réf.041-01965), lequel contient 0,6 g/l glutamine, 3,7 g/l $NaHCO_3$ et est complémenté avec du sérum de veau foetal (Gibco) à raison de 5 %. Après environ 16 h de culture à 37°C dans une atmosphère contenant 5 % de dioxyde de carbone, le milieu de culture est enlevé par aspiration et les cellules sont lavées avec 3 ml de tampon PBS (le Phosphate Buffered Saline de Gibco). On y ajoute alors le mélange suivant : 1000 $\mu$l de (DMEM + 10 % sérum de veau foetal (Gibco)), 110 $\mu$l de diéthylaminoé-thyl-dextrane de poids moléculaire moyen 500 000 (Pharmacia), à une concentration de 2 mg/ml, 1,1 $\mu$l de chloroquine 100mM (Sigma) et 6 $\mu$g d'ADN plasmidique du clone NC30, préparé selon la technique de lyse alcaline suivie de la purification de l'ADN plasmidique sur un gradient de chlorure de césium (Sambrook et al, op cité). Après 5 h d'incubation à 37°C dans une atmosphère contenant 5 % de dioxyde de carbone, le mélange est retiré des cellules. On y ajoute alors 2 ml de tampon PBS contenant 10 % de diméthyl sulfoxyde (qualité Spectroscopie, Merck). Après 1 min d'incubation à la température ambiante, le mélange est retiré et les cellules sont lavées deux fois avec du PBS et sont incubées dans du milieu DMEM contenant 2 % de sérum de veau foetal . L'incubation est poursuivie pendant 40 h à 37°C sous atmosphère contenant 5 % de dioxyde de carbone.

D'autre part, on a préparé des cellules COS témoins en effectuant les opérations décrites ci-dessus avec l'ADN du plasmide pSE1.

2) Marquage des protéines :

L'ensemble des opérations décrites ci-dessous est effectué avec les cellules COS transfectées par l'ADN plasmidique du clone NC30 et les cellules COS témoins.

Le milieu de culture est enlevé par aspiration et les cellules sont lavées deux fois avec 3 ml de tampon PBS. On ajoute 5 ml de milieu MEM (Minimal Eagle's Medium) sans méthionine (Gibco - réf.041-01900H), complété avec 3 g/ml de glucose et 4 mM de glutamine. On incube pendant 2 h à 37°C. on enlève le milieu de culture et on rajoute 2 ml de même milieu additionné de 200 $\mu$Ci de méthionine $^{35}$S (réf. SJ1015 Amersham). On incube pendant 6 h à 37°C. On prélève le milieu de culture, on le centrifuge pendant 5 min pour éliminer les débris cellulaires et les cellules en suspension, et on garde le surnageant.

3) Analyse des protéines radiomarquées des cellules COS transfectées par électrophorèse sur gel de polyacrylamide :

On précipite 1 ml du surnageant des cellules COS transfectées et 9 ml d'acétone à -20°C. On centrifuge et on récupère les culots de protéines. On les reprend dans un tampon de composition : Tris-HCl 0,125M pH 6,8, SDS 4 %, glycérol 20 % et on chauffe à 100°C pendant 10 min. Une partie aliquote de la suspension obtenue, correspondant à une radioactivité de 200 000 cpm, est analysée par électrophorèse sur un gel de polyacrylamide 15 % en présence de SDS, selon la technique décrite par U.K. Laemmli, Anal. Biochem., 1977, 78, 459. Le gel est séché sous vide. Les protéines radiomarquées sont révélées par autoradiographie.

On constate sur l'autoradiogramme la présence de deux bandes surnuméraires pour les cellules transfectées par l'ADN plasmidique du clone NC30 par rapport aux cellules témoins : une bande nette de forte intensité correspondant à une masse moléculaire apparente de 9,0 ± 2 kDa et une bande diffuse de faible intensité correspondant à une masse moléculaire apparente de 16,0 ± 2 kDa. Le clone NC30 code donc pour une protéine sécrétée, appelée par la suite protéine NC30. La masse moléculaire apparente pour la forme de la protéine de l'invention correspondant à la bande de 9 ± 2 kDa est inférieure à la masse moléculaire calculée pour la protéine mature de 112 acides aminés de 12 366 Da. Cette différence est probablement provoquée par une mobilité électrophorétique inattendue de cette forme de la protéine de l'invention.

La bande de masse moléculaire apparente 16,0 ± 2 kDa peut correspondre à une forme N-glycosylée de la protéine de l'invention. Celle-ci présente en effet quatre sites possibles de N-glycosylation soulignés en pointillés sur la figure 2 et correspondant à la séquence consensus décrite par Donner et al, 1987, J. Cell. Biol., 105, 2665.

4) Mise en évidence de la N-glycosylation probable de la protéine de masse moléculaire apparente 16 ± 2 kDa.

Le marquage des protéines est effectué comme en 2) ci-dessus mais en présence de 10 $\mu$g/ml de tunicamycine (Sigma-réf. T7765), agent inhibiteur de la N-glycosylation des protéines.

L'analyse des protéines sur gel de polyacrylamide est effectuée comme en 3).

On constate sur l'autoradiogramme la présence d'une seule bande surnuméraire de 9 ± 2 kDa pour les cellules transfectées par l'ADN plasmidique du clone NC30, par rapport aux cellules témoins. Ces résultats montrent que la forme de la protéine recombinante observée en 3), qui correspond à une masse moléculaire de 16 ± 2 kDa est N-glycosylée.

SECTION 7 : Production de la protéine NC30 dans les cellules COS

4,3 x 10$^7$ cellules COS sont ensemencées dans un flacon de culture cylindrique, dénommé habituellement "roller", de surface 850 cm$^2$ dans 150 ml de milieu DMEM , lequel contient 0,6 g/l glutamine, 3,7 g/l NaHCO$_3$ et est complémenté avec du sérum de veau foetal (Gibco) à raison de 5 %, puis tamponné avec du dioxyde de carbone.

Après environ 16 h de culture à 37°C sur chassis roulant (vitesse de rotation d'environ 0,2 tr/min), le milieu de culture est enlevé par aspiration et les cellules sont lavées avec du tampon PBS (Phosphate Buffered Saline). On y ajoute alors le mélange suivant : 35 ml de milieu DMEM + 10 % sérum de veau foetal (Sigma), 4 ml de diéthylaminoéthyl-dextrane (Pharmacia, poids moléculaire moyen 500 000) à une concentration de 2 mg/ml, 40 $\mu$l de chloroquine 100 mM (Sigma) et 128 $\mu$g d'ADN plasmidique du clone NC30, préparé selon la technique de lyse alcaline, suivie de la purification de l'ADN plasmidique sur un gradient de chlorure de césium (Sambrook et al, op. cité). Après 5 h d'incubation à 37°C dans une atmosphère contenant 5 % de dioxyde de carbone, le mélange est retiré des cellules. On y ajoute alors 35 ml de tampon PBS à 4°C contenant 7 % de diméthylsulfoxyde (qualité Spectroscopie, Merck). Après 1 min et 30 s de rotation à la température ambiante, le mélange est retiré et les cellules sont lavées deux fois avec du PBS. On ajoute 150 ml de milieu DMEM (Sigma) contenant 1 % de sérum de veau foetal (SVF) par roller et on incube les cellules à 37°C en présence de 5 % de $CO_2$ (rotation à 0,2 tr/min). Un jour après la transfection, le milieu est retiré par aspiration, les cellules sont rincées 2 fois avec du PBS. On ajoute alors 150 ml par roller de milieu DMEM (Sigma) sans sérum et les rollers sont remis à 37°C en présence de 5 % de $CO_2$ (en rotation à 0,2 tr/min) pendant 5 jours.

La récolte a lieu au 6ème jour après la transfection.

On centrifuge le milieu de culture à 7 000 tr/min pendant 10 min ; le surnageant est filtré sur un filtre 0,2 $\mu$m de Nalgène.

**SECTION 8** : Purification de la protéine NC-30 produite dans les cellules COS, et détermination de sa séquence amino-terminale

1) Purification de la protéine NC30

La forme majoritaire de la protéine recombinante de masse moléculaire apparente 9 ± 2 kDa a été purifiée à partir de 500 ml du surnageant obtenu dans la section 7 à l'aide des étapes suivantes :

- chromatographie d'échange d'ions sur une colonne S fast flow (Pharmacia) (15 x 100 mm), préalablement équilibrée avec une solution d'acétate de sodium 50 mM pH 4,0, avec comme éluant une solution de NaCl 1M dans un tampon acétate de sodium 50 mM pH 4,0 avec un débit de 2 ml/min.
- l'éluat est concentré sur une membrane centriprep 10 (Amicon) jusqu'à un volume d'environ 1 ml puis soumis à une chromatographie HPLC (phase inverse sur colonne C4 (Bownlee) (100 x 2,1 mm), préalablement équilibrée avec une solution contenant 30 % d'acétonitrile et 0,1 % de TFA, avec un gradient linéaire de 30 à 70 % d'acétonitrile dans une solution contenant 0,1 % de TFA. Les fractions contenant la protéine recombinante (déterminée par analyse électrophorétique sur gel de polyacrylamide en présence de SDS) sont rassemblées.

2) Détermination de la séquence aminoterminale de la protéine NC30 de masse moléculaire apparente 9 ± 2 kDa :

La protéine obtenue à l'issue de 1) a été soumise à une analyse électrophorétique sur gel de polyacrylamide 16 % en présence de SDS. Les protéines sont transférées pendant 1 h avec une intensité de 0,8 mA/cm$^2$ dans un tampon de composition Tris-borate 25 mM pH9,0 et méthanol 10 % sur une membrane Immobilon (Millipore), puis révélées au bleu de Coomassie.

La bande de masse moléculaire apparente 9 ± 2 kDa a été découpée et déposée sur un séquenceur d'Applied Biosystems modèle 470 A couplé à un analyseur des dérivés phénylthiohidantoïques d'Applied Biosystems modèle 120 A/

La séquence aminoterminale obtenue est la séquence (tr$_o$) suivante :

**Gly Pro Val Pro Pro Ser Thr Ala Leu Arg Glu Leu Ile Glu Glu Leu**

| 1 | 5 | 10 | 15 |

laquelle correspond au quatrième site de clivage prévu par le logiciel PS (cf. section 5 2).

**SECTION 9** : Construction d'un vecteur d'expression de l'ADNc NC30 dans la levure, le plasmide pEMR673 et transformation d'une souche de levure à l'aide de ce plasmide :

1) Construction du plasmide pEMR673

Le plasmide pEMR583 (décrit dans la demande de brevet EP-435776) a été soumis à une digestion complète par les enzymes HindIII et BamHI. Le grand fragment (appelé ci-après fragment A) comprenant l'origine de replication et le locus STB du 2 $\mu$, le gène LEU2d, le gène de résistance à l'ampicilline, l'origine de pBR322, le terminateur du gène PGK, le gène URA3, le promoteur artificiel et le début de la région prépro de la phéromone alpha a été purifié.

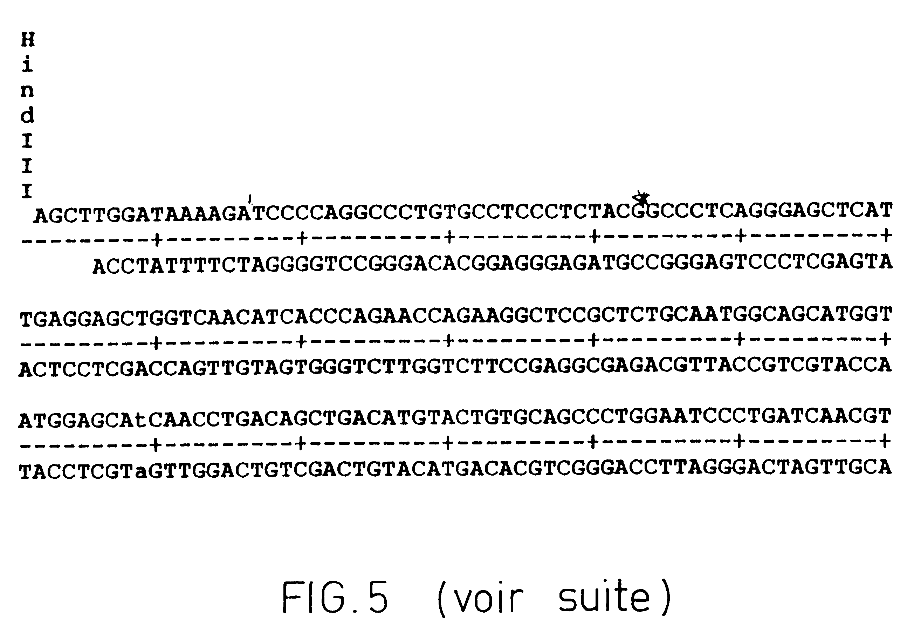

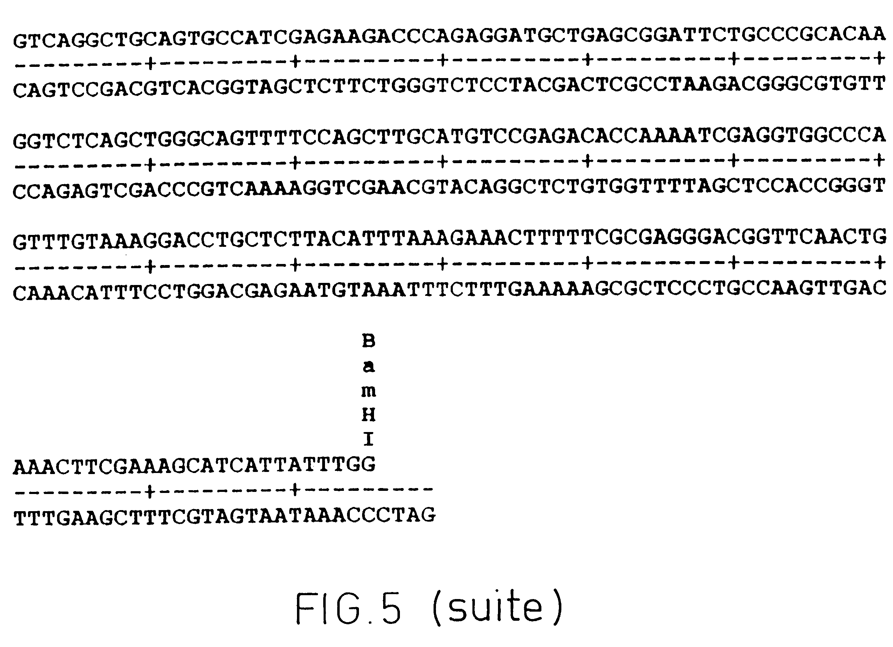

Le fragment HindIII - BamHI (nommé ci-après fragment B) comprenant la fin de la région prépro de la phéromone alpha et l'ADNc codant pour la protéine mature, flanqué du site de restriction BamHI en 3', a été obtenu par amplification par la technique PCR à partir du plasmide pSE1-NC30 . La séquence de ce fragment est précisée sur la figure 5. Les fragments A et B ont été ligués de façon à obtenir le plasmide pEMR673

a) Description de la technique de la réaction polymérase en chaîne (Polymerase chain reaction : PCR)

La technique de la réaction de polymérase en chaîne (PCR) est une méthode bien connue de l'homme de l'art qui permet de copier simultanément les deux brins d'une séquence d'ADN préalablement dénaturé en utilisant deux oligonucléotides comme amorces (cf. notamment l'ouvrage de H.A. Erlich : "PCR Technology : Principles and Applications for DNA amplification" publié en 1989 par les éditions Macmillan Publishers Ltd, Royaume-Uni et celui de M.A. Innis et al. "PCR Protocols" publié en 1990 par Academic Press Inc. San Diego, Californie 92101, USA). Le principe de cette technique est résumé ci-après.

La technique du PCR est basée sur la répétition de trois étapes, permettant d'obtenir, après entre 10 et 30 cycles, des centaines de milliers de copies de la matrice originale, à l'aide d'une ADN polymérase de Thermus aquaticus, habituellement appelée polymérase Taq. Les trois étapes sont les suivantes :

- Dénaturation de la matrice :

L'ADN double brin est dénaturé en ADN simple brin par incubation à haute température (de 92°C à 96°C) pendant approximativement 2 min.

- Hybridation des amorces :

Ces amorces sont une paire d'oligonucléotides synthétiques qui s'hybrident avec les extrémités de la région à amplifier. Les deux amorces s'hybrident avec les brins opposés. Les amorces sont ajoutées en excès, de façon à ce que la formation du complexe amorce-matrice soit favorisée.

- Extension des amorces :

L'étape au cours de laquelle la polymérase Taq assure l'extension du complexe amorce-matrice de 5' vers 3' est effectuée à 72°C.

Dans la technique du PCR, le produit d'intérêt apparaît au troisième cycle et il est alors amplifié de manière significative. Au cours du déroulement des cycles, le produit d'amplification devient rapidement la matrice avec laquelle les amorces s'hybrident.

b) Description des amorces utilisées

Deux oligonucléothides synthétiques ont été préparés.

Le premier oligonucléotide, appelé amorce 1, dont la séquence est la suivante :

CAGTGAATTC A AGC TTG GAT AAA AGA    TCC CCA GGC CCT GTG CCT CC
Ser  Leu Asp  Lys  Arg    Ser  Pro  Gly  Pro  Val  Pro


Région 1                                                 Région 2


possède deux régions distinctes : la région 1, qui contient la fin de la région prépro de la phéromone $\alpha$ modifiée par rapport à la séquence naturelle décrite par Kurjan et al, 1982, Cell, 30, 933-943 par une mutation silencieuse qui permet d'introduire un site de clivage HindIII juste avant la partie codante de la région 1 (onzième nucléotide de la région 1), et la région 2, qui est une région destinée à s'hybrider avec la région codante correspondant au début de la protéine mature de 114 acides aminés (cf. section 5), du brin non codant de l'ADNc NC30.

Le deuxième oligonucléotide, appelé amorce 2, dont la séquence est la suivante :


CGACGGATCC      CAAATAATGA TGCTTTCGAA G


Région 1                          Région 2


est également constitué de deux régions distinctes : la région 1 qui porte sur le quatrième nucléotide un site BamHI, et la région 2, qui porte une séquence nucléotidique correspondant à la région 3' non traduite de l'ADNc NC30.
Cette région est destinée à s'hybrider avec le brin codant de l'ADNc NC30 .

c) Obtention du fragment amplifié HindIII - BamHI représentant la fin de la région prépro de la phéromone $\alpha$ et l'ADNc codant pour la protéine mature NC30

On utilise comme matrice le plasmide pSE1 - NC30 qui porte l'ADNc codant pour la protéine NC30.
Dans un tube on ajoute 100 ng du plamide pSE1 - NC30, 100 ng de l'amorce 1, 100 ng de l'amorce 2, 5 $\mu$l de mélange réactionnel concentré 10 fois (concentration finale : 67mM Tris-HCl pH 8,8, 16,6 mM $(NH_4)_2SO_4$, 1 mM $\beta$ mercaptoéthanol, 6,7 mM EDTA, 0,15 % Triton x 100, 2mM $MgCl_2$, 0,2 mM de dNTP, 200 ng de gelatine) ; le volume du mélange est ensuite porté à 50 $\mu$l en ajoutant de l'eau.
On ajoute 0,5 $\mu$l, soit 2,5 unités de Polymérase Taq (Boehringer Manheim réf 1146 - 173). On couvre alors le mélange avec de la paraffine afin d'éviter l'évaporation de la solution aqueuse.
L'amplification se fait au cours de 15 cycles réactionnels dont les étapes d'un cycle sont les suivantes :
- 1 min à 94°C -> dénaturation
- 1 min à 55°C -> hybridation
- 1 min à 72°C -> polymérisation
Après les 15 cycles, la réaction enzymatique est arrêtée par l'addition de 20 mM EDTA.
Le fragment d'ADN ainsi amplifié, qui présente la taille attendue d'environ 380 pb, est ensuite isolé et purifié sur gel d'agarose 1%, purifié par chromatographie sur colonne de gel de polyacrylamide P10 (Pharmacia), puis hydrolysé totalement et simultanément par les enzymes HindIII et BamHI selon les techniques habituelles bien connues de l'homme de l'art (Sambrook,1983 ) afin de former les extrémités cohésives HindIII et BamHI. Après hydrolyse le fragment est purifié sur colonne P10.
La séquence du fragment B obtenu est représentée sur la figure 5. Elle comprend dans sa partie codant pour la protéine NC30 une mutation silencieuse par rapport à l'ADNc NC30, indiquée par une astérisque sur la figure 5.
Les fragments A et B ont été ligués de manière à obtenir le plasmide pEMR673.

2) Transformation de la souche de levure EMY761 par le plasmide pEMR673 et expression de la protéine NC30 par la souche transformée

La souche EMY761 (Mat alpha, leu2, ura3, his3) décrite dans le brevet EP-0408461 et pouvant être obtenue par curage plasmidique de la souche déposée à la CNCM le 27 Décembre 1989 sous le n° I-1021 contient des mutations (leu2 et ura3), susceptibles d'être complémentées par le marqueur de sélection défectif LEU2d et le marqueur de sélection URA3, présents dans le plasmide pEMR673. Elle a été transformée par le plasmide pEMR673 avec sélection pour la prototrophie de leucine selon une variante de la technique de transformation décrite par Beggs et al. (Beggs et al, 1978, Nature, 275, 104-109) qui consiste à soumettre les levures à un traitement de protoplastisation en présence d'un stabilisant osmotique, le sorbitol en concentration 1 M.

Le protocole précis de transformation est précisé ci-après :

a) 200 ml du milieu YPG liquide (cf. tableau 1 ci-après) sont inoculés avec environ 5 x $10^6$ cellules d'une culture en phase stationnaire et la culture ainsi inoculée est placée pendant une nuit sous agitation à 30°C.

b) Lorsque la culture atteint environ $10^7$ cellules par ml, les cellules sont centrifugées à 4 000 tr/min pendant 5 min et le culot est lavé avec une solution de sorbitol 1 M.

c) Les cellules sont mises en suspension dans 5 ml de solution de sorbitol 1 M contenant 25 mM EDTA et 50 mM de dithiothréitol et incubées pendant 10 min à 30°C.

d) les cellules sont lavées une fois avec 10 ml de solution de sorbitol 1 M et mises en suspension dans 20 ml de solution de sorbitol. De la zymolase-100T (préparation commercialisée par Seykagaku Kogyo Co. Ltd., obtenue par purification partielle sur une colonne d'affinité du surnageant de culture d'Athrobacter luteus et contenant de la $\beta$-1,3-glucanase-laminaripenta-hydrolase) est ajoutée à une concentration finale de 20 $\mu$g/ml et on incube la suspension à température ambiante pendant environ 15 min.

e) Les cellules sont remises en suspension dans 20 ml d'un milieu contenant du sorbitol appelé milieu YPGsorbitol (cf. tableau I ci-après) et incubées pendant 20 min à 30°C sous agitation douce.

f) On centrifuge pendant 3 min à 2 500 tr/min.

g) On remet en suspension les cellules dans 9 ml de tampon de transformation de composition : sorbitol 1 M, Tris-HCl pH 7,5 10 mM et $CaCl_2$ 10 mM).

h) On ajoute 0,1 ml de cellules et 5 $\mu$l de solution d'ADN (environ 5 $\mu$g) et on laisse la suspension obtenue pendant 10 à 15 min à température ambiante.

i) On ajoute 1 ml de la solution : polyéthylène glycol PEG 4000 20 %, Tris-HCl 10 mM pH 7,5 et $CaCl_2$ 10 mM.

j) On verse 0,1 ml de la suspension obtenue en i) dans un tube contenant du milieu solide de régénération sans leucine (cf. tableau I ci-après) préalablement fondu et maintenu liquide à environ 45°C. On verse la suspension sur une boîte de Pétri contenant une couche solidifiée de 15 ml de milieu de régénération solide sans leucine.

Les transformés commencent à apparaître au bout de trois jours. On a ainsi retenu un transformé, appelé souche EMY761 pEMR673.

## Tableau 1

Composition et préparation des principaux milieux utilisés dans le protocole
de transformation de la souche de levure EMY761

– milieu YPG liquide

10 g d'extrait de levure (Bacto–Yeast extract de Difco)

20 g de peptone (Bacto–peptone de Difco)

20 g de glucose

mélanger les ingrédients dans de l'eau distillée. Compléter le volume final à 1 l
avec de l'eau distillée – Autoclaver pendant 15 min à 120°C.

– milieu YPG sorbitol

utiliser la formule du milieu YPG liquide auquel on ajoute, après autoclavage,
du sorbitol à une concentration de 1 M.

– milieu solide de régénération sans leucine

6,7 g de base azotée de levure sans acides aminés (Yeast nitrogen base without Amino Acids de Difco)

20 mg d'adénine

20 mg d'uracile

20 mg de L–tryptophane

20 mg de L–histidine

20 mg de L–arginine

20 mg de L–méthionine

30 mg de L–tyrosine

30 mg de L–isoleucine

30 mg de L–lysine

50 mg de L–phénylalanine

100 mg de L–acide glutamique

150 mg de L–valine

20 g de glucose

30 g d'agar

182 g de sorbitol

mélanger tous les ingrédients dans l'eau distillée. Compléter le volume final à 1 l, avec de l'eau distillée. Autoclaver pendant 15 min à 120°C. Après autoclavage, ajouter 200 mg de L-thréonine et 100 mg d'acide L-aspartique.

**SECTION 10** : Expression en erlenmeyer de la protéine NC30 par la souche de levure transformée et mise en évidence de la protéine dans le milieu de culture sur gel de polyacrylamide en présence de SDS

1) Culture de la souche EMY761 pEMR673

Une colonie de la souche EMY761 pEMR673 (obtenue dans la section 9) a été mise en culture dans 50 ml de milieu liquide sans uracile. Ce milieu contient pour 1 litre :
- 6,7 g de base azotée de levure sans acides aminés (Yeast nitrogen base without aminoacids de Difco).
- 5,0 g d'hydrolysat de caséine (casaminoacids de Difco)
- 10 g de glucose

Après une nuit à 30°C sous agitation, la culture a été centrifugée pendant 10 min, le culot a été repris dans 10 ml d'eau stérile et de nouveau centrifugée pendant 10 min. L'expression de la protéine NC30 a été induite en reprenant les cellules dans 50 ml de milieu de composition suivante :
- 6,7 g/l de yeast nitrogen base without Aminoacids de Difco
- 5,0 g/l d'hydrolysat de caséine (casaminoacids de Difco)
- 30,0 g/l de glycérol
- 30,0 g/l de galactose
- 10 ml/l d'éthanol.

La culture a été replacée à 30°C sous agitation pendant 24 h.

2) Analyse de la protéine exprimée

a) gel de polyacrylamide en présence de SDS

Préparation des échantillons

Une partie des cellules cultivées pendant 1 nuit dans un milieu appelé milieu liquide sans uracile avec glucose dont la composition est précisée dans le tableau 2 ci-après, a été centrifugée : échantillon non induit. Les cellules cultivées pendant 1 nuit dans un milieu, appelé milieu liquide sans uracile avec éthanol, glycérol et galactose (tableau 2 ci-après) ont été centrifugées : échantillon induit. Le surnageant a été recueilli. A 10 ml de surnageant, 5 ml d'acide trichloroacétique à 50% contenant 2 mg/ml de désoxycholate ont été rajoutés.

Le mélange a été mis à la température de + 4°C pendant 30 min, puis centrifugé pendant 30 min. Le culot a été repris dans environ 1 ml d'acétone froid (+ 4°C) et de nouveau centrifugé pendant 30 min. Le culot, après avoir été séché, est repris dans environ 20 $\mu$l d'un tampon dénommé tampon de charge constitué de Tris-HCl 0,125 M pH 6,8, SDS 4%, bleu de bromophénol 0,002 %, glycérol 20 %, $\beta$-mercaptoéthanol 10 % selon le protocole décrit par Laemmli en 1970, bien connu de l'homme de l'art. Le culot est solubilisé par ébullition pendant 15 min puis neutralisé.

Les échantillons sont déposés sur un gel de polyacrylamide en présence de SDS et soumis à une électrophorèse.

Résultats : L'analyse du gel (révélation au bleu de Coomassie) montre pour l'échantillon induit la présence de plusieurs bandes supplémentaires par rapport à l'échantillon non induit, dont les deux principales correspondent à un poids moléculaire apparent de 9 ± 2 et 16 ± 2 kDa. Les autres bandes supplémentaires observées, qui sont assez nombreuses et diffuses, correspondent probablement à un degré variable de glycosylation.

On sait que la N-glycosylation d'une protéine par la levure fait intervenir une N-glycosylation simple ("core glycosylation") dans le reticulum endoplasmique et une N-hyperglycosylation ("outer-chain glycosylation") dans l'appareil de Golgi (R.A. Hitzeman et al, 1990, "Methods in Enzymology, n° 185", Academic Press, p. 421-440). En général la N-glycosylation simple conduit à une glyco-protéine de poids moléculaire apparent homogène (une bande) et la N-hyperglycosylation à une glycoprotéine de poids moléculaire apparent hétérogène (pluralité de bandes diffuses)

b) Immunoempreinte (Western blot) avec traitement éventuel à l'endoglycosidase H

Préparation des échantillons

Une partie des cellules cultivées pendant une nuit en milieu liquide sans uracile avec glucose (tableau 2) a été centrifugée : échantillon non induit. Les cellules cultivées pendant une nuit en milieu liquide sans uracile avec éthanol, avec glycérol et galactose,(tableau 2) ont été centrifugées : échantillon induit. Le surnageant a été recueilli. A 10 ml de surnageant, 5 ml d'acide trichloroacétique à 50% contenant 2 mg/ml de désoxycholate ont été rajoutés.

Le mélange a été mis à la température de + 4°C pendant 30 min, puis centrifugé pendant 30 min. Le culot a été repris dans environ 1 ml d'acétone froid (+ 4°C) et de nouveau centrifugé pendant 30 min. Le culot est repris dans 20 $\mu$l d'un tampon de solubilisation (de composition Tris-HCl pH 6.8 10 mM, $\beta$ mercaptoéthanol 2 %, SDS 1 %). Le culot est porté à 100°C pendant 5 min.

L'échantillon est ensuite partagé en deux parties
- à la première partie de 10 $\mu$l, on ajoute 10 $\mu$l d'un tampon citrate de sodium 50 mM pH 5,5 contenant de l'endoglycosidase H (5 mUI : Boehringer réf. 1088726). L'échantillon est placé à 37°C pendant environ 1 nuit. On ajoute ensuite 20 $\mu$l de tampon de charge
- à la deuxième partie de 10 $\mu$l on ajoute 10 $\mu$l de tampon de charge. Les échantillons sont portés à ébullition pendant 10 min.

On dépose les échantillons sur gel de polyacrylamide en présence de SDS et on effectue une électrophorèse selon le protocole de Laemmli (référence déjà citée).

Les protéines contenues dans le gel sont ensuite transférées sur membrane de nitrocellulose (selon la technique de H. Towbin et al, 1979, Proc. Natl. Acad. Sci. USA, 76, 4350-4354). L'immunodétection, réalisée selon le protocole décrit dans l'immuno-Blot Assay Kit de Bio-Rad (réf. 170-6450) implique les étapes suivantes :
- la saturation de la membrane de nitrocellulose avec un tampon TBS ("Tris Buffered Saline") qui contient 3 g/100 ml de gélatine pendant 30 min.
- le rinçage de la membrane avec un tampon dénommé T. TBS (tampon TBS qui contient 0,05 % de Tween 20), deux fois pendant 5 min
- la mise en contact de la membrane avec l'immunsérum préparé dans la section 13, pendant 1 h à température ambiante
- le rinçage de la membrane avec le tampon T. TBS, deux fois pendant 5 min
- la mise en contact de la membrane avec l'anticorps conjugué du kit
- le rinçage de la membrane avec le tampon T.TBS, deux fois pendant 5 min et une fois pendant 5 min avec le tampon TBS
- le complexe Antigène-Anticorps est révélé par la mise en contact de la membrane avec un tampon de développement contenant du 5-bromo-4-chloro-3-indolyl-phosphate (BCIP) et du nitroblue tétrazolium (NBT)
- le rinçage de la membrane avec de l'eau.

Résultats : L'analyse de l'immunoempreinte montre pour l'échantillon induit non traité à l'endoglycosidase H la présence de plusieurs bandes supplémentaires par rapport à l'échantillon non induit, dont les deux principales correspondent à un poids moléculaire apparent de 9 ± 2 et 16 ± 2 kDa. D'autres bandes nombreuses et diffuses, de poids moléculaire supérieur sont également mises en évidence. Toutes ces bandes sont reconnues par l'immunsérum préparé dans la section 13.

Dans l'échantillon induit la bande correspondant à un poids moléculaire apparent de 16 ± 2 kDa tend à disparaître après traitement à l'endoglycosidase H tandis que la bande à 9 ± 2kDa augmente en intensité dans les mêmes conditions. Ces résultats montrent que la protéine de masse moléculaire apparente de 16 ± 2 kDa est N-glycosylée.

Les bandes diffuses de poids moléculaire supérieur disparaissent également et l'on remarque l'apparition de 2 bandes correspondant à des poids moléculaires apparents d'environ 18 ± 2 et 20 ± 2 kDa Les formes de la protéine NC30 résistant au traitement à l'endoglycosidase H peuvent correspondre au précurseur ayant gardé la séquence pro de la phéromone ou à des formes O- glycosylées de la protéine NC30.

## Tableau 2

Composition et préparation de certains milieux
utilisés pour la préparation des échantillons

– Milieu liquide sans uracile avec glucose :

– 6,7 g de base azotée de levure sans acides aminés
 (Yeast nitrogen base without amino acids de Difco)

– 5,0 g d'hydrolysat de caséine (casaminoacids de Difco)

– 10,0 g de glucose

mélanger tous les ingrédients dans de l'eau distillée, et compléter à 1 l final avec de l'eau distillée.

Autoclaver pendant 10 min à 120°C.

– Milieu liquide sans uracile avec éthanol, glycérol et galactose :

utiliser la formule du milieu liquide sans uracile décrite ci-dessus mais sans glucose. Après autoclavage ajouter 10 ml d'éthanol 100 %, 30 g de glycérol et 30 g de galactose

**SECTION 11** : Production de la protéine NC30 en fermenteur à l'aide de la souche EMY761 pEMR673

La culture de la souche EMY761 pEMR673 se fait en fermenteur de la manière suivante :

a) Phase de préculture en fiole conique à bafles

A partir de 1 ml de suspension de culture contenant 20 % de glycérol de la souche ci-dessus avec un nombre de cellules correspondant à une D.O. de 3 pour $\lambda$ = 600 nm (sur spectro Kontron), on ensemence une fiole conique à bafles de 500 ml contenant 90 ml de milieu de croissance hémisynthétique phase autoclavable (MCHPA), complémentés par 1,28 g de tampon MES-acide 2-(N-morpholino) ethanesulfonique (M 8250 de Sigma) et 10 ml de milieu de croissance hémisynthétique phase filtrée (MCHPF). Les compositions chimiques et le mode préparatoire des milieux MCHPA et MCHPF sont précisés ci-après. Après 24 h d'incubation sous agitation à 30°C, la densité optique de la culture (D.O) à $\lambda$ = 600 nm est d'environ 7.

b) Phase de croissance en fermenteur

La culture ci-dessus est utilisée pour ensemencer un fermenteur de 2,5 l, prérempli avec :
800 ml de milieu MCHPA
100 ml de milieu MCHPF
Le pH de la culture est régulé par le fermenteur à une valeur de consigne de 5,5. De même la pression d'oxygène est maintenue au-dessus de 4 000 Pa (30 mm Hg) par régulation de l'agitation. Initialement le débit d'air est fixé à 1 l/min, soit environ 1 VVM, puis est augmenté par paliers selon les besoins.

Après 6 à 7 h de culture à 30°C, on ajoute de façon linéaire et pendant 9 h, 72 ml d'une solution de glucose à 500 g/l, soit en tout 36 g de glucose.

c) Phase d'expression en fermenteur

Au mélange précédemment décrit, on ajoute 100 ml de milieu d'expression hémisynthétique phase autoclavable (MEHPA) et 100 ml de milieu d'expression hémisynthétique phase filtrée (MEHPF), dont les compositions chimiques et le mode de préparation son précisés ci-après. La culture est alors continuée, pendant environ 5 h sans ajout. Les concentrations des trois sources en carbone (glycérol, galactose, éthanol) sont suivies par HPLC et complétées par des injections stériles de manière à se rapprocher des valeurs suivantes : glycérol 15 g/l, éthanol 15 g/l, galactose 7,5 g/l.

Entre 23 à 24 h après et sous induction une D.O à $\lambda$ = 600 nm voisine de 90 est atteinte et la culture est arrêtée.

La suspension de culture est alors centrifugée à 11 500 g pendant 30 min. Le culot de cellules de levure est éliminé, le surnageant conservé est congelé à -80°C.

COMPOSITION CHIMIQUE DES MILIEUX DE CROISSANCE ET D'EXPRESSION

- Milieu de croissance hémisynthétique phase autoclavable "MCHPA"

|  | Pour 800 ml final (avec de l'eau ultrapurifiée) |
|---|---|
| NTA (acide nitrilotriacétique) | 1 g |
| $K_2SO_4$ | 1 g |
| NaCl | 0,5 g |
| $MgSO_4, 7H_2O$ | 1,0 g |
| $CaCl_2, 7H_2O$ | 700 mg |
| acide glutamique | 3,7 g |
| HY-CASE SF (Sheffield Products) | 25 g |
| leucine | 1,8 g |
| histidine | 500 mg |
| méthionine | 1 g |
| oligoéléments de type I-S (cf ci-après) | 5 ml |
| Ajuster le pH à 5,5 avec $H_2SO_4$ concentré ou KOH concentré. Autoclaver pendant 20 min à 120°C. | |

- Liste des oligoéléments de type I - S

|  |  | Pour 1 litre final (avec de l'eau ultrapurifiée) |
|---|---|---|
| sulfate de cuivre | $CuSO_4, 5H_2O$ | 780 mg |
| acide borique | $H_3BO_3$ | 5 g |
| sulfate de zinc | $ZnSO_4, 7H_2O$ | 3 g |
| iodure de potassium | KI | 1 g |
| sulfate de manganèse | $MnSO_4, 2H_2O$ | 3,5 g |
| molybdate de sodium | $Na_2MoO_4, 2H_2O$ | 2 g |
| chlorure ferrique | $FeCl_3, 6H_2O$ | 4,8 g |
| Ajouter à la solution 100 ml d'acide chlorhydrique concentré Compléter à 1 000 ml. | | |

- Milieu de croissance hémisynthétique phase filtrée "MCHPF"

|  | pour 100 ml final (avec de l'eau ultrapurifiée) |
| --- | --- |
| $KH_2PO_4$ | 4 g |
| tryptophane | 350 mg |
| vitamines de type I-S (cf-ci-après) | 1 ml |
| glucose | 15 g |
| Chauffer pour dissoudre, tempérer, ajouter les vitamines de type I - S et stériliser par filtration sur membrane 0,2 $\mu$m. | |

- Liste des vitamines de type I - S

|  | pour 100 ml final (avec de l'eau ultrapurifiée) |
| --- | --- |
| biotine | 5 mg |
| acide folique | 4 mg |
| niacine | 6mg |
| (acide nicotinique pyridoxine.HCl) | 250 mg |
| thiamine. HCl | 1 g |
| Ca. pantothénate | 5 g |
| m. inositol | 10 g |
| Compléter à 100 ml après dissolution<br>Filtrer à froid stérilement sur membrane 0,2 $\mu$m.<br>Conserver à une température de + 4°C. | |

- Milieu d'expression hémisynthétique phase autoclavable "MEHPA"

|  | pour 400 ml (avec de l'eau ultrapurifiée) |
|---|---|
| NTA | 1 g |
| $K_2SO_4$ | 1,74 g |
| acide glutamique | 5 g |
| HY-CASE SF (Sheffield Products) | 20 g |
| leucine | 1,8 g |
| histidine | 500 mg |
| méthionine | 1 g |
| tryptophane | 350 mg |
| $MgSO_4, 2H_2O$ | 600 mg |
| oligoéléments de type I-S (cf ci-dessus) | 5 ml |
| Ajuster le pH à 5,5 avec $H_2SO_4$ ou KOH concentré Autoclaver pendant 20 min à une température de 120°C. | |

- Milieu d'expression hémisynthétique phase filtrée "MEHPF"

|  | pour 100 ml final (avec de l'eau purifiée) |
|---|---|
| $KH_2PO_4$ | 2 g |
| tryptophane | 350 mg |
| vitamines de type I-S (cf ci-dessus) | 1 ml |
| glycérol | 15 g |
| éthanol | 15 g |
| galactose | 7,5 g |
| Chauffer pour dissoudre, tempérer, ajouter les vitamines et stériliser par filtration sur membrane 0,2 $\mu$m. | |

d) Analyse de la protéine produite

Des échantillons ont été préparés de manière analogue à celle décrite dans la section 10 et soumis à une électrophorèse sur gel de polyacrylamide en présence de SDS. On observe une répartition des bandes sur le gel identique à celle observée dans la section 10 pour l'échantillon induit.

**SECTION 12** : Purification de la protéine NC30 produite dans la levure et détermination de sa séquence aminoterminale et de sa carte peptidique

1) Purification des deux formes majoritaires de la protéine NC30

Les deux formes majoritaires de la protéine recombinante de levure, celles des bandes correspondant à des masses moléculaires apparentes après électrophorèse sur gel de polyacrylamide en présence de SDS de 9 ± 2 kDa et 16 ± 2 kDa ont été isolées et purifiées à partir de 500 ml du surnageant obtenu dans la section 11 selon la méthode suivante :
Plusieurs étapes ont été réalisées successivement :

- chromatographie d'échange d'ions sur une colonne Q fast flow (Pharmacia) (5 x 5 cm), préalablement équilibrée avec une solution d'acétate de sodium 50 mM pH 4.0. Débit : 1 ml/min. Le pH du surnageant est préalablement ajusté à 4,0 Dans ces conditions opératoires, la protéine ne se fixe pas sur le gel.
- chromatographie d'échange d'ions sur une colonne S fast flow (Pharmacia) (5 x 4 cm), préalablement équilibrée avec une solution d'acétate de sodium 50 mM pH 4.0., avec comme éluant une solution de NaCl 1M dans un tampon acétate de sodium 50 mM pH 4,0, Débit : 1 ml/min.
- L'éluat est concentré sur une membrane YM5 (Amicon) jusqu'à un volume d'environ 2 ml puis déposé sur une colonne de gel filtration ACA 54 (IBF) (100 x 1,5 cm) équilibrée dans un tampon phosphate 0,1 M contenant 0,14 M de NaCl, débit :0,2 ml/min. Les fractions contenant la protéine recombinante (déterminée par analyse électrophorétique sur gel de polyacrylamide en présence de SDS) sont rassemblées.

La solution ainsi obtenue a été soumise à une analyse électrophorétique sur gel de polyacrylamide en présence de SDS et révélation au nitrate d'argent. On observe les deux formes majoritaires de la protéine NC30 correspondant à des masses moléculaires apparentes de 9 ± 2 kDa et 16 ± 2 KDa, avec un taux de pureté supérieur à 70 %. Cette solution a été utilisée dans les tests d'activité biologique décrits ci-après.

Dans une autre expérience, une étape d'HPLC phase inverse sur colonne C4 (Brownlee) avec comme éluant un gradient linéaire d'acétonitrile / 0,1 % TFA (acide trifluoroacétique), de 30 % à 70 %, a été introduite dans ce protocole de purification, permettant d'obtenir un produit présentant un taux de pureté supérieur à 90 % (estimation par analyse électrophorétique sur gel de polyacrylamide en présence de SDS et révélation au nitrate d'argent).

2) Détermination de la séquence aminoterminale des deux formes majoritaires de la protéine NC30

La protéine purifiée a été soumise à une électrophorèse sur gel de polyacrylamide 16 % en présence de SDS. Les protéines du gel sont transférées sur une membrane Immobilon (Millipore) à 0,8 mA/cm$^2$ pendant 1 h dans un tampon de composition tris-borate 25 mM pH9,0 et méthanol 10 %.

Les deux bandes correspondant aux masses moléculaires apparentes de 9 ±2 kDa et 16 ± 2 kDa ont été découpées et déposées sur un séquenceur Applied Biosystems modèle 470 A, couplé à un analyseur de dérivés phénylthiohidantoïques Applied Biosystems, modèle 120 A.

Ces deux bandes présentent la même séquence amino-terminale (tr$_1$) :

$$\text{Ser Pro Gly Pro Val Pro Pro Ser Thr Ala Leu,}$$
$$1 \qquad\qquad 5 \qquad\qquad 10$$

laquelle est la séquence aminoterminale attendue : celle de la protéine mature de 114 acides aminés (cf. figure 2) décrite dans la section 5, dont la séquence codante est introduite dans le vecteur pEMR673 décrit dans la section 9.

3) Détermination de la carte peptidique de la forme de la protéine NC30 de masse moléculaire apparente 9 ± 2 kDa

La protéine de masse moléculaire apparente 9 ± 2 kDa a été digérée dans le gel par la trypsine porcine et les peptides séparés par HPLC phase inverse dans les conditions suivantes :

Le surnageant de levure obtenu dans la section 11 a été précipité à l'acide trichloroacétique et le précipitat, après solubilisation à 100°C dans un tampon contenant du SDS, soumis à une électrophorèse sur gel de polyacrylamide. Les protéines du gel ont été révélées à l'aide de bleu de Coomassie. La bande de masse moléculaire apparente 9 ± 2 kDa a été découpée du gel et digérée par la trypsine porcine dans le gel selon la méthode décrite dans l'article de J. Rosenfeld et al, en cours de publication, "In gel digestion of proteins for internal sequence analysis after one or two dimensional gel electrophoresis"

Puis les peptides tryptiques ont été séparés par chromatographie HPLC phase inverse sur colonne Altex C18 (0,21 x 25 cm) Beckman avec un gradient de 1 à 70 % d'acétonitrile dans une solution de TFA 0,1 % pendant 60 min. Les pics sont détectés par mesure de la densité optique à 218 nm.

Deux fractions correspondant chacune à un pic, appelées ci-après première fraction et deuxième fraction, ont été anlysées à l'aide d'un séquenceur Applied Biosystems modèle 470 A, comme précédemment.

Les séquences aminoterminales obtenues sont les suivantes :
- pour la premièe fraction :

Val Ser Ala Gly Gln Phe Ser Ser Leu His Val

qui correspond aux acides aminés 108-118 de la protéine NC30 traduite (cf. fig. 2)
- pour la deuxième fraction :

Ser Pro Gly Pro Val Pro Pro Ser Thr Ala Leu

et

Lys Leu Phe

qui correspondent respectivement aux acides aminés 33-43 et 138-140 de la protéine NC30 traduite (cf. fig. 2).

**SECTION 13** : Expression cytoplasmique de la protéine NC30 dans E. coli et préparation d'un immunsérum

1) Expression cytoplasmique de la protéine NC30 dans E. coli :

Un vecteur d'expression de la protéine NC30 mature de 112 acides aminés méthionylée dans E. Coli , dénommé pSE714.12 a été construit par insertion dans le vecteur pET3a ouvert aux sites NdeI et BamhI, d'un fragment d'ADN portant une partie de l'ADNc NC30. Ce vecteur d'expression comprend de 5' vers 3' :
- le promoteur de l'ARN polymérase du phage T7, contenu dans le plasmide pET3a, décrit par Rosenberg et al, Gene, 56, 125-135
- la partie de l'ADNc NC30 qui code pour la protéine mature de 112 acides aminés (cf. section 5), précédée d'un ATG initiateur de la traduction
- le terminateur du gène 10 du phage T7 (Studier et al, 1986, J. Mol. Biol.,189, 113-130).

Cette cassette d'expression ne fonctionne qu'en présence de l'ARN polymérase spécifique du phage T7. Il convient donc de faire synthétiser cette ARN polymérase à la souche E. Coli hôte. Une cassette d'expression de cet ARN polymérase a été construite en plaçant la séquence codante de cet enzyme (clonée dans l'ADN du phage lambda CE6 par Studier et al, 1986, J. Mol. Biol, 189, 113-130) sous la dépendance du promoteur PR de lambda. Cette cassette d'expression comprend également un allèle de CI (CI857) codant pour une forme thermosensible du répresseur de ce promoteur PR (P. Leplatois et al, 1983, Biochimie, 65, 317-324). Par conséquent, à basse température, la cassette d'expression de l'ADN polymérase est réprimée, à haute température, l'expression est déréprimée. Cette cassette d'expression a été cloné dans un vecteur d'intégration pEJL407 dérivé des plasmides de N. Kleckner (1984, Gene, 32, 369-379). Le vecteur obtenu est le plasmide pEMR648. Ce vecteur est maintenu à l'état épisomique dans la cellule mais il provoque l'intégration d'une ou de plusieurs copies de la cassette d'expression de la polymérase (y compris le répresseur CI) lorsqu'on induit la transposition avec de l'IPTG (isopropyl-$\beta$-thiogalactoside). La transposase responsable de cette intégration est sous contrôle du gène lacI mais n'est elle-même pas transposée, ce qui permet d'obtenir des intégrants stables après curage du plasmide. En transformant la souche de E. Coli K12 HB101 (Gibco BRL - réf. 8260 SA) par pEMR648, puis en provoquant les évènements d'intégration sur les transformés on a obtenu un dérivé de HB101, appelé VG112, qui comprend 2 cassettes d'expression de la polymérase du phage T7 sous le contrôle du système PL-CI thermosensible, intégrées dans le chromosome. La souche VG112 E. Coli a été curée du plasmide pEMR648 et transformée, à basse température (30°C) par le plasmide pEMR714.

Le transformé retenu, appelé souche VG112 pSE714.12, et déposé à la CNCM le 20 Décembre 1991 sous le n° I-1162 a été cultivé sur du milieu LB contenant de l'ampicilline à 100 $\mu$g/ml à 30°C jusqu'à une DO à 600 nm de 1. Puis l'expression du gène de la polymérase a été induite à l'aide de l'IPTG à 41°C pendant 2 h. L'analyse d'un extrait total cellulaire sur gel polyacrylamide dénaturant a permis de mettre en évidence une protéine de 9 kDa correspondant à une bande surnuméraire par rapport à la souche VG112 curée et non transformée (souche témoin). Une lyse des cellules par sonication suivie d'une centrifugation permet de séparer l'extrait cellulaire en 2 fractions : une fraction soluble (surnageant) et une fraction insoluble (culot). La protéine NC30 se retrouve avec les protéines de la fraction insoluble et réprésente environ 50 % (en masse) des protéines de cette fraction.

2) Préparation d'un immunsérum reconnaissant la protéine NC30

Cette fraction insoluble a été utilisée pour immuniser un lapin (mâle de 2 Kg environ, New-Zealand). Les immunisations ont été effectuées tous les 15 jours selon le protocole décrit par Vaitukaitis, 1981, Methods in Enzymology, 73, 46. Pour la première injection, un volume de solution antigénique est émulsifié par un volume d'adjuvant complet de Freund (Sigma- réf.4258). 6 rappels ont été administrés en adjuvant incomplet de Freund (Sigma- réf.5506).

L'immunsérum obtenu est capable de reconnaître la protéine NC30 produite par la levure et par les cellules COS par immunodétection après électrophorèse sur gel de polyacrylamide en présence de SDS.

3) Caractérisation de la protéine NC30 par carte peptidique

La fraction insoluble obtenue en 1) est soumise à une électrophorèse sur gel de polyacrylamide en présence de SDS. Les protéines du gel sont révélées par coloration au bleu de Coomassie. La bande correspondant à une masse moléculaire apparente de 9 ± 2 kDa a été découpée du gel et digérée par la trypsine porcine dans le gel et les peptides tryptiques ont été séparés comme décrit dans la section 12-3).

Une fraction correspondant à un pic a été analysée à l'aide d'un séquenceur Applied Biosystems modèle 470A.

La séquence aminoterminale obtenue est la suivante :

Val Ser Ala Gly Gln Phe Ser Ser Leu N Val Arg

dans laquelle N représente un acide aminé non déterminé.

Cette séquence correspond aux acides aminés 108-119 de la protéine NC30 traduite (cf. fig. 2) et au peptide de la première fraction analysée dans la section 12.

**SECTION 14** : Mise en évidence pour la protéine NC30 d'une activité inhibitrice sur la production des ARN messagers de l'IL-1 $\beta$ et de l'IL-6 par des monocytes du sang périphérique stimulés par LPS

1) - Méthode utilisée

a) Préparation des cellules

A partir d'une poche de sang périphérique (prélevé sur un volontaire sain dans un centre de transfusion sanguine) on enlève la plupart des hématies par sédimentation à 37°C pendant 30 min dans un milieu contenant 0,6 % dextrane, 0,09 % NaCl. Par la suite les cellules sont déposées par dessus une couche de Ficoll-Paque (Pharmacia) et centrifugées à 400 g pendant 30 min. Les cellules mononucléaires du sang périphérique (PBMNC), qui sont présentes à l'interface entre le Ficoll et le surnageant, sont prélevées. Les PBMNC sont placées dans du milieu RPMI (milieu RPMI 1640-Gibco BRL) contenant 10 % de sérum de veau foetal (SVF), sur des boîtes de culture de 15 cm de diamètre, à raison de 1 à 5 x 10$^7$ cellules par boîte. Après 30 min le milieu est aspiré et les cellules adhérentes sur la boîte (principalement constituées de monocytes) sont incubées, comme décrit ci-dessous.

b) Incubation des cellules avec du LPS et la protéine NC30

Les PBMNC adhérentes sont incubées dans 20 ml de RPMI/10 % SVF pendant 4 h à 37°C sous une atmosphère contenant 5 % $CO_2$ en présence de 5 $\mu$g par ml de Lipopolysaccharide LPS (réf. L4391- Sigma) et de concentrations croissantes de la protéine NC30 issue de levure purifiée (0,1 à 10 ng/ml), ou de surnageants de cellules COS soit transfectées avec le plasmide pSE1 NC30 et cultivées comme décrit dans la section 7, soit transfectées par le plasmide pSE1 et cultivées dans les mêmes conditions (témoin).

c) Préparation et analyse des ARN

Les cellules sont lavées avec du PBS, puis grattées directement dans 1 ml du tampon D (de composition : 4M thiocyanate de guanidinium, 25 mM citrate de sodium, 0,5 % sarcosyl, 0,1 M $\beta$-mercaptoéthanol : Chomczynski P. et Sacchi N. (1987), Anal. Biochem., 162, 156-159). L'ARN est préparé par la méthode d'extraction phénolique à pH acide décrite par ces auteurs. On dépose entre 1 à 5 $\mu$g d'ARN sur gel d'agarose 1 %, en présence de formaldéhyde (Sambrook et al, op cité). Après migration, les ARN sont transférés sur une membrane de nitrocellulose renforcée (Schleicher et Schuell) et hybridés avec des sondes ADNc radiomarquées comme dans la section 4. Les intensités d'hybridation de chaque ARN avec les différentes sondes sont quantifiées par analyse de phosphorescence sur un appareil Phosphorimager (Molecular Dynamics, 800E. Arques Avenue, Sunnyvale, Ca, 94086- Etats-Unis).

2) - Résultats

Les moyennes et les écarts types des résultats obtenus avec la protéine NC30 purifiée dans quatre expériences, sont rassemblés dans le tableau 3 ci-dessous, dans lequel les quantités d'ARN messagers mesurées par analyse de phosphorescence sont exprimées en pourcentage par rapport à la quantité d'ARN messager mesurée pour l'échantillon provenant des cellules traitées avec LPS seul.

Tableau 3

| Quantité des ARN messagers de l'IL-1$\beta$ et de l'IL-6 mesurées pour différentes concentrations de la protéine NC30 | | |
|---|---|---|
| | Quantité d'ARN messagers | |
| | IL-1 $\beta$ | IL-6 |
| condition de stimulation des cellules | | |
| LPS | 100 | 100 |
| LPS + protéine NC30 à la concentration de 0,1 ng/ml | 97 ± 36 | 77 ± 18 |
| LPS + protéine NC30 à la concentration de 1 ng/ml | 54 ± 39 | 24 ± 11 |
| LPS + protéine NC30 à la concentration de 10 ng/ml | 30 ± 5 | 13 ±4 |

On constate à la lecture du tableau ci-dessus, que l'accumulation des ARN messagers de l'IL-1$\beta$ et de l'IL-6 dans les monocytes traités au LPS est inhibée par la protéine NC30. L'inhibition la plus importante est observée pour une concentration de 10 ng/ml de la protéine NC30, et la dose pour obtenir une inhibition à 50 % (IC 50) est est de l'ordre de 1 ng/ml.

On constate de même une inhibition de la production des ARN messagers de l'L-1$\beta$ et de l'IL-6, en présence des surnageants de cellules COS transfectées par le plasmide pSE1-NC30 et aucune inhibition en présence des surnageants de cellules COS témoins.

On a également constaté lors d'autres expériences une inhibition de la production des protéines IL-1$\beta$ et IL-6 dans les milieux de culture des monocytes traités au LPS en présence de la protéine NC30. L'IL-6 a été dosée par son effet sur la prolifération de la lignée d'hybridome B9 selon la méthode décrite par L.A. Aarden, 1987, Eur. J. Immun., 17, 1411-1416. (La quantité de la protéine NC30 présente dans les échantillons n'interfère pas avec le dosage par la lignée B9 des quantités d'IL-6 produites par les monocytes). Le dosage de l'IL-1$\beta$ a été effectué sur cellules EL4 selon la méthode décrite par E. W. Palaszynski, 1987, Biochem and Biophys. Res. Comm., 147, p. 204-211, qui consiste à mesurer la compétition de la liaison avec de l'IL-1$\beta$ radiomarqué.

**SECTION 15** : Mise en évidence pour la protéine NC30 de la modulation de la quantité de l'antigène de surface CD23 par les cellules B d'amygdales

1) Méthode utilisée

a) préparation des cellules

Les amygdales humaines ont été prélevées après intervention chirurgicale sur une fillette âgée de 6 ans. Les amygdales ont été dilacérées au scalpel dans du milieu RPMI refroidi à 4°C. Les cellules relarguées dans le milieu après cette opération sont filtrées sur de la gaze de manière à former une suspension cellulaire homogène. Après deux lavages, les cellules sont numérées et reprises dans du sérum de veau foetal contenant 10 % de DMSO (Merck). 7,5.10$^6$ cellules sous un volume de 1 ml sont distribuées dans chaque tube de congélation. Les cellules sont placées dans un thermos à -80°C pendant 24 h puis stockées dans de l'azote liquide.

b) Incubation des cellules avec la protéine NC30

Le jour de l'expérience, une partie aliquote des cellules est décongelée à 37°C, puis diluée lentement dans 50 ml de milieu RPMI contenant 10 % de sérum de veau foetal. Les cellules sont centrifugées pour éliminer le DMSO. Après comptage des cellules, 100 $\mu$l d'une suspension cellulaire réajustée à $4.10^6$ cellules/ml sont distribuées dans des plaques de microtitration de 96 puits (NUNC).

La protéine NC30 purifiée est ajoutée à différentes concentrations dans du milieu RPMI contenant 10 % de sérum de veau foetal. 100 $\mu$l des différentes concentrations sont rajoutés aux cellules dans les puits de microculture. L'incubation se poursuit pendant 48 h à 37°C en atmosphère contenant 5 % de $CO_2$.

c) Marquage des cellules pour l'immunofluorescence

Après incubation, les cellules sont transvasées dans des tubes micronics (Labsystem). Pour l'analyse en simple-immunofluorescence, 10 $\mu$l d'anticorps anti-CD23 couplé au FITC (isothiocyanate de fluoresceine) (Immunotech) sont rajoutés à la suspension cellulaire. Pour l'analyse en double-immunofluorescence,10 $\mu$l d'anticorps anti-CD23 couplé à la phycoérythrine et 10 $\mu$l d'anticorps anti-CD20 couplé au FITC (Becton Dickinson) sont rajoutés simultanément à la suspension cellulaire.

L'incubation se poursuit pendant 30 min à 4°C, puis les cellules sont centrifugées et le culot repris par 250 $\mu$l de tampon PBS froid. 50 $\mu$l d'une solution d'iodure de propidium (Sigma) à 20 $\mu$g/ml sont rajoutés dans le but de distinguer les cellules mortes lors de l'analyse.

d) Analyse en cytométrie en flux

Les échantillons sont analysés par dosage de fluorescence sur un trieur de cellules FacStar Plus (Becton Dickinson) avec une longueur d'onde laser d'excitation de la fluorescence de 488 nm. Lors de l'analyse en simple-immunofluorescence, les émissions du FITC et de l'iodure de propidium sont collectées en utilisant respectivement des filtres interférentiels de 530 nm et 630 nm. Lors de l'analyse en double-immunofluorescence, l'émission supplémentaire due à la phycoérythrine est collectée à travers un filtre interférentiel de 575 nm. Pour cette dernière analyse, un système de compensation électronique est utilisé pour éviter la contamination de la fluorescence du FITC dans le canal de la phycoérythrine, de la fluorescence de la phycoérythrine dans le canal de l'iodure de propidium et de la fluorescence de l'iodure de propidium dans le canal de la phycoérythrine. Les résultats sont collectés et traités en utilisant le logiciel LysIS II (Becton Dickinson).

2) Résultats

a) Modulation de la quantité de l'antigène CD23 sur cellules d'amygdales par la protéineNC30

Les moyennes et les écarts types des résultats obtenus sont rassemblés dans le tableau 4 ci-après, pour une incubation des cellules d'amygdales pendant 48 h et une gamme des concentrations de la protéine NC30 de $10^{-3}$ à $10^2$ ng/ml

Tableau 4

| Variation du pourcentage des cellules d'amygdales exprimant l'antigène CD23 en présence de différentes concentrations de la protéine NC30 | |
| --- | --- |
| **Concentration en NC30 (ng/ml)** | **% des cellules exprimant CD23** |
| 0 | 4,8 ± 1,2 |
| $10^{-3}$ | 4,8 ± 0,8 |
| $10^{-2}$ | 4,9 ± 0,4 |
| $10^{-1}$ | 12,0 ± 2,4 |
| 1 | 17,0 ± 2,5 |
| 10 | 27,3 ± 1,6 |
| $10^2$ | 20,4 ± 2,5 |

On constate que le pourcentage des cellules d'amygdales exprimant l'antigène CD23 (récepteur de faible affinité aux IgE) est plus important pour une concentration de la protéine NC30 supérieure à $10^{-1}$

ng/ml que le pourcentage obtenu en l'absence de la protéine NC30. L'effet le plus important de cette protéine est constatée à une concentration de 10 ng/ml.

b) Caractérisation des cellules sur lesquelles la protéine NC30 module l'expression de l'antigène CD23

La caractérisation des cellules qui expriment l'antigène CD23 a été réalisée en double-immunofluorescence en utilisant un anticorps anti-CD23 couplé à la phycoérythrine et un anticorps anti-CD20 couplé au FITC. Ce dernier est dirigé contre un récepteur présent uniquement sur les cellules B. L'analyse en cytométrie en flux montre que seule une fraction des cellules B exprime l'antigène CD23 sous l'action de la protéine NC30.

**SECTION 16** : Mise en évidence de l'action de la protéine NC30 sur la prolifération de la ligne d'hybridome B9

L'activité de stimulation de la prolifération de la lignée d'hybridome B9 a été mise en évidence avec des surnageants de culture de cellules COS transfectées par le plasmide $pSE_1$-NC30 (cf. section 7), et avec la protéine NC30 purifiée issue de la levure (cf. section 12). Cette lignée est utilisée d'habitude pour effectuer un dosage biologique de l'IL-6 (L.A. Aarden, 1987, Eur. J. Immun., 17, 1411-1416).

1) - Méthode utilisée

a) Principe du dosage

Le principe de ce dosage est décrit par T. Mosman, 1983, J. Immun. Methods, 65, 55-63 et résumé ci-après :

Les mitochondries renferment des déshydrogénases multiples capables de réduire le cycle tétrazolium en formazan. Un sel de ce type, le MTT (bromure de 3- (4,5 - diméthyl thiazol - 2,5 - diphényl tétrazolium) ainsi réduit donne une coloration bleue, avec une forte absorption vers 565 nm. Le dosage colorimétrique décrit ici permet de mesurer quantitativement le nombre de mitochondries, donc le nombre de cellules.

b) Culture des cellules

Les cellules utilisées appartiennent à la lignée B9, lignée hybridome souris/souris décrite par L.A. Aarden, 1987, Eur. J. Immunol., 17, 1411-1416. Ce sont des cellules non adhérentes qui prolifèrent en présence d'IL-6 murine ou d'IL-6 humaine.

**Milieu de culture**

500 ml de milieu RPMI 1640 sans glutamine
réf. 041-01870M (Gibco)
+ 50 ml de sérum de veau foetal
(décomplémenté, c'est-à-dire chauffé à 55°C pendant 30 min pour inactiver les fragments du complément du sérum)
(Sigma réf. F 4135)
+ 12,5 ml de Pyruvate de sodium 100 mM
réf. 043-01360H (Gibco)
+ 2,5 ml d'Hépes 1 M pH 7,3
réf. 043-05630D (Gibco)
+ 10 ml de Glutamine 200 mM
réf. 043-05030D Gibco
Extemporanément, on rajoute :
- le $\beta$-Mercaptoéthanol (Sigma réf. M-6250) $5 \times 10^{-5}$ M final ;
- de l'IL-6 à la concentration finale de 500 pg/ml.

c) Préparation des échantillons :

Deux types d'échantillons ont été utilisés, l'un obtenu à partir du surnageant de cellules COS, soit transfectées par le plasmide $pSE_1$ NC30 et cultivées comme décrit dans la section 7, soit transfectées avec

le plasmide $pSE_1$ et cultivées dans les mêmes conditions (témoin), l'autre à partir de la solution de la protéine NC30 issue de levure purifiée dans la section 12, à la concentration de 50 ng/ml.

d) Protocole de dosage

Le dosage est effectué dans des plaques de culture de 96 puits à fonds plats, chaque échantillon étant dosé sur une rangée de 12 puits à des concentrations variables.

Les cellules de la lignée B9 sont mises en culture, lavées 2 fois avec du milieu de culture sans IL-6, remises en suspension dans du milieu de culture (sans IL-6) et incubées pendant 2 h à 37°C. Cette incubation permet de mieux éliminer l'IL-6, responsable de bruit de fond dans le dosage. Finalement, les cellules sont à nouveau centrifugées et remises en suspension dans le milieu de culture ci-dessus (sans IL-6), à la concentration de $2.10^5$ cellules/ml.

On distribue successivement dans les plaques de 96 puits :
- 50 $\mu$l du milieu de culture (sans IL-6) dans chaque puits (sauf dans les premiers de chaque ligne)
- 100 $\mu$l de l'échantillon à tester dans le premier puits de chaque ligne (avec une dilution d'un facteur 2 de puits en puits)
- 50 $\mu$l de la suspension cellulaire dans chaque puits (10 000 cellules par puits).

Les plaques sont ensuite placées dans une étuve à 37°C sous une atmosphère contenant 5 % de $CO_2$.

Après 3 jours d'incubation, on ajoute dans chaque puits de façon stérile 10 $\mu$l d'une solution de MTT (Sigma-Réf. 2128) à 5 mg/ml dans du tampon PBS. La plaque est remise à l'étuve. Au microscope, on peut suivre l'apparition du formazan qui est produit sous forme de cristaux bleutés par les cellules survivantes. Au bout de quatre heures, les cellules sont mortes, le surnageant de chaque puits est aspiré avec précaution et les cristaux sont dissous avec 100 ml d'une solution de 66 % de n-propanol contenant 10 % de SDS et 0,04 N de HCl. Les plaques sont mises quelques instants sur un agitateur de plaques afin d'homogénéiser la coloration. Puis la lecture est effectuées à l'aide d'un lecteur de plaques à une longueur d'onde de 565 nm.

A la place du dosage à l'aide du MTT décrit ci-dessus, on peut aussi effectuer un comptage cellulaire au microscope.

2) Résultats

a) Surnageant de cellules COS contenant la protéine NC30

On a réalisé 6 séries de mesures : 3 avec différents surnageants de cellules COS contenant la protéine NC30 et 3 avec différents surnageants de cellules COS témoins, avec pour chaque série, différents facteurs de dilution de la solution de surnageant COS.

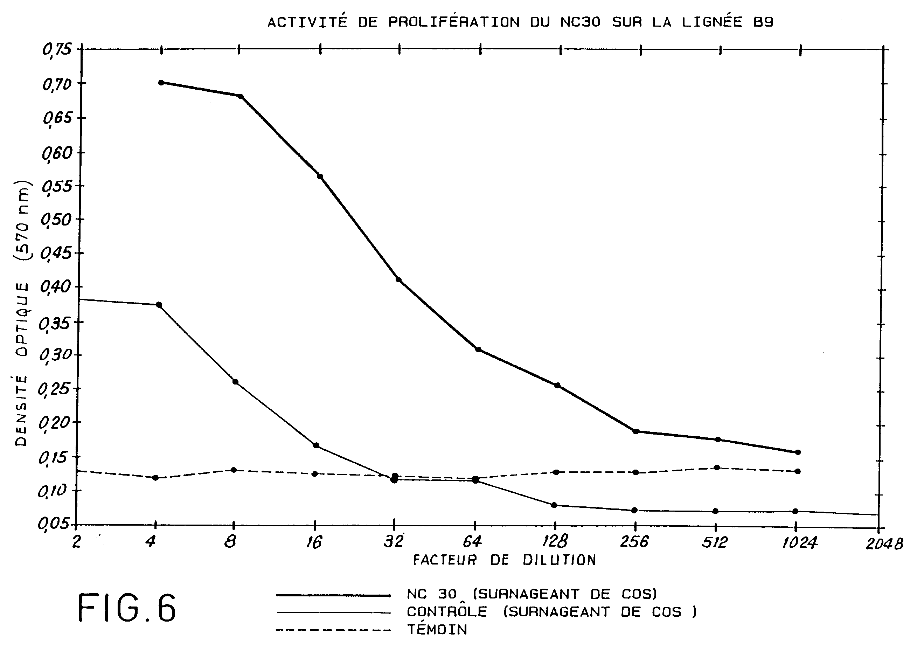

Les résultats obtenus sont représentés sur la figure 6, qui représente la variation de la densité optique (chaque point est la moyenne de la densité optique mesurée pour le même facteur de dilution dans les 3 séries d'expériences) en fonction du facteur de dilution de la solution de surnageant COS.

On constate à la lecture de cette figure que la solution de surnageant COS contenant la protéine NC30 est de 4 à 6 fois plus active dans la prolifération de la lignée B9 que la solution de surnageant COS témoin.

L'activité de stimulation de la prolifération de la lignée B9 constatée avec la solution de surnageant COS témoin est due à la production endogène d'IL-6 par les cellules COS, laquelle peut être quantifiée également par dosage RIA (Radio-Immuno-Assay), notamment à l'aide du kit d'Amersham- réf : RPA 537. Ce dosage RIA a permis de vérifier que le surcroît d'activité de stimulation de la prolifération de la lignée B9 (par le surnageant COS contenant la protéine NC 30) n'était pas liée à une surproduction d'IL-6 par cellules COS.

b) Protéine NC30 purifiée issue de la levure :

On a réalisé deux séries de mesures, l'une de la densité optique après coloration avec le MTT, l'autre de la densité cellulaire par comptage des cellules aux microscope, avec, pour chaque série, différentes concentrations de la protéine NC30.

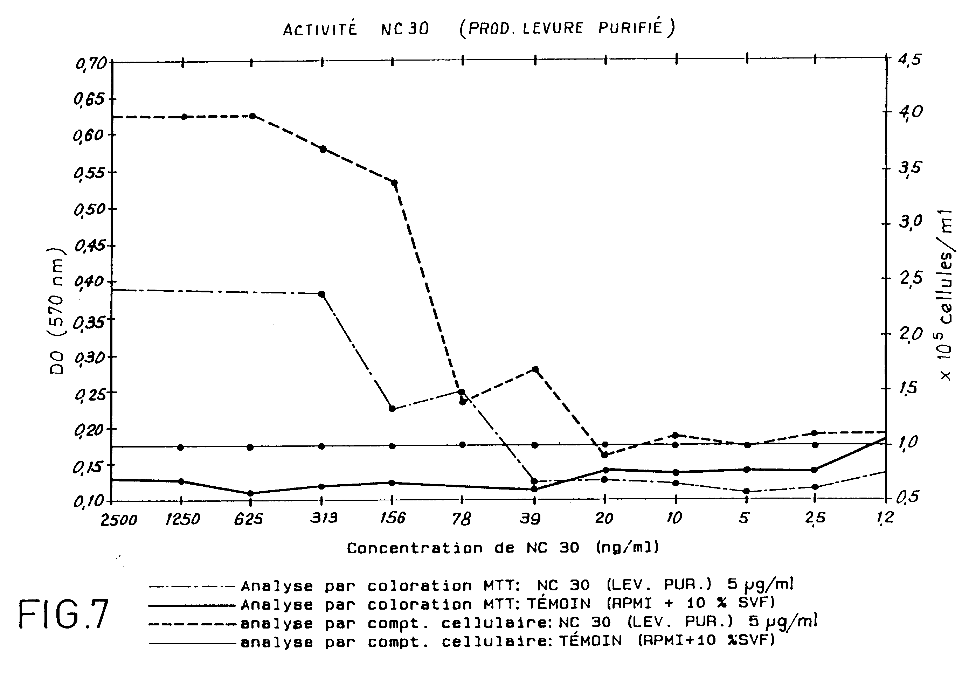

Les résultats obtenus sont rassemblés dans la figure 7, qui représente la variation de la densité optique et de la densité cellulaire en fonction de la concentration en protéine NC30 exprimée en ng/ml.

On constate que la protéine NC30 purifiée stimule la prolifération de la lignée B9. L'$ED_{50}$ (concentratin à laquelle on constate une activité égale à la moitié de l'activité maximum obtenue) est de l'ordre de 100 ng/ml.

Remarquons que les cellules B9 sont des cellules de souris, ce qui peut expliquer la nécessité d'utiliser des concentrations élevées de la protéine NC30 par rapport aux concentrations utilisées pour une action sur les cellules humaines (cf. sections 14, 15 et 17).

**SECTION 17** : Mise en évidence de l'action de la protéine NC30 sur la prolifération de la lignée mégakaryoblastique humaine MO7e en présence de GMCSF

L'augmentation de l'activité proliférative du GMCSF sur la lignée mégakaryoblastique humaine MO7e a été mise en évidence avec la protéine NC30 produite dans la levure (section 12). Cette lignée cellulaire décrite par M.F. Brizzi et al., 1990, British Journal of Haematology, 76, 203-209, est strictement dépendante pour sa croissance des cytokines IL3 ou GMCSF.

1) Méthode utilisée

a) But

Il s'agit de comparer la prolifération des cellules de la lignée MO7e cultivées, soit en présence d'un quantité de GMCSF nécessaire à la moitié de la prolifération maximale, soit en présence de cette même quantité de GMCSF à laquelle on rajoute la protéine NC30 en concentration variable.

b) Principe du dosage

La prolifération cellulaire est déterminée par la mesure en radioactivité de l'incorporation de thymidine tritiée par les cellules en culture.

Les cellules proliférantes utilisent la thymidine pour leur synthèse d'ADN. La thymidine tritiée introduite dans la culture va entrer en compérition avec la thymidine "froide" du milieu et être incorporée dans les cellules.

Après un temps déterminé, les cellules sont récupérées sur un filtre et lavées pour enlever l'excès de thymidine tritiée non incorporée dans les cellules. Chaque filtre est ensuite analysé à l'aide d'un compteur gamma. L'activité proliférative est exprimée en nombre de dmp de la thymidine tritiée incorporée.

c) Culture de cellules

Les cellules utilisées appartiennent à la lignée MO7e, lignée mégakaryoblastique humaine établie par M.F. Brizzi et al. (référence citée ci-dessus). Ce sont des cellules non adhérentes qui prolifèrent en présence d'IL3 humaine ou de GMCSF humains. La moitié de l'activité maximum obtenue ($ED_{50}$) est de :

35 pg/ml pour le GMCSF (Genzyme réf. RM-CSF-C)
0,7 pg/ml pour l'IL3 (Genzyme réf. HIL3 C).

Milieu de culture

- 500 ml de Milieu de Dulbecco modifié par Iscove (milieu IMDM- Gibco - réf. 04101980)
- 50 ml de sérum de veau foetal (décomplémenté, c'est-à-dire chauffé à 55°C pendant 30 min pour inactiver les fragments du complément de sérum) (Sigma réf. F4135)
+ 10 mg/ml de Gentamycine (1 ml de la solution Gibco de réf - 043.05710 D)
Extemporanément, on rajoute de l'IL3 humaine recombinante (Genzyme réf. HIL3.C) à la concentration finale de 4 ng/ml.

d) Préparation des échantillons

Les échantillons à tester sont préparés (par dilution dans le milieu de culture dépourvu d'IL3) à partir d'une solution de protéine NC30 issue de levure purifiée dans la section 12, à la concentration de 500 ng/ml.

e) Protocole de dosage

Le dosage est effectué dans des plaques de culture de 96 puits à fonds plats, chaque échantillon étant dosé sur une rangée de 12 puits à des concentrations variables. Les cellules de la lignée MO7e mises en culture doivent être en phase exponentielle de croissance.

Pour le dosage, les cellules sont lavées 2 fois avec le milieu de culture sans IL3 et incubées pendant 3 h à 37°C. Cette incubation permet de mieux éliminer l'IL3, responsable de bruit de fond dans le dosage. Enfin, les cellules sont à nouveau centrifugées et remises en suspension dans le milieu de culture ci-dessus (sans IL3) à la concentration de $2.10^5$ cellules/ml.

On distribue successivement dans les plaques de 96 puits :

- soit 50 $\mu$l de milieu de culture (sans IL3) dans chaque puits ;
- soit 50 $\mu$l de milieu de culture (sans IL3) et 10 $\mu$l d'une solution GMCSF à 200 pg/ml ;
- soit 50 $\mu$l de l'échantillon à tester à différentes concentrations et 10 $\mu$l d'une solution de GMCSF à 200 pg/ml ;
- puis 50 $\mu$l de suspension cellulaires dans chaque puits (10 000 cellules/puits).

Les plaques sont ensuite placées dans une étuve à 37°C sous une atmosphère contenant 5 % de $CO_2$.

Après 3 jours d'incubation, on ajoute dans chaque puits, de façon stérile, 50 $\mu$l d'une solution de thymidine tritiée (10 $\mu$Ci/ml) (Amersham Réf. TRA6 = 1 mCi/ml à 10 $\mu$Ci/ml) dans du milieu de culture sans IL3. La plaque est remise à l'étuve. Au bout de 4 h, le contenu de chaque puits est déposé sur un filtre par aspiration des puits et lavage à l'eau distillée et la radioactivité du filtre est mesurée.

2) Résultats

Les principaux résultats obtenus sont rassemblés dans le tableau 5 ci-dessous, dans lequel figurent la valeur de la radioactivité exprimée en dmp pour le milieu sans GMCSF et sans protéine NC30 et la valeur de la radioactivité pour le milieu contenant 18 pg/ml de GMCSF en fonction de la concentration en protéine NC30.

Les valeurs de radioactivité présentées ici sont les moyennes de 11 essais pour le milieu sans GMCSF ainsi que le milieu contenant uniquement du GMCSF et de 7 essais pour les milieux contenant du GMCSF et de la protéine NC30. Les valeurs de ces moyennes ont été comparées en utilisant le test de Student avec un taux de signification supérieur à 99,95 %.

Tableau 5

| Radioactivité en fonction de la concentration en protéine NC30 | | | | | | | |
|---|---|---|---|---|---|---|---|
| Concentration de la protéine NC30 (ng/ml) | 0 | 0,49 | 1,95 | 7,8 | 31,2 | 125 | 500 |
| Radioactivité (dpm) pour milieu sans GMCSF | 550 | / | / | / | / | / | / |
| Radioactivité (dpm) pour milieu contenant 18 pg/ml de GMCSF | 14462 | 16852 | 18840 | 20264 | 20549 | 20685 | 21784 |
| Ecart significatif avec une probabilité > 99,95 % | / | non | oui | oui | oui | oui | oui |

On constate à la lecture du tableau ci-dessus que la protéine NC30 augmente significativement la prolifération de la lignée MO7e en présence de GMCSF.

**SECTION 18** : Mise en évidence pour la protéine NC30 d'une activité chimiotactique

1) Méthode utilisée

a) Isolement des neutrophiles

A partir du sang périphérique, on enlève la plupart des hématies par sédimentation à 37°C pendant 30 min dans une solution contenant 0,6 % dextrane T500 (Pharmacia -réf : 17-0320-01) et 0,09 % NaCl. Par la suite, les cellules sont déposées par-dessus une couche de Ficoll-Paque (Pharmacia) et centrifugées à 400 g pendant 30 min. Les cellules mononucléaires du sang périphérique (PBMNC) sont présentes à l'interface entre le Ficoll et le surnageant alors que les hématies résiduelles et les polynucléaires (principalement des neutrophiles) se trouvent dans le culot cellulaire. Ce culot est remis en suspension dans une solution de NH₄Cl 0,8 %, 10 mM Hepes et incubé à 37°C pendant 7 min pour faire éclater les hématies. Les cellules résiduelles (principalement des neutrophiles) sont centrifugées et lavées dans du tampon HBSS : Solution saline équilibrée de Hanks (Gibco BRL-Réf. 041-04025 H), appelé ci-après solution HBSS.

b) Isolement des monocytes

Le principe d'isolement des monocytes a été décrit par A. Boyum, 1983, Scan. J. Immunol., 17, 429-436. Il est résumé ci-après. La méthode consiste à séparer les monocytes du sang en utilisant un milieu de gradient iodé, le Nycodenz (N,N'-Bis(dihydroxypropyl-2,3)-[N-(dihydroxypropyl-2,3) acétamido]-5-triodo-isophtalamide-2,4,6). Pour accentuer la différence de densité entre les monocytes et les lymphocytes, l'osmolarité de la solution est augmentée, donc les lymphocytes rejettent de l'eau et deviennent plus denses. On peut utiliser du milieu "NycoPrep 1.068", qui contient du Nycodenz, du chlorure de sodium et de la tricine/NaOH à des concentration optimales pour la séparation des monocytes (Nycomed Pharma AS, Norvège-réf. 223510).

Le protocole utilisé est le suivant :

A partir du sang périphérique, on enlève la plupart des hématies par sédimentation à 37°C pendant 30 min dans une solution contenant 0,6 % de dextrane et 0,09 % NaCl. La phase supérieure du plasma contenant les monocytes, les lymphocytes et les neutrophiles est prélevée. Pour séparer les monocytes des autres cellules, des tubes sont préparés de la façon suivante : 6 ml de plasma sont déposés sur une couche de 3 ml de NycoPrep 1.068 (Nycomed Pharma AS, Norvège, réf. 223510) dans un tube de diamètre 13-14 mm. Après centrifugation à 600 g pendant 15 min, on prélève le plasma clarifié jusqu'à 3-4 mm au-dessus de l'interphase et on recueille le reste du plasma et toute la solution de NycoPrep jusqu'à environ 1 cm au-dessus du culot cellulaire, ce qui permet de ne pas prélever les lymphocytes. La suspension de monocytes recueillie est complétée jusqu'à un volume de 6-7 ml avec une solution de composition : 0,9 % NaCl 0,13 % EDTA 1 % BSA, puis centrifugées pendant 7 min à 600 g.

Les monocytes sont contaminés avec des plaquettes. Pour éliminer ces dernières, on centrifuge puis on enlève le surnageant et on remet en suspension avec la même solution, en répétant ces opérations 3 fois.

Les cellules sont remises en suspension dans du milieu RPMI 1640 (Gibco) contenant de l'albumine sérique bovine (BSA) 0,5 %.

c) Protocole de mise en évidence du chimiotactisme

Le test utilisé est celui décrit par W. Falk et al, 1980, J. Imm. Meth., 33, 239-247. Le protocole précis utilisé est exposé ci-après :

On utilise la chambre de Boyden modifiée pour mesure de chimiotactisme, commercialisée par Neuroprobe (réf. AP48). On met les échantillons à tester dilués dans une solution HBSS pour les tests sur les neutrophiles, et le milieu RPMI contenant 0,5 % BSA pour les tests sur les monocytes, dans les puits de la plaque inférieure. On dépose sur celle-ci une membrane de polycarbonate (taille des pores : 5 mm-Nuclepore réf. 155845) avec le côté brillant en bas. On dépose la plaque supérieure sur la membrane. On met les cellules (50 000 pour 50 μl tampon) dans les puits de la plaque supérieure. On incube la chambre à 37°C dans une étuve humidifiée ou dans une boîte contenant du coton mouillé pendant 1 h pour le test sur les neutrophiles, et 3 h pour le test sur les monocytes. On retire la membrane et on enlève les cellules qui sont sur le côté mat (cellules qui n'ont pas migré) en essuyant la membrane et en le raclant avec un grattoir en caoutchouc, ces deux dernières opérations étant répétées 1 fois. Les cellules qui ont migré sont colorées et fixées en utilisant le kit "Diff-quick" (Dade-réf. 130832). Par observation microscopique, on compte le nombre de cellules sur le côté brillant de la membrane (cellules qui ont migré). On calcule ensuite l'indice de chimiotactisme de l'échantillon vis-à-vis des cellules considérées (monocytes ou neutrophiles), lequel est défini comme le rapport du nombre de cellules qui ont migré vers l'échantillon sur le nombre de cellules qui ont migré dans une expérience témoin vers le milieu ou tampon de dilution.

3) Préparation des échantillons

a) Echantillons de la protéine NC30 recombinante :

protéine NC30 issue de levure, purifiée comme décrit dans la section 12, à des concentrations de 0,1, 1, 10 et 100 ng/ml.

b) Témoin :

Le peptide formyl-Met-Leu-Phe, généralement appelé fMLP (Sigma- réf. F 3506), à une concentration de 1 $\mu$M (concentration employée habituellement pour une utilisation comme témoin positif de chimiotactisme.

4) Résultats :

Les principaux résultats obtenus sont rassemblés dans le tableau 6 ci-après, qui précise l'indice de chimiotactisme vis-à-vis des monocytes et l'indice de chimiotactisme vis-à-vis des neutrophiles pour la protéine NC30 à différentes concentrations et le témoin fMLP. Cet indice a été calculé en faisant la moyenne de quatre expériences indépendantes.

Tableau 6

| Protéine NC30 ng/ml | Indice de chimiotactisme vis-à-vis des monocytes | Indice de chimiotactisme vis-à-vis des neutrophiles |
|---|---|---|
| 0,1 | 2,2 | 0,6 |
| 1 | 4,1 | 1,0 |
| 10 | 6,1 | 1,2 |
| 100 | 5,1 | 1,1 |
| fMLP 1 $\mu$M | 3,0 | 3,8 |

On constate qu'aux concentrations testées, la protéine NC30 n'a pas d'effet significtif sur les neutrophiles mais qu'à une concentration de 1, 10 et 100 nm/ml, celle-ci a un indice de chimiotactisme vis-à-vis des monocytes nettement supérieur à celui du fMLP.

La protéine NC30 est donc un chimioattractant puissant et spécifique des monocytes.

**SECTION 19** : Activité immunomodulatrice in vivo chez la souris

La protéine NC30 purifiée issue de la levure a été testée pour son activité immunomodulatrice dans deux modèles infectieux systémiques chez la souris.

1) Matériel et méthode

a) Animaux

Des souris femelles CD 1 provenant de C. River (France) et ayant un poids moyen de 25 g ont été utilisées dans cette étude. Les lots utilisés comportent 8 ou 10 souris.

b) Souches bactériennes

Une souche Listeria monocytogenes disponible auprès de la Collection de l'Institut Pasteur sous le n° CIP 5734 et une couche E. coli, isolat clinique, conservées à -70°C, ont été les couches infectantes;

c) Echantillons

La protéine NC30 issue de levure purifiée comme décrit dans la section 12 a été utilisée diluée dans une solution de NaCl 0,15 M contenant 1 % en volume de plasma de souris. Cette solution sert de témoin.

d) Traitement des souris

Après répartition au hasard des souris en plusieurs lots, la protéine NC30 a été administrée par voie intrapéritonéale aux doses de 2 et 20 $\mu$g/kg, à des intervalles de temps de 24 ou 4 h avant l'infection. Le lot témoin a été traité par le diluant.

e) Modèles infectieux

Deux modèles infectieux septicémiques faisant appel à des souches E. Coli et L. monocytogenes ont été utilisés. Ces modèles ont été décrits par Kong-Tek Chong, 1987, Infection and Immunity, 1987, 55, 3, p. 668-673 et M. Haakfrendscho et al, Infection and Immunity, 1989, 57, 10, p. 3014-3021.

Les souches E. coli et L. monocytogenes ont été cultivées dans un bouillon nutritif (Nutrient Broth, Oxoid) pendant 18 h à 37°C. Un volume de 0,5 ml d'une dilution appropriée du bouillon de culture correspondant à 5 x 10$^6$ UFC (unité formant colonie) a été administré aux souris par la voie intrapéritonéale. Les mortalités dans les différents lots ont été enregistrées quotidiennement jusqu'au dixième jour.

f) Exploitation statistique

Le nombre de souris survivantes observé dans les lots traités a été comparé à celui du lot témoin par le test du khi2. La différence a été considérée significative lorsque la probabilité était supérieure à 95 %.

2) Résultats

a) Infection par L. Monocytogenes

Les lots de souris traités par voie intrapéritonéale a des intervalles de temps T de 24 ou 4 h avant l'infection par 2 ou 20 µg/kg de la protéine NC30 se sont composés comme les lots témoins. Aucune amélioration du taux de survie n'a été constatée.

b) Infection par E. Coli

Les résultats sont rassemblés dans le tableau 7 ci-après qui précise la proportion de souris survivantes dans les lots testés.

Tableau 7

| Nombre de souris survivantes/nombre de souris infectées par E. Coli selon la dose de protéine NC30 et l'intervalle de temps T séparant le traitement de l'infection. | | | | |
|---|---|---|---|---|
| T | 24 heures | 4 heures | | |
| Dose (µg/kg) | Exp. 1 | Exp. 1 | Exp. 2 | Exp. 3 |
| 0 (témoin) | 3/8 | 1/10 | 1/8 | 2/8 |
| 2 | 3/8 | 5/8* | 3/8 | 2/8 |
| 20 | 7/8* | non testé | 5/8* | 3/8 |

* différence significative par rapport au témoin avec une probabilité supérieure à 95 %.

On constate :

Les souris traitées par voie intrapéritonéale 24 h avant l'infection par 20 µg/kg de la protéine NC30 ont significativement mieux résisté à l'infection microbienne que les souris du lot témoin.

Le traitement préventif administré par voie intrapéritonéale 4 h avant l'infection a été efficace dans deux des trois expériences réalisées et a permis l'augmentation significative du taux de survie des animaux traités par rapport à celui des animaux témoins. Dans une première expérience, la seule dose de 2 µg/kg testée a été active. Dans une seconde expérience, seule la dose de 20 µg/kg a protégé les souris contre l'infection par E. coli.

Dans une autre série d'expériences effectuées sur des lots de 20 souris on a constaté qu'une dose de 32 µg/kg de protéine NC30 issue de levure, purifiée comme décrit dans la section 12, administrée 24 heures avant l'infection par E. coli, permettait un taux de survie nettement plus important des souris : 11 sur 20 contre 2 sur 20 pour le témoin. La différence constatée est significative avec une probabilité supérieure à 99 % selon le test de Student.

La protéine NC30 a donc aussi in vivo un effet immunomodulateur.

La protéine NC30 est donc une nouvelle lymphokine possédant des activités immunomodulatrices de type cytokine in vitro (prolifération cellulaire, activation cellulaire, chimiotactisme et régulation de la synthèse d'autres cytokines) et in vivo. Elle agit sur au moins deux cellules clés du système immunitaire : les monocytes et les lymphocytes B. Il s'agit donc d'une nouvelle interleukine. Certaines de ses propriétés sont en commun avec l'interleukine-4 : inhibition de la synthèse de l'interleukine-1$\beta$ et de l'interleukine-6 par les monocytes humains du sang périphérique activés au LPS et modulation de l'expression de l'antigène CD23 sur les lymphocytes B des amygdales (W. Paul, 1991, Blood, 77, 1959 et Waal Malefyt et al, 1991, J. Exp. Med., 174, 1199-1220).

**Revendications**

**Revendications pour les Etats contractants suivants : AT, BE, CH, DE, DK, FR, GB, IT, LI, LU, NL, PT, SE**

**1.** Protéine à activité de type cytokine, caractérisée en ce qu'elle comprend la séquence ($a_1$) ci-après :

$$
\begin{array}{l}
\text{Gly Pro Val Pro Pro Ser Thr Ala Leu Arg Glu Leu Ile Glu Glu Leu} \\
\qquad 1 \qquad\qquad 5 \qquad\qquad\quad 10 \qquad\qquad\quad 15 \\
\text{Val Asn Ile Thr Gln Asn Gln Lys Ala Pro Leu Cys Asn Gly Ser Met} \\
\qquad\qquad 20 \qquad\qquad\quad 25 \qquad\qquad\quad 30 \\
\text{Val Trp Ser Ile Asn Leu Thr Ala Xaa Met Tyr Cys Ala Ala Leu Glu} \\
\qquad\quad 35 \qquad\qquad\quad 40 \qquad\qquad\quad 45 \\
\text{Ser Leu Ile Asn Val Ser Gly Cys Ser Ala Ile Glu Lys Thr Gln Arg} \\
\qquad\quad 50 \qquad\qquad\quad 55 \qquad\qquad\quad 60 \\
\text{Met Leu Ser Gly Phe Cys Pro His Lys Val Ser Ala Gly Gln Phe Ser} \\
\qquad\; 65 \qquad\qquad\quad 70 \qquad\qquad\quad 75 \qquad\qquad\quad 80 \\
\text{Ser Leu His Val Arg Asp Thr Lys Ile Glu Val Ala Gln Phe Val Lys} \\
\qquad\qquad 85 \qquad\qquad\quad 90 \qquad\qquad\quad 95 \\
\text{Asp Leu Leu Leu His Leu Lys Lys Leu Phe Arg Glu Gly Arg Phe Asn} \\
\qquad\qquad 100 \qquad\qquad\quad 105 \qquad\qquad\quad 110
\end{array}
$$

dans laquelle Xaa représente Asp ou Gly,
ou une séquence présentant un degré d'homologie élevé avec la séquence ($a_1$)

**2.** Protéine selon la revendication 1, caractérisée en ce qu'elle comprend immédiatement en amont de la séquence ($a_1$) la séquence : Ser Pro.

**3.** Protéine selon l'une des revendications 1 et 2, caractérisée en ce qu'elle a une masse moléculaire apparente de 9,0 ± 2 kDa.

**4.** Protéine selon l'une des revendications 1 et 2, caractérisée en ce qu'elle a une masse moléculaire apparente de 16,0 ± 2 kDa.

**5.** Protéine selon l'une des revendications 1 et 2, caractérisée en ce qu'elle est N-glycosylée.

**6.** Protéine selon l'une des revendications 1 et 2, caractérisée en ce qu'elle présente un degré de pureté, déterminé par électrophorèse sur gel de polyacrylamide en présence de SDS et révélation à l'argent, supérieur à 70 %.

**7.** Protéine selon l'une des revendications 1 et 2, caractérisée en ce qu'elle présente un degré de pureté, déterminé par électrophorèse sur gel de polyacrylamide en présence de SDS et révélation à l'argent, supérieur à 90 %.

8. Protéine selon la revendication 1, caractérisée en ce qu'elle comprend un résidu méthionine immédiatement en amont de la séquence ($a_1$).

9. Protéine selon la revendication 1, caractérisée en ce qu'elle comprend en outre une séquence signal de séquence ($b_4$) :

Met Ala Leu Leu Leu Thr Thr Val Ile Ala Leu Thr Cys Leu Gly Gly
1                     5                    10                   15

Phe Ala Ser Pro
20

10. Protéine selon la revendication 9, caractérisée en ce que la séquence ($b_4$) est située immédiatement en amont de la séquence ($a_1$).

11. ADN recombinant, caractérisé, en ce qu'il code pour une protéine selon l'une des revendications 1 et 2 ou pour un précurseur d'une protéine selon l'une des revendications 1 et 2.

12. ADN recombinant, caractérisé en ce qu'il code pour un précurseur d'une protéine selon l'une des revendications 1 et 2, qui comprend une séquence signal.

13. ADN recombinant selon la revendication 12, caractérisé en ce que la séquence codant pour la protéine mature comprend la séquence suivante (Na1):

GGCCCTGTGC CTCCCTCTAC AGCCCTCAGG GAGCTCATTG
AGGAGCTGGT CAACATCACC CAGAACCAGA AGGCTCCGCT
CTGCAATGGC AGCATGGTAT GGAGCATCAA CCTGACAGCT
GACATGTACT GTGCAGCCCT GGAATCCCTG ATCAACGTGT
CAGGCTGCAG TGCCATCGAG AAGACCCAGA GGATGCTGAG
CGGATTCTGC CCGCACAAGG TCTCAGCTGG GCAGTTTTCC
AGCTTGCATG TCCGAGACAC CAAAATCGAG GTGGCCCAGT
TTGTAAAGGA CCTGCTCTTA CATTTAAAGA AACTTTTTCG
CGAGGGACGG TTCAAC

14. ADN recombinant selon la revendication 12, caractérisé en ce que la séquence codant pour la protéine mature comprend la séquence suivante (Na1'):

GGCCCTGTGC CTCCCTCTAC AGCCCTCAGG GAGCTCATTG
AGGAGCTGGT CAACATCACC CAGAACCAGA AGGCTCCGCT
CTGCAATGGC AGCATGGTAT GGAGCATCAA CCTGACAGCT

GGCATGTACT GTGCAGCCCT GGAATCCCTG ATCAACGTGT

CAGGCTGCAG TGCCATCGAG AAGACCCAGA GGATGCTGAG

CGGATTCTGC CCGCACAAGG TCTCAGCTGG GCAGTTTTCC

AGCTTGCATG TCCGAGACAC CAAAATCGAG GTGGCCCAGT

TTGTAAAGGA CCTGCTCTTA CATTTAAAGA AACTTTTTCG

CGAGGGACGG TTCAAC

**15.** ADN recombinant selon l'une des revendications 12 à 14, caractérisé en ce que la séquence signal est une séquence choisie parmi les séquences (b1), (b2), (b3) et (b4) suivantes :

(b1)

Met His Pro Leu Leu Asn Pro Leu Leu Leu Ala Leu Gly Leu Met Ala
1          5                   10              15
Leu Leu Leu Thr Thr Val Ile Ala Leu Thr Cys Leu Gly Gly Phe Ala
           20              25              30

(b2)

Met His Pro Leu Leu Asn Pro Leu Leu Leu Ala Leu Gly Leu Met Ala
1          5                   10              15
Leu Leu Leu Thr Thr Val Ile Ala Leu Thr Cys Leu Gly Gly Phe Ala
           20              25              30
Ser Pro

(b3)

Met Ala Leu Leu Leu Thr Thr Val Ile Ala Leu Thr Cys Leu Gly Gly
1          5                   10              15
Phe Ala

(b4)

Met Ala Leu Leu Leu Thr Thr Val Ile Ala Leu Thr Cys Leu Gly Gly
1          5                   10              15
Phe Ala Ser Pro
           20

**16.** ADN recombinant selon la revendication 15, caractérisé en ce que la séquence nucléotidique codant pour le peptide signal est choisie parmi les séquences (Nb1), (Nb2), (Nb3) et (Nb4) suivantes :

(Nb1)

```
ATGCATCCGC  TCCTCAATCC  TCTCCTGTTG  GCACTGGGCC
TCATGGCGCT  TTTGTTGACC  ACGGTCATTG  CTCTCACTTG
CCTTGGCGGC  TTTGCC
```

(Nb2)

```
ATGCATCCGC  TCCTCAATCC  TCTCCTGTTG  GCACTGGGCC
TCATGGCGCT  TTTGTTGACC  ACGGTCATTG  CTCTCACTTG
CCTTGGCGGC  TTTGCCTCCC  CA
```

(Nb3)

```
ATGGCGCTTT  TGTTGACCAC  GGTCATTGCT  CTCACTTGCC
TTGGCGGCTT  TGCC
```

(Nb4)

```
ATGGCGCTTT  TGTTGACCAC  GGTCATTGCT  CTCACTTGCC
TTGGCGGCTT  TGCCTCCCCA
```

17. Vecteur d'expression, caractérisé en ce qu'il contient, avec les moyens nécessaires à son expression, un ADN recombinant selon l'une des revendications 11 à 16.

18. Cellules eucaryotes, caractérisées en ce qu'elles contiennent, avec les moyens nécessaires à leur expression, un ADN recombinant selon l'une des revendications 11 à 16.

19. Cellules eucaryotes selon la revendication 18, caractérisées en ce qu'elles sont des cellules animales.

20. Cellules animales selon la revendication 19, caractérisées en ce qu'elles contiennent un vecteur d'expression selon la revendication 17.

21. Cellules animales selon la revendication 20, caractérisées en ce qu'elles sont des cellules CHO.

22. Cellules animales selon la revendication 20, caractérisées en ce qu'elles sont des cellules COS.

23. Cellules eucaryotes selon la revendication 18, caractérisées en ce qu'elles sont des cellules de levure.

24. Microorganisme procaryote caractérisé en ce qu'il est transformé par un vecteur d'expression selon la revendication 17.

25. Microorganisme procaryote selon la revendication 24, caractérisé en ce qu'il appartient à l'espèce E. coli.

26. Procédé de préparation d'une protéine selon l'une des revendications 1 à 8, caractérisé en ce qu'il comprend une étape de culture de cellules animales selon l'une des revendications 19 à 22, suivie de l'isolement et de la purification de la protéine recombinante.

27. Procédé de préparation d'une protéine selon l'une des revendications 1 à 8, caractérisé en ce qu'il comprend une étape de culture de cellules de levure selon la revendication 23 suivie de l'isolement et de la purification de la protéine recombinante.

28. Médicament caractérisé en ce qu'il contient une protéine selon l'une quelconque des revendications 1 à 8.

**Revendications pour l'Etat contractant suivant : ES**

1. Procédé d'obtention d'une protéine à activité de type cytokine, qui comprend la séquence ($a_1$) suivante :

$$\text{Gly Pro Val Pro Pro Ser Thr Ala Leu Arg Glu Leu Ile Glu Glu Leu}$$
$$1 \qquad 5 \qquad 10 \qquad 15$$
$$\text{Val Asn Ile Thr Gln Asn Gln Lys Ala Pro Leu Cys Asn Gly Ser Met}$$
$$20 \qquad 25 \qquad 30$$
$$\text{Val Trp Ser Ile Asn Leu Thr Ala Xaa Met Tyr Cys Ala Ala Leu Glu}$$
$$35 \qquad 40 \qquad 45$$
$$\text{Ser Leu Ile Asn Val Ser Gly Cys Ser Ala Ile Glu Lys Thr Gln Arg}$$
$$50 \qquad 55 \qquad 60$$
$$\text{Met Leu Ser Gly Phe Cys Pro His Lys Val Ser Ala Gly Gln Phe Ser}$$
$$65 \qquad 70 \qquad 75 \qquad 80$$
$$\text{Ser Leu His Val Arg Asp Thr Lys Ile Glu Val Ala Gln Phe Val Lys}$$
$$85 \qquad 90 \qquad 95$$
$$\text{Asp Leu Leu Leu His Leu Lys Lys Leu Phe Arg Glu Gly Arg Phe Asn}$$
$$100 \qquad 105 \qquad 110$$

   dans laquelle Xaa représente Asp ou Gly,
   ou une séquence présentant un degré d'homologie élevé avec la séquence ($a_1$), qui consiste à
   (i) cultiver des cellules animales ou de levure qui contiennent un vecteur d'expression contenant, avec les moyens nécessaires à son expression, un ADN recombinant codant pour ladite protéine ou un précurseur de celle-ci,
   (ii) isoler les cellules cultivées, et
   (iii) purifier la protéine recombinante à partir des cellules isolées.

2. Procédé selon la revendication 1, caractérisé en ce que ladite protéine comprend immédiatement en amont de la séquence ($a_1$) la séquence : Ser Pro.

3. Procédé selon l'une des revendications 1 et 2, caractérisé en ce que ladite protéine a une masse moléculaire apparente de 9,0 ± 2 kDa.

4. Procédé selon l'une des revendications 1 et 2, caractérisé en ce que ladite protéine a une masse moléculaire apparente de 16,0 ± 2 kDa.

5. Procédé selon l'une des revendications 1 et 2, caractérisé en ce ladite protéine est N-glycosylée.

6. Procédé selon l'une des revendications 1 et 2, caractérisé en ce que ladite protéine présente un degré de pureté, déterminé par électrophorèse sur gel de polyacrylamide en présence de SDS et révélation à l'argent, supérieur à 70 %.

7. Procédé selon l'une des revendications 1 et 2, caractérisé en ce que ladite protéine présente un degré de pureté, déterminé par électrophorèse sur gel de polyacrylamide en présence de SDS et révélation à l'argent, supérieur à 90 %.

8. Procédé selon la revendication 1, caractérisé en ce que ladite protéine comprend un résidu méthionine immédiatement en amont de la séquence ($a_1$).

**9.** Procédé selon la revendication 1, caractérisé en ce que ladite protéine comprend en outre une séquence signal de séquence (b₄) :

$$\text{Met Ala Leu Leu Leu Thr Thr Val Ile Ala Leu Thr Cys Leu Gly Gly}$$

$$1 \qquad\qquad 5 \qquad\qquad 10 \qquad\qquad 15$$

$$\text{Phe Ala Ser Pro}$$

$$20$$

**10.** Procédé selon la revendication 9, caractérisé en ce que la séquence (b₄) est située immédiatement en amont de la séquence (a₁).

**11.** Procédé selon la revendication 1 ou la revendication 2, caractérisé en ce que l'ADN recombinant code pour un précurseur de ladite protéine, qui comprend une séquence signal.

**12.** Procédé selon la revendication 11, caractérisé en ce que la séquence de l'ADN recombinant codant pour la protéine mature comprend la séquence suivante (Na1):

GGCCCTGTGC CTCCCTCTAC AGCCCTCAGG GAGCTCATTG
AGGAGCTGGT CAACATCACC CAGAACCAGA AGGCTCCGCT
CTGCAATGGC AGCATGGTAT GGAGCATCAA CCTGACAGCT
GACATGTACT GTGCAGCCCT GGAATCCCTG ATCAACGTGT
CAGGCTGCAG TGCCATCGAG AAGACCCAGA GGATGCTGAG
CGGATTCTGC CCGCACAAGG TCTCAGCTGG GCAGTTTTCC
AGCTTGCATG TCCGAGACAC CAAAATCGAG GTGGCCCAGT
TTGTAAAGGA CCTGCTCTTA CATTTAAAGA AACTTTTTCG
CGAGGGACGG TTCAAC

**13.** Procédé selon la revendication 11, caractérisé en ce que la séquence de l'ADN recombinant codant pour la protéine mature comprend la séquence suivante (Na1'):

GGCCCTGTGC CTCCCTCTAC AGCCCTCAGG GAGCTCATTG
AGGAGCTGGT CAACATCACC CAGAACCAGA AGGCTCCGCT
CTGCAATGGC AGCATGGTAT GGAGCATCAA CCTGACAGCT
GGCATGTACT GTGCAGCCCT GGAATCCCTG ATCAACGTGT
CAGGCTGCAG TGCCATCGAG AAGACCCAGA GGATGCTGAG
CGGATTCTGC CCGCACAAGG TCTCAGCTGG GCAGTTTTCC
AGCTTGCATG TCCGAGACAC CAAAATCGAG GTGGCCCAGT
TTGTAAAGGA CCTGCTCTTA CATTTAAAGA AACTTTTTCG
CGAGGGACGG TTCAAC

**14.** Procédé selon l'une des revendications 11 à 13, caractérisé en ce que ladite séquence signal est une séquence choisie parmi les séquences (b1), (b2), (b3) et (b4) suivantes :

(b1)

Met His Pro Leu Leu Asn Pro Leu Leu Leu Ala Leu Gly Leu Met Ala
1             5             10             15

Leu Leu Leu Thr Thr Val Ile Ala Leu Thr Cys Leu Gly Gly Phe Ala
          20             25             30

(b2)

Met His Pro Leu Leu Asn Pro Leu Leu Leu Ala Leu Gly Leu Met Ala
1             5             10             15

Leu Leu Leu Thr Thr Val Ile Ala Leu Thr Cys Leu Gly Gly Phe Ala
          20             25             30

Ser Pro

(b3)

Met Ala Leu Leu Leu Thr Thr Val Ile Ala Leu Thr Cys Leu Gly Gly
1             5             10             15

Phe Ala

(b4)

Met Ala Leu Leu Leu Thr Thr Val Ile Ala Leu Thr Cys Leu Gly Gly
1             5             10             15

Phe Ala Ser Pro
          20

15. Procédé selon la revendication 14, caractérisé en ce que la séquence nucléotidique codant pour le peptide signal est choisie parmi les séquences (Nb1), (Nb2), (Nb3) et (Nb4) suivantes :

(Nb1)

ATGCATCCGC  TCCTCAATCC  TCTCCTGTTG  GCACTGGGCC
TCATGGCGCT  TTTGTTGACC  ACGGTCATTG  CTCTCACTTG
CCTTGGCGGC  TTTGCC

(Nb2)

ATGCATCCGC  TCCTCAATCC  TCTCCTGTTG  GCACTGGGCC
TCATGGCGCT  TTTGTTGACC  ACGGTCATTG  CTCTCACTTG
CCTTGGCGGC  TTTGCCTCCC  CA

(Nb3)

ATGGCGCTTT  TGTTGACCAC  GGTCATTGCT  CTCACTTGCC
TTGGCGGCTT  TGCC

(Nb4)

ATGGCGCTTT  TGTTGACCAC  GGTCATTGCT  CTCACTTGCC
TTGGCGGCTT  TGCCTCCCCA

**16.** Procédé selon l'une quelconque des revendications 1 à 15, caractérisé en ce que les cellules cultivées sont des cellules animales choisies parmi les cellules CHO et les cellules COS.

**17.** Procédé selon l'une quelconque des revendications 1 à 15, caractérisé en ce que les cellules cultivées sont des cellules de levure.

**Revendications pour l'Etat contractant suivant : GR**

**1.** Protéine à activité de type cytokine, caractérisée en ce qu'elle comprend la séquence ($a_1$) ci-après :

Gly Pro Val Pro Pro Ser Thr Ala Leu Arg Glu Leu Ile Glu Glu Leu
1          5                    10                  15

Val Asn Ile Thr Gln Asn Gln Lys Ala Pro Leu Cys Asn Gly Ser Met
               20                  25                  30

Val Trp Ser Ile Asn Leu Thr Ala Xaa Met Tyr Cys Ala Ala Leu Glu
          35                  40                  45

Ser Leu Ile Asn Val Ser Gly Cys Ser Ala Ile Glu Lys Thr Gln Arg
          50                  55              60

Met Leu Ser Gly Phe Cys Pro His Lys Val Ser Ala Gly Gln Phe Ser
65                  70                  75                  80

Ser Leu His Val Arg Asp Thr Lys Ile Glu Val Ala Gln Phe Val Lys
               85                  90                  95

Asp Leu Leu Leu His Leu Lys Lys Leu Phe Arg Glu Gly Arg Phe Asn
               100                 105                 110

dans laquelle Xaa représente Asp ou Gly,

51

ou une séquence présentant un degré d'homologie élevé avec la séquence (a$_1$)

2. Protéine selon la revendication 1, caractérisée en ce qu'elle comprend immédiatement en amont de la séquence (a$_1$) la séquence : Ser Pro.

3. Protéine selon l'une des revendications 1 et 2, caractérisée en ce qu'elle a une masse moléculaire apparente de 9,0 ± 2 kDa.

4. Protéine selon l'une des revendications 1 et 2, caractérisée en ce qu'elle a une masse moléculaire apparente de 16,0 ± 2 kDa.

5. Protéine selon l'une des revendications 1 et 2, caractérisée en ce qu'elle est N-glycosylée.

6. Protéine selon l'une des revendications 1 et 2, caractérisée en ce qu'elle présente un degré de pureté, déterminé par électrophorèse sur gel de polyacrylamide en présence de SDS et révélation à l'argent, supérieur à 70 %.

7. Protéine selon l'une des revendications 1 et 2, caractérisée en ce qu'elle présente un degré de pureté, déterminé par électrophorèse sur gel de polyacrylamide en présence de SDS et révélation à l'argent, supérieur à 90 %.

8. Protéine selon la revendication 1, caractérisée en ce qu'elle comprend un résidu méthionine immédiatement en amont de la séquence (a$_1$).

9. Protéine selon la revendication 1, caractérisée en ce qu'elle comprend en outre une séquence signal de séquence (b$_4$) :

Met Ala Leu Leu Leu Thr Thr Val Ile Ala Leu Thr Cys Leu Gly Gly
1                      5                        10                        15
Phe Ala Ser Pro
20

10. Protéine selon la revendication 9, caractérisée en ce que la séquence (b$_4$) est située immédiatement en amont de la séquence (a$_1$).

11. ADN recombinant, caractérisé, en ce qu'il code pour une protéine selon l'une des revendications 1 et 2 ou pour un précurseur d'une protéine selon l'une des revendications 1 et 2.

12. ADN recombinant, caractérisé en ce qu'il code pour un précurseur d'une protéine selon l'une des revendications 1 et 2, qui comprend une séquence signal.

13. ADN recombinant selon la revendication 12, caractérisé en ce que la séquence codant pour la protéine mature comprend la séquence suivante (Na1):

```
GGCCCTGTGC   CTCCCTCTAC   AGCCCTCAGG   GAGCTCATTG
AGGAGCTGGT   CAACATCACC   CAGAACCAGA   AGGCTCCGCT
CTGCAATGGC   AGCATGGTAT   GGAGCATCAA   CCTGACAGCT
GACATGTACT   GTGCAGCCCT   GGAATCCCTG   ATCAACGTGT
CAGGCTGCAG   TGCCATCGAG   AAGACCCAGA   GGATGCTGAG
CGGATTCTGC   CCGCACAAGG   TCTCAGCTGG   GCAGTTTCC
AGCTTGCATG   TCCGAGACAC   CAAAATCGAG   GTGGCCCAGT
TTGTAAAGGA   CCTGCTCTTA   CATTTAAAGA   AACTTTTTCG
CGAGGGACGG   TTCAAC
```

**14.** ADN recombinant selon la revendication 12, caractérisé en ce que la séquence codant pour la protéine mature comprend la séquence suivante (Na1') :

```
GGCCCTGTGC   CTCCCTCTAC   AGCCCTCAGG   GAGCTCATTG
AGGAGCTGGT   CAACATCACC   CAGAACCAGA   AGGCTCCGCT
CTGCAATGGC   AGCATGGTAT   GGAGCATCAA   CCTGACAGCT
GGCATGTACT   GTGCAGCCCT   GGAATCCCTG   ATCAACGTGT
CAGGCTGCAG   TGCCATCGAG   AAGACCCAGA   GGATGCTGAG
CGGATTCTGC   CCGCACAAGG   TCTCAGCTGG   GCAGTTTCC
```

```
AGCTTGCATG   TCCGAGACAC   CAAAATCGAG   GTGGCCCAGT
TTGTAAAGGA   CCTGCTCTTA   CATTTAAAGA   AACTTTTTCG
CGAGGGACGG   TTCAAC
```

**15.** ADN recombinant selon l'une des revendications 12 à 14, caractérisé en ce que la séquence signal est une séquence choisie parmi les séquences (b1), (b2), (b3) et (b4) suivantes :

(b1)

Met His Pro Leu Leu Asn Pro Leu Leu Leu Ala Leu Gly Leu Met Ala
1 5 10 15

Leu Leu Leu Thr Thr Val Ile Ala Leu Thr Cys Leu Gly Gly Phe Ala
20 25 30

(b2)

Met His Pro Leu Leu Asn Pro Leu Leu Leu Ala Leu Gly Leu Met Ala
1 5 10 15

Leu Leu Leu Thr Thr Val Ile Ala Leu Thr Cys Leu Gly Gly Phe Ala
20 25 30

Ser Pro


(b3)

Met Ala Leu Leu Leu Thr Thr Val Ile Ala Leu Thr Cys Leu Gly Gly
1 5 10 15

Phe Ala

(b4)

Met Ala Leu Leu Leu Thr Thr Val Ile Ala Leu Thr Cys Leu Gly Gly
1 5 10 15

Phe Ala Ser Pro
20


16. ADN recombinant selon la revendication 15, caractérisé en ce que la séquence nucléotidique codant pour le peptide signal est choisie parmi les séquences (Nb1), (Nb2), (Nb3) et (Nb4) suivantes :

(Nb1)

ATGCATCCGC TCCTCAATCC TCTCCTGTTG GCACTGGGCC

```
          TCATGGCGCT  TTTGTTGACC  ACGGTCATTG CTCTCACTTG
          CCTTGGCGGC  TTTGCC
      (Nb2)
          ATGCATCCGC  TCCTCAATCC  TCTCCTGTTG GCACTGGGCC
          TCATGGCGCT  TTTGTTGACC  ACGGTCATTG CTCTCACTTG
          CCTTGGCGGC  TTTGCCTCCC  CA
      (Nb3)
          ATGGCGCTTT  TGTTGACCAC  GGTCATTGCT CTCACTTGCC
          TTGGCGGCTT  TGCC
      (Nb4)
          ATGGCGCTTT  TGTTGACCAC  GGTCATTGCT CTCACTTGCC
          TTGGCGGCTT  TGCCTCCCCA
```

17. Vecteur d'expression, caractérisé en ce qu'il contient, avec les moyens nécessaires à son expression, un ADN recombinant selon l'une des revendications 11 à 16.

18. Cellules eucaryotes, caractérisées en ce qu'elles contiennent, avec les moyens nécessaires à leur expression, un ADN recombinant selon l'une des revendications 11 à 16.

19. Cellules eucaryotes selon la revendication 18, caractérisées en ce qu'elles sont des cellules animales.

20. Cellules animales selon la revendication 19, caractérisées en ce qu'elles contiennent un vecteur d'expression selon la revendication 17.

21. Cellules animales selon la revendication 20, caractérisées en ce qu'elles sont des cellules CHO.

22. Cellules animales selon la revendication 20, caractérisées en ce qu'elles sont des cellules COS.

23. Cellules eucaryotes selon la revendication 18, caractérisées en ce qu'elles sont des cellules de levure.

24. Microorganisme procaryote caractérisé en ce qu'il est transformé par un vecteur d'expression selon la revendication 17.

25. Microorganisme procaryote selon la revendication 24, caractérisé en ce qu'il appartient à l'espèce E. coli.

26. Procédé de préparation d'une protéine selon l'une des revendications 1 à 8, caractérisé en ce qu'il comprend une étape de culture de cellules animales selon l'une des revendications 19 à 22, suivie de l'isolement et de la purification de la protéine recombinante.

27. Procédé de préparation d'une protéine selon l'une des revendications 1 à 8, caractérisé en ce qu'il comprend une étape de culture de cellules de levure selon la revendication 23 suivie de l'isolement et de la purification de la protéine recombinante.

28. Procédé de préparation d'un médicament contenant la protéine selon l'une quelconque des revendications 1 à 8, caractérisé en ce qu'il consiste à mélanger ladite protéine avec un excipient pharmaceutiquement acceptable.

**Claims**
**Claims for the following Contracting States : AT, BE, CH, DE, DK, FR, GB, IT, LI, LU, NL, PT, SE**

1. Protein having cytokine type activity, characterised in that it comprises the sequence ($a_1$) below:

$$
\begin{array}{llllllllllllll}
\text{Gly} & \text{Pro} & \text{Val} & \text{Pro} & \text{Pro} & \text{Ser} & \text{Thr} & \text{Ala} & \text{Leu} & \text{Arg} & \text{Glu} & \text{Leu} & \text{Ile} & \text{Glu} & \text{Glu} \\
1 & & & & 5 & & & & & 10 & & & & & 15
\end{array}
$$

$$
\begin{array}{llllllllllllll}
\text{Leu} & \text{Val} & \text{Asn} & \text{Ile} & \text{Thr} & \text{Gln} & \text{Asn} & \text{Gln} & \text{Lys} & \text{Ala} & \text{Pro} & \text{Leu} & \text{Cys} & \text{Asn} & \text{Gly} \\
& & & & & 20 & & & & 25 & & & & & 30
\end{array}
$$

$$
\begin{array}{llllllllllllll}
\text{Ser} & \text{Met} & \text{Val} & \text{Trp} & \text{Ser} & \text{Ile} & \text{Asn} & \text{Leu} & \text{Thr} & \text{Ala} & \text{Xaa} & \text{Met} & \text{Tyr} & \text{Cys} & \text{Ala} \\
& & & & & 35 & & & & 40 & & & & & 45
\end{array}
$$

$$
\begin{array}{llllllllllllll}
\text{Ala} & \text{Leu} & \text{Glu} & \text{Ser} & \text{Leu} & \text{Ile} & \text{Asn} & \text{Val} & \text{Ser} & \text{Gly} & \text{Cys} & \text{Ser} & \text{Ala} & \text{Ile} & \text{Glu} \\
& & & & & 50 & & & & 55 & & & & & 60
\end{array}
$$

$$
\begin{array}{llllllllllllll}
\text{Lys} & \text{Thr} & \text{Gln} & \text{Arg} & \text{Met} & \text{Leu} & \text{Ser} & \text{Gly} & \text{Phe} & \text{Cys} & \text{Pro} & \text{His} & \text{Lys} & \text{Val} & \text{Ser} \\
& & & & & 65 & & & & 70 & & & & & 75
\end{array}
$$

$$
\begin{array}{llllllllllllll}
\text{Ala} & \text{Gly} & \text{Gln} & \text{Phe} & \text{Ser} & \text{Ser} & \text{Leu} & \text{His} & \text{Val} & \text{Arg} & \text{Asp} & \text{Thr} & \text{Lys} & \text{Ile} & \text{Glu} \\
& & & & & 80 & & & & 85 & & & & & 90
\end{array}
$$

$$
\begin{array}{llllllllllllll}
\text{Val} & \text{Ala} & \text{Gln} & \text{Phe} & \text{Val} & \text{Lys} & \text{Asp} & \text{Leu} & \text{Leu} & \text{Leu} & \text{His} & \text{Leu} & \text{Lys} & \text{Lys} & \text{Leu} \\
& & & & & 95 & & & & 100 & & & & & 105
\end{array}
$$

$$
\begin{array}{llllll}
\text{Phe} & \text{Arg} & \text{Glu} & \text{Gly} & \text{Arg} & \text{Phe} & \text{Asn} \\
& & & 110
\end{array}
$$

in which Xaa represents Asp or Gly
or a sequence possessing a high degree of homology with the sequence ($a_1$).

2. Protein according to Claim 1, characterised in that it comprises, immediately upstream of the sequence ($a_1$), the sequence: Ser Pro.

3. Protein according to one of Claims 1 and 2, characterised in that it has an apparent molecular mass of $9.0 \pm 2$ kDa.

4. Protein according to one of Claims 1 and 2, characterised in that it has an apparent molecular mass of $16.0 \pm 2$ kDa.

5. Protein according to one of Claims 1 and 2, characterised in that it is N-glycosylated.

6. Protein according to one of Claims 1 and 2, characterised in that it possesses a degree of purity, determined by polyacrylamide gel electrophoresis in the presence of SDS and visualisation with silver, of greater than 70%.

7. Protein according to one of Claims 1 and 2, characterised in that it possesses a degree of purity, determined by polyacrylamide gel electrophoresis in the presence of SDS and visualisation with silver, of greater than 90%.

8. Protein according to Claim 1, characterised in that it comprises a methionine residue immediately upstream of the sequence ($a_1$).

9. Protein according to Claim 1, characterised in that it further comprises a signal sequence of sequence ($b_4$):

Met Ala Leu Leu Leu Thr Thr Val Ile Ala Leu Thr Cys Leu Gly
1                    5                    10                    15

Gly Phe Ala Ser Pro
                    20

10. Protein according to Claim 9, characterised in that the sequence (b4) is located immediately upstream of the sequence (a1).

11. Recombinant DNA, characterised in that it codes for a protein according to one of Claims 1 and 2 or for a precursor of a protein according to one of Claims 1 and 2.

12. Recombinant DNA, characterised in that it codes for a precursor of a protein according to one of Claims 1 and 2 which comprises a signal sequence.

13. Recombinant DNA according to Claim 12, characterised in that the sequence coding for the mature protein comprises the following sequence (Na1) :

GGCCCTGTGC    CTCCCTCTAC  AGCCCTCAGG    GAGCTCATTG

AGGAGCTGGT    CAACATCACC  CAGAACCAGA    AGGCTCCGCT

CTGCAATGGC    AGCATGGTAT  GGAGCATCAA    CCTGACAGCT

GACATGTACT    GTGCAGCCCT  GGAATCCCTG    ATCAACGTGT

CAGGCTGCAG    TGCCATCGAG  AAGACCCAGA    GGATGCTGAG

CGGATTCTGC    CCGCACAAGG  TCTCAGCTGG    GCAGTTTTCC

AGCTTGCATG    TCCGAGACAC  CAAAATCGAG    GTGGCCCAGT

TTGTAAAGGA    CCTGCTCTTA  CATTTAAAGA    AACTTTTTCG

CGAGGGACGG    TTCAAC

14. Recombinant DNA according to Claim 12, characterised in that the sequence coding for the mature protein comprises the following sequence (Na1') :

GGCCCTGTGC    CTCCCTCTAC  AGCCCTCAGG    GAGCTCATTG

AGGAGCTGGT    CAACATCACC  CAGAACCAGA    AGGCTCCGCT

CTGCAATGGC    AGCATGGTAT  GGAGCATCAA    CCTGACAGCT

GGCATGTACT    GTGCAGCCCT  GGAATCCCTG    ATCAACGTGT

CAGGCTGCAG    TGCCATCGAG  AAGACCCAGA    GGATGCTGAG

CGGATTCTGC    CCGCACAAGG  TCTCAGCTGG    GCAGTTTTCC

AGCTTGCATG    TCCGAGACAC  CAAAATCGAG    GTGGCCCAGT

TTGTAAAGGA    CCTGCTCTTA  CATTTAAAGA    AACTTTTTCG

CGAGGGACGG    TTCAAC

15. Recombinant DNA according to one of Claims 12 to 14, characterised in that the signal sequence is a sequence chosen from the following sequences (b1), (b2), (b3) and (b4):

(b1)

Met His Pro Leu Leu Asn Pro Leu Leu Leu Ala Leu Gly Leu Met
1               5                    10                   15
Ala Leu Leu Leu Thr Thr Val Ile Ala Leu Thr Cys Leu Gly Gly
                    20                   25                   30
Phe Ala

(b2)

Met His Pro Leu Leu Asn Pro Leu Leu Leu Ala Leu Gly Leu Met
1               5                    10                   15
Ala Leu Leu Leu Thr Thr Val Ile Ala Leu Thr Cys Leu Gly Gly
                    20                   25                   30
Phe Ala Ser Pro

(b3)

Met Ala Leu Leu Leu Thr Thr Val Ile Ala Leu Thr Cys Leu Gly
1               5                    10                   15
Gly Phe Ala

(b4)

Met Ala Leu Leu Leu Thr Thr Val Ile Ala Leu Thr Cys Leu Gly
1               5                    10                   15
Gly Phe Ala Ser Pro
                    20

16. Recombinant DNA according to Claim 15, characterised in that the nucleotide sequence coding for the signal peptide is chosen from the following sequences (Nb1), (Nb2), (Nb3) and (Nb4):

**(Nb1)**

ATGCATCCGC     TCCTCAATCC  TCTCCTGTTG GCACTGGGCC

TCATGGCGCT     TTTGTTGACC  ACGGTCATTG CTCTCACTTG

CCTTGGCGGC     TTTGCC

**(Nb2)**

ATGCATCCGC     TCCTCAATCC  TCTCCTGTTG GCACTGGGCC

TCATGGCGCT     TTTGTTGACC  ACGGTCATTG CTCTCACTTG

CCTTGGCGGC     TTTGCCTCCC CA

**(Nb3)**

ATGGCGCTTT     TGTTGACCAC  GGTCATTGCT CTCACTTGCC

TTGGCGGCTT     TGCC

**(Nb4)**

ATGGCGCTTT     TGTTGACCAC  GGTCATTGCT CTCACTTGCC

TTGGCGGCTT     TGCCTCCCCA

**17.** Expression vector, characterised in that it contains a recombinant DNA according to one of Claims 11 to 16 with the means needed for its expression.

**18.** Eukaryotic cells, characterised in that they contain a recombinant DNA according to one of Claims 11 to 16 with the means needed for their expression.

**19.** Eukaryotic cells according to Claim 18, characterised in that they are animal cells.

**20.** Animal cells according to Claim 19, characterised in that they contain an expression vector according to Claim 17.

**21.** Animal cells according to Claim 20, characterised in that they are CHO cells.

**22.** Animal cells according to Claim 20, characterised in that they are COS cells.

**23.** Eukaryotic cells according to Claim 18, characterised in that they are yeast cells.

**24.** Prokaryotic microorganism, characterised in that it is transformed by an expression vector according to Claim 17.

**25.** Prokaryotic microorganism according to claim 24, characterised in that it belongs to the species E. coli.

**26.** Method for preparing a protein according to one of Claims 1 to 8, characterised in that it comprises a step of culturing animal cells according to one of Claims 19 to 22, followed by isolation and purification of the recombinant protein.

**27.** Method for preparing a protein according to one of Claims 1 to 8, characterised in that it comprises a step of culturing yeast cells according to Claim 23, followed by isolation and purification of the recombinant protein.

**28.** Medicinal product, characterised in that it contains a protein according to any one of Claims 1 to 8.

**Claims for the following Contracting State : ES**

**1.** Method for obtaining a protein having cytokine type activity, which comprises the following sequence (a₁):

            Gly Pro Val Pro Pro Ser Thr Ala Leu Arg Glu Leu Ile Glu Glu
            1               5                       10                      15
            Leu Val Asn Ile Thr Gln Asn Gln Lys Ala Pro Leu Cys Asn Gly
                            20                      25                      30
            Ser Met Val Trp Ser Ile Asn Leu Thr Ala Xaa Met Tyr Cys Ala
                            35                      40                      45
            Ala Leu Glu Ser Leu Ile Asn Val Ser Gly Cys Ser Ala Ile Glu
                            50                      55                      60
            Lys Thr Gln Arg Met Leu Ser Gly Phe Cys Pro His Lys Val Ser
                            65                      70                      75
            Ala Gly Gln Phe Ser Ser Leu His Val Arg Asp Thr Lys Ile Glu
                            80                      85                      90
            Val Ala Gln Phe Val Lys Asp Leu Leu Leu His Leu Lys Lys Leu
                            95                      100                     105
            Phe Arg Glu Gly Arg Phe Asn
                            110

in which Xaa represents Asp or Gly
or a sequence possessing a high degree of homology with the sequence (a₁),
which consists in
   (i) culturing animal or yeast cells which contain an expression vector containing, with the means needed for its expression, a recombinant DNA coding for said protein or a precursor thereof,
   (ii) isolating the cultured cells, and
   (iii) purifying the recombinant protein from the isolated cells.

**2.** Method according to Claim 1, characterised in that said protein comprises, immediately upstream of the sequence (a₁), the sequence: Ser Pro.

**3.** Method according to one of Claims 1 and 2, characterised in that said protein has an apparent molecular mass of 9.0 ± 2 kDa.

**4.** Method according to one of Claims 1 and 2, characterised in that said protein has an apparent molecular mass of 16.0 ± 2 kDa.

**5.** Method according to one of Claims 1 and 2, characterised in that said protein is N-glycosylated.

**6.** Method according to one of Claims 1 and 2, characterised in that said protein possesses a degree of purity, determined by polyacrylamide gel electrophoresis in the presence of SDS and visualisation with silver, of greater than 70%.

**7.** Method according to one of Claims 1 and 2, characterised in that said protein possesses a degree of purity, determined by polyacrylamide gel electrophoresis in the presence of SDS and visualisation with silver, of greater than 90%.

8. Method according to Claim 1, characterised in that said protein comprises a methionine residue immediately upstream of the sequence $(a_1)$.

9. Method according to Claim 1, characterised in that said protein further comprises a signal sequence of sequence $(b_4)$:

Met Ala Leu Leu Leu Thr Thr Val Ile Ala Leu Thr Cys Leu Gly
1 5 10 15
Gly Phe Ala Ser Pro
20

10. Method according to Claim 9, characterised in that the sequence $(b_4)$ is located immediately upstream of the sequence $(a_1)$.

11. Method according to Claim 1 or Claim 2, characterised in that the recombinant DNA codes for a precursor of said protein, which comprises a signal sequence.

12. Method according to Claim 11, characterised in that the sequence of the recombinant DNA coding for the mature protein comprises the following sequence (Na1) :

GGCCCTGTGC CTCCCTCTAC AGCCCTCAGG GAGCTCATTG

AGGAGCTGGT CAACATCACC CAGAACCAGA AGGCTCCGCT

CTGCAATGGC AGCATGGTAT GGAGCATCAA CCTGACAGCT

GACATGTACT GTGCAGCCCT GGAATCCCTG ATCAACGTGT

CAGGCTGCAG TGCCATCGAG AAGACCCAGA GGATGCTGAG

CGGATTCTGC CCGCACAAGG TCTCAGCTGG GCAGTTTTCC

AGCTTGCATG TCCGAGACAC CAAAATCGAG GTGGCCCAGT

TTGTAAAGGA CCTGCTCTTA CATTTAAAGA AACTTTTTCG

CGAGGGACGG TTCAAC

13. Method according to Claim 11, characterised in that the sequence of the recombinant DNA coding for the mature protein comprises the following sequence (Na1') :

GGCCCTGTGC CTCCCTCTAC AGCCCTCAGG GAGCTCATTG

AGGAGCTGGT CAACATCACC CAGAACCAGA AGGCTCCGCT

CTGCAATGGC AGCATGGTAT GGAGCATCAA CCTGACAGCT

GGCATGTACT GTGCAGCCCT GGAATCCCTG ATCAACGTGT

CAGGCTGCAG TGCCATCGAG AAGACCCAGA GGATGCTGAG

CGGATTCTGC CCGCACAAGG TCTCAGCTGG GCAGTTTTCC

AGCTTGCATG TCCGAGACAC CAAAATCGAG GTGGCCCAGT

TTGTAAAGGA CCTGCTCTTA CATTTAAAGA AACTTTTTCG

CGAGGGACGG TTCAAC

14. Method according to one of Claims 11 to 13, characterised in that said signal sequence is a sequence chosen from the following sequences (b1), (b2), (b3) and (b4):

(b1)

Met His Pro Leu Leu Asn Pro Leu Leu Leu Ala Leu Gly Leu Met
1         5         10         15
Ala Leu Leu Leu Thr Thr Val Ile Ala Leu Thr Cys Leu Gly Gly
         20         25         30
Phe Ala

(b2)

Met His Pro Leu Leu Asn Pro Leu Leu Leu Ala Leu Gly Leu Met
1         5         10         15
Ala Leu Leu Leu Thr Thr Val Ile Ala Leu Thr Cys Leu Gly Gly
         20         25         30

Phe Ala Ser Pro

(b3)

Met Ala Leu Leu Leu Thr Thr Val Ile Ala Leu Thr Cys Leu Gly
1           5           10          15
Gly Phe Ala

(b4)

Met Ala Leu Leu Leu Thr Thr Val Ile Ala Leu Thr Cys Leu Gly
1           5            10          15
Gly Phe Ala Ser Pro
         20

**15.** Method according to Claim 14, characterised in that the nucleotide sequence coding for the signal peptide is chosen from the following sequences (Nb1), (Nb2), (Nb3) and (Nb4):

(Nb1)

ATGCATCCGC   TCCTCAATCC  TCTCCTGTTG GCACTGGGCC
TCATGGCGCT   TTTGTTGACC  ACGGTCATTG CTCTCACTTG
CCTTGGCGGC   TTTGCC

(Nb2)

ATGCATCCGC   TCCTCAATCC  TCTCCTGTTG GCACTGGGCC
TCATGGCGCT   TTTGTTGACC  ACGGTCATTG CTCTCACTTG
CCTTGGCGGC   TTTGCCTCCC CA

(Nb3)

ATGGCGCTTT   TGTTGACCAC  GGTCATTGCT CTCACTTGCC
TTGGCGGCTT   TGCC

(Nb4)

ATGGCGCTTT   TGTTGACCAC  GGTCATTGCT CTCACTTGCC
TTGGCGGCTT   TGCCTCCCCA

**16.** Method according to any one of Claims 1 to 15, characterised in that the cultured cells are animal cells chosen from the CHO cells and the COS cells.

**17.** Method according to any one of Claims 1 to 15, characterised in that the cultured cells are yeast cells.

**Claims for the following Contracting State : GR**

1. Protein having cytokine type activity, characterised in that it comprises the sequence ($a_1$) below:

```
Gly Pro Val Pro Pro Ser Thr Ala Leu Arg Glu Leu Ile Glu Glu
 1            5                   10                  15
Leu Val Asn Ile Thr Gln Asn Gln Lys Ala Pro Leu Cys Asn Gly
                 20                  25                  30
Ser Met Val Trp Ser Ile Asn Leu Thr Ala Xaa Met Tyr Cys Ala
                 35                  40                  45
Ala Leu Glu Ser Leu Ile Asn Val Ser Gly Cys Ser Ala Ile Glu
                 50                  55                  60
Lys Thr Gln Arg Met Leu Ser Gly Phe Cys Pro His Lys Val Ser
                 65                  70                  75
Ala Gly Gln Phe Ser Ser Leu His Val Arg Asp Thr Lys Ile Glu
                 80                  85                  90
Val Ala Gln Phe Val Lys Asp Leu Leu Leu His Leu Lys Lys Leu
                 95                  100                 105
Phe Arg Glu Gly Arg Phe Asn
                 110
```

in which Xaa represents Asp or Gly
or a sequence possessing a high degree of homology with the sequence ($a_1$).

2. Protein according to Claim 1, characterised in that it comprises, immediately upstream of the sequence ($a_1$), the sequence: Ser Pro.

3. Protein according to one of Claims 1 and 2, characterised in that it has an apparent molecular mass of 9.0 ± 2 kDa.

4. Protein according to one of Claims 1 and 2, characterised in that it has an apparent molecular mass of 16.0 ± 2 kDa.

5. Protein according to one of Claims 1 and 2, characterised in that it is N-glycosylated.

6. Protein according to one of Claims 1 and 2, characterised in that it possesses a degree of purity, determined by polyacrylamide gel electrophoresis in the presence of SDS and visualisation with silver, of greater than 70%.

7. Protein according to one of Claims 1 and 2, characterised in that it possesses a degree of purity, determined by polyacrylamide gel electrophoresis in the presence of SDS and visualisation with silver, of greater than 90%.

8. Protein according to Claim 1, characterised in that it comprises a methionine residue immediately upstream of the sequence ($a_1$).

9. Protein according to Claim 1, characterised in that it further comprises a signal sequence of sequence ($b_4$):

Met Ala Leu Leu Leu Thr Thr Val Ile Ala Leu Thr Cys Leu Gly
  1        5        10        15

Gly Phe Ala Ser Pro
          20

10. Protein according to Claim 9, characterised in that the sequence (b₄) is located immediately upstream of the sequence (a₁).

11. Recombinant DNA, characterised in that it codes for a protein according to one of Claims 1 and 2 or for a precursor of a protein according to one of Claims 1 and 2.

12. Recombinant DNA, characterised in that it codes for a precursor of a protein according to one of Claims 1 and 2 which comprises a signal sequence.

13. Recombinant DNA according to Claim 12, characterised in that the sequence coding for the mature protein comprises the following sequence (Na1) :

| | | | |
|---|---|---|---|
| GGCCCTGTGC | CTCCCTCTAC | AGCCCTCAGG | GAGCTCATTG |
| AGGAGCTGGT | CAACATCACC | CAGAACCAGA | AGGCTCCGCT |
| CTGCAATGGC | AGCATGGTAT | GGAGCATCAA | CCTGACAGCT |
| GACATGTACT | GTGCAGCCCT | GGAATCCCTG | ATCAACGTGT |
| CAGGCTGCAG | TGCCATCGAG | AAGACCCAGA | GGATGCTGAG |
| CGGATTCTGC | CCGCACAAGG | TCTCAGCTGG | GCAGTTTTCC |
| AGCTTGCATG | TCCGAGACAC | CAAAATCGAG | GTGGCCCAGT |
| TTGTAAAGGA | CCTGCTCTTA | CATTTAAAGA | AACTTTTTCG |
| CGAGGGACGG | TTCAAC | | |

14. Recombinant DNA according to Claim 12, characterised in that the sequence coding for the mature protein comprises the following sequence (Na1'):

| | | | |
|---|---|---|---|
| GGCCCTGTGC | CTCCCTCTAC | AGCCCTCAGG | GAGCTCATTG |
| AGGAGCTGGT | CAACATCACC | CAGAACCAGA | AGGCTCCGCT |
| CTGCAATGGC | AGCATGGTAT | GGAGCATCAA | CCTGACAGCT |
| GGCATGTACT | GTGCAGCCCT | GGAATCCCTG | ATCAACGTGT |
| CAGGCTGCAG | TGCCATCGAG | AAGACCCAGA | GGATGCTGAG |
| CGGATTCTGC | CCGCACAAGG | TCTCAGCTGG | GCAGTTTTCC |
| AGCTTGCATG | TCCGAGACAC | CAAAATCGAG | GTGGCCCAGT |
| TTGTAAAGGA | CCTGCTCTTA | CATTTAAAGA | AACTTTTTCG |
| CGAGGGACGG | TTCAAC | | |

65

**15.** Recombinant DNA according to one of Claims 12 to 14, characterised in that the signal sequence is a sequence chosen from the following sequences (b1), (b2), (b3) and (b4):

**(b1)**

Met His Pro Leu Leu Asn Pro Leu Leu Leu Ala Leu Gly Leu Met
1 5 10 15
Ala Leu Leu Leu Thr Thr Val Ile Ala Leu Thr Cys Leu Gly Gly
20 25 30
Phe Ala

**(b2)**

Met His Pro Leu Leu Asn Pro Leu Leu Leu Ala Leu Gly Leu Met
1 5 10 15
Ala Leu Leu Leu Thr Thr Val Ile Ala Leu Thr Cys Leu Gly Gly
20 25 30
Phe Ala Ser Pro

**(b3)**

Met Ala Leu Leu Leu Thr Thr Val Ile Ala Leu Thr Cys Leu Gly
1 5 10 15
Gly Phe Ala

**(b4)**

Met Ala Leu Leu Leu Thr Thr Val Ile Ala Leu Thr Cys Leu Gly
1 5 10 15
Gly Phe Ala Ser Pro
20

**16.** Recombinant DNA according to Claim 15, characterised in that the nucleotide sequence coding for the signal peptide is chosen from the following sequences (Nb1), (Nb2), (Nb3) and (Nb4):

## (Nb1)

ATGCATCCGC     TCCTCAATCC  TCTCCTGTTG GCACTGGGCC

TCATGGCGCT     TTTGTTGACC  ACGGTCATTG CTCTCACTTG

CCTTGGCGGC     TTTGCC

## (Nb2)

ATGCATCCGC     TCCTCAATCC  TCTCCTGTTG GCACTGGGCC

TCATGGCGCT     TTTGTTGACC  ACGGTCATTG CTCTCACTTG

CCTTGGCGGC     TTTGCCTCCC CA

## (Nb3)

ATGGCGCTTT     TGTTGACCAC  GGTCATTGCT CTCACTTGCC

TTGGCGGCTT     TGCC

## (Nb4)

ATGGCGCTTT     TGTTGACCAC  GGTCATTGCT CTCACTTGCC

TTGGCGGCTT     TGCCTCCCCA

**17.** Expression vector, characterised in that it contains a recombinant DNA according to one of Claims 11 to 16 with the means needed for its expression.

**18.** Eukaryotic cells, characterised in that they contain a recombinant DNA according to one of Claims 11 to 16 with the means needed for their expression.

**19.** Eukaryotic cells according to Claim 18, characterised in that they are animal cells.

**20.** Animal cells according to Claim 19, characterised in that they contain an expression vector according to Claim 17.

**21.** Animal cells according to Claim 20, characterised in that they are CHO cells.

**22.** Animal cells according to Claim 20, characterised in that they are COS cells.

**23.** Eukaryotic cells according to Claim 18, characterised in that they are yeast cells.

**24.** Prokaryotic microorganism, characterised in that it is transformed by an expression vector according to Claim 17.

**25.** Prokaryotic microorganism according to claim 24, characterised in that it belongs to the species E. coli.

**26.** Method for preparing a protein according to one of Claims 1 to 8, characterised in that it comprises a step of culturing animal cells according to one of Claims 19 to 22, followed by isolation and purification of the recombinant protein.

**27.** Method for preparing a protein according to one of Claims 1 to 8, characterised in that it comprises a step of culturing yeast cells according to Claim 23, followed by isolation and purification of the recombinant protein.

**28.** Method for preparing a medicinal product containing the protein according to any one of Claims 1 to 8, characterised in that it consists in mixing said protein with a pharmaceutically acceptable excipient.

**Patentansprüche**

**Patentansprüche für folgende Vertragsstaaten : AT, BE, CH, DE, DK, FR, GB, IT, LI, LU, NL, PT, SE**

**1.** Protein mit Wirksamkeit vom Typ eines Cytokins, dadurch gekennzeichnet, daß es die nachstehende Sequenz ($a_1$):

Gly Pro Val Pro Pro Ser Thr Ala Leu Arg Glu Leu Ile Glu Glu Leu
1       5               10              15

Val Asn Ile Thr Gln Asn Gln Lys Ala Pro Leu Cys Asn Gly Ser Met
20              25              30

Val Trp Ser Ile Asn Leu Thr Ala Xaa Met Tyr Cys Ala Ala Leu Glu
35              40              45

Ser Leu Ile Asn Val Ser Gly Cys Ser Ala Ile Glu Lys Thr Gln Arg
50              55              60

Met Leu Ser Gly Phe Cys Pro His Lys Val Ser Ala Gly Gln Phe Ser
65              70              75              80

Ser Leu His Val Arg Asp Thr Lys Ile Glu Val Ala Gln Phe Val Lys
85              90              95

Asp Leu Leu Leu His Leu Lys Lys Leu Phe Arg Glu Gly Arg Phe Asn
100             105             110

worin Xaa Asp oder Gly darstellt, oder eine Sequenz, die einen hohen Grad der Homologie mit der Sequenz ($a_1$) aufweist, umfaßt.

**2.** Protein nach Anspruch 1, dadurch gekennzeichnet, daß es unmittelbar stromaufwärts von der Sequenz ($a_1$) die Sequenz Ser Pro aufweist.

**3.** Protein nach einem der Ansprüche 1 und 2, dadurch gekennzeichnet, daß es eine scheinbare Molmasse von 9,0 ± 2 kDa aufweist.

**4.** Protein nach einem der Ansprüche 1 und 2, dadurch gekennzeichnet, daß es eine scheinbare Molmasse von 16,0 ± 2 kDa aufweist.

**5.** Protein nach einem der Ansprüche 1 und 2, dadurch gekennzeichnet, daß es N-glykosyliert ist.

**6.** Protein nach einem der Ansprüche 1 und 2, dadurch gekennzeichnet, daß es einen Reinheitsgrad, bestimmt durch Elektrophorese auf Polyacrylamid-Gel in Anwesenheit von SDS und unter Silber-Färbung, von mehr als 70 % aufweist.

**7.** Protein nach einem der Ansprüche 1 und 2, dadurch gekennzeichnet, daß es einen Reinheitsgrad, bestimmt durch Elektrophorese auf Polyacrylamid-Gel in Anwesenheit von SDS und unter Silber-Färbung, von mehr als 90 % aufweist.

**8.** Protein nach Anspruch 1, dadurch gekennzeichnet, daß es einen Methionin-Rest unmittelbar stromaufwärts von der Sequenz ($a_1$) aufweist.

**9.** Protein nach Anspruch 1, dadurch gekennzeichnet, daß es außerdem eine Signalsequenz mit der Sequenz ($b_4$) umfaßt:

Met Ala Leu Leu Leu Thr Thr Val Ile Ala Leu Thr Cys Leu Gly Gly
1           5            10          15

Phe Ala Ser Pro
20

**10.** Protein nach Anspruch 9, dadurch gekennzeichnet, daß die Sequenz ($b_4$) unmittelbar stromaufwärts von der Sequenz ($a_1$) angeordnet ist.

**11.** Rekombinante DNS, dadurch gekennzeichnet, daß sie für ein Protein nach einem der Ansprüche 1 und 2 oder für einen Vorläufer eines Proteins nach einem der Ansprüche 1 und 2 codiert.

**12.** Rekombinante DNS, dadurch gekennzeichnet, daß sie für einen Vorläufer eines Proteins nach einem der Ansprüche 1 und 2 codiert, der eine Signalsequenz umfaßt.

**13.** Rekombinante DNS nach Anspruch 12, dadurch gekennzeichnet, daß die für das reife Protein codierende Sequenz die folgende Sequenz (Na1) umfaßt:

GGCCCTGTGC  CTCCCTCTAC  AGCCCTCAGG  GAGCTCATTG
AGGAGCTGGT  CAACATCACC  CAGAACCAGA  AGGCTCCGCT
CTGCAATGGC  AGCATGGTAT  GGAGCATCAA  CCTGACAGCT
GACATGTACT  GTGCAGCCCT  GGAATCCCTG  ATCAACGTGT
CAGGCTGCAG  TGCCATCGAG  AAGACCCAGA  GGATGCTGAG
CGGATTCTGC  CCGCACAAGG  TCTCAGCTGG  GCAGTTTTCC
AGCTTGCATG  TCCGAGACAC  CAAAATCGAG  GTGGCCCAGT
TTGTAAAGGA  CCTGCTCTTA  CATTTAAAGA  AACTTTTTCG
CGAGGGACGG  TTCAAC

**14.** Rekombinante DNS nach Anspruch 12, dadurch gekennzeichnet, daß die für das reife Protein codierende Sequenz die folgende Sequenz (Na1') umfaßt:

GGCCCTGTGC  CTCCCTCTAC  AGCCCTCAGG  GAGCTCATTG
AGGAGCTGGT  CAACATCACC  CAGAACCAGA  AGGCTCCGCT
CTGCAATGGC  AGCATGGTAT  GGAGCATCAA  CCTGACAGCT
GGCATGTACT  GTGCAGCCCT  GGAATCCCTG  ATCAACGTGT
CAGGCTGCAG  TGCCATCGAG  AAGACCCAGA  GGATGCTGAG
CGGATTCTGC  CCGCACAAGG  TCTCAGCTGG  GCAGTTTTCC
AGCTTGCATG  TCCGAGACAC  CAAAATCGAG  GTGGCCCAGT
TTGTAAAGGA  CCTGCTCTTA  CATTTAAAGA  AACTTTTTCG
CGAGGGACGG  TTCAAC

**15.** Rekombinante DNS nach einem der Ansprüche 12 bis 14, dadurch gekennzeichnet, daß die Signalsequenz eine Sequenz ist, die aus den folgenden Sequenzen (b1), (b2), (b3) und (b4) ausgewählt ist:

**(b1)**

Met His Pro Leu Leu Asn Pro Leu Leu Leu Ala Leu Gly Leu Met Ala
1           5          10         15

Leu Leu Leu Thr Thr Val Ile Ala Leu Thr Cys Leu Gly Gly Phe Ala
20        25        30

**(b2)**

Met His Pro Leu Leu Asn Pro Leu Leu Leu Ala Leu Gly Leu Met Ala
1           5           10         15

Leu Leu Leu Thr Thr Val Ile Ala Leu Thr Cys Leu Gly Gly Phe Ala
20        25        30

Ser Pro

**(b3)**

Met Ala Leu Leu Leu Thr Thr Val Ile Ala Leu Thr Cys Leu Gly Gly
1           5           10         15

Phe Ala

**(b4)**

Met Ala Leu Leu Leu Thr Thr Val Ile Ala Leu Thr Cys Leu Gly Gly
1           5           10         15

Phe Ala Ser Pro
20

**16.** Rekombinante DNS nach Anspruch 15, dadurch gekennzeichnet, daß die Nucleotidsequenz, die für das Signalpeptid codiert, aus den folgenden Sequenzen (Nb1), (Nb2), (Nb3) und (Nb4) ausgewählt ist:

(Nb1)

ATGCATCCGC TCCTCAATCC TCTCCTGTTG GCACTGGGCC
TCATGGCGCT TTTGTTGACC ACGGTCATTG CTCTCACTTG
CCTTGGCGGC TTTGCC

(Nb2)

ATGCATCCGC TCCTCAATCC TCTCCTGTTG GCACTGGGCC
TCATGGCGCT TTTGTTGACC ACGGTCATTG CTCTCACTTG
CCTTGGCGGC TTTGCCTCCC CA

(Nb3)

ATGGCGCTTT TGTTGACCAC GGTCATTGCT CTCACTTGCC
TTGGCGGCTT TGCC

(Nb4)

ATGGCGCTTT TGTTGACCAC GGTCATTGCT CTCACTTGCC
TTGGCGGCTT TGCCTCCCCA

17. Expressionsvektor, dadurch gekennzeichnet, daß er, mit den für seine Expression notwendigen Mitteln, eine rekombinante DNS nach einem der Ansprüche 11 bis 16 enthält.

18. Eukaryotische Zellen, dadurch gekennzeichnet, daß sie, mit den für ihre Expression notwendigen Mitteln, eine rekombinante DNS nach einem der Ansprüche 11 bis 16 enthalten.

19. Eukaryotische Zellen nach Anspruch 18, dadurch gekennzeichnet, daß sie tierische Zellen sind.

20. Tierische Zellen nach Anspruch 19, dadurch gekennzeichnet, daß sie einen Expressionsvektor nach Anspruch 17 enthalten.

21. Tierische Zellen nach Anspruch 20, dadurch gekennzeichnet, daß sie CHO-Zellen sind.

22. Tierische Zellen nach Anspruch 20, dadurch gekennzeichnet, daß sie COS-Zellen sind.

23. Eukaryotische Zellen nach Anspruch 18, dadurch gekennzeichnet, daß sie Hefezellen sind.

24. Prokaryotischer Mikroorganismus, dadurch gekennzeichnet, daß er durch einen Expressionsvektor nach Anspruch 17 transformiert wird.

25. Prokaryotischer Mikroorganismus nach Anspruch 24, dadurch gekennzeichnet, daß er zur Gattung E. coli gehört.

26. Verfahren zur Herstellung eines Proteins nach einem der Ansprüche 1 bis 8, dadurch gekennzeichnet, daß es einen Kultivierungsschritt tierischer Zellen nach einem der Ansprüche 19 bis 22 umfaßt, gefolgt von der Isolierung und der Reinigung des rekombinanten Proteins.

27. Verfahren zur Herstellung eines Proteins nach einem der Ansprüche 1 bis 8, dadurch gekennzeichnet, daß es einen Kultivierungsschritt von Hefezellen nach Anspruch 23 umfaßt, gefolgt von der Isolierung und der Reinigung des rekombinanten Proteins.

28. Medikament, dadurch gekennzeichnet, daß es ein Protein nach einem der Ansprüche 1 bis 8 enthält.

EP 0 506 574 B1

**Patentansprüche für folgenden Vertragsstaat : ES**

1. Verfahren zur Herstellung eines Proteins mit Wirksamkeit vom Typ eines Cytokins, das die nachstehende Sequenz ($a_1$):

$$
\begin{array}{l}
\text{Gly Pro Val Pro Pro Ser Thr Ala Leu Arg Glu Leu Ile Glu Glu Leu} \\
\qquad 1 \qquad\qquad 5 \qquad\qquad\quad 10 \qquad\qquad\quad 15 \\
\text{Val Asn Ile Thr Gln Asn Gln Lys Ala Pro Leu Cys Asn Gly Ser Met} \\
\qquad\qquad 20 \qquad\qquad\quad 25 \qquad\qquad\quad 30 \\
\text{Val Trp Ser Ile Asn Leu Thr Ala Xaa Met Tyr Cys Ala Ala Leu Glu} \\
\qquad\qquad 35 \qquad\qquad\quad 40 \qquad\qquad\quad 45 \\
\text{Ser Leu Ile Asn Val Ser Gly Cys Ser Ala Ile Glu Lys Thr Gln Arg} \\
\qquad\quad 50 \qquad\qquad\quad 55 \qquad\qquad\quad 60 \\
\text{Met Leu Ser Gly Phe Cys Pro His Lys Val Ser Ala Gly Gln Phe Ser} \\
\qquad 65 \qquad\qquad\quad 70 \qquad\qquad\quad 75 \qquad\qquad\quad 80 \\
\text{Ser Leu His Val Arg Asp Thr Lys Ile Glu Val Ala Gln Phe Val Lys} \\
\qquad\qquad 85 \qquad\qquad\quad 90 \qquad\qquad\quad 95 \\
\text{Asp Leu Leu Leu His Leu Lys Lys Leu Phe Arg Glu Gly Arg Phe Asn} \\
\qquad\qquad 100 \qquad\qquad\quad 105 \qquad\qquad\quad 110
\end{array}
$$

worin Xaa Asp oder Gly darstellt, oder eine Sequenz, die einen hohen Grad der Homologie mit der Sequenz ($a_1$) aufweist, umfaßt, welches Verfahren darin besteht, daß

(i) tierische oder Hefezellen, die einen Expressionsvektor enthalten, der, mit den für seine Expression erforderlichen Mitteln, eine rekombinante DNS enthält, die für das genannte Protein oder einen Vorläufer hievon codiert, kultiviert werden,

(ii) die Kulturzellen isoliert werden, und

(iii) das rekombinante Protein aus isolierten Zellen gereinigt wird.

2. Verfahren nach Anspruch 1, dadurch gekennzeichnet, daß das genannte Protein unmittelbar stromaufwärts von der Sequenz ($a_1$) die Sequenz Ser Pro aufweist.

3. Verfahren nach einem der Ansprüche 1 und 2, dadurch gekennzeichnet, daß das genannte Protein eine scheinbare Molmasse von 9,0 ± 2 kDa aufweist.

4. Verfahren nach einem der Ansprüche 1 und 2, dadurch gekennzeichnet, daß das genannte Protein eine scheinbare Molmasse von 16,0 ± 2 kDa aufweist.

5. Verfahren nach einem der Ansprüche 1 und 2, dadurch gekennzeichnet, daß das genannte Protein N-glykosyliert wird.

6. Verfahren nach einem der Ansprüche 1 und 2, dadurch gekennzeichnet, daß das genannte Protein einen Reinheitsgrad, bestimmt durch Elektrophorese auf Polyacrylamid-Gel in Anwesenheit von SDS und unter Silber-Färbung, von mehr als 70 % aufweist.

7. Verfahren nach einem der Ansprüche 1 und 2, dadurch gekennzeichnet, daß das genannte Protein einen Reinheitsgrad, bestimmt durch Elektrophorese auf Polyacrylamid-Gel in Anwesenheit von SDS und unter Silber-Färbung, von mehr als 90 % aufweist.

8. Verfahren nach Anspruch 1, dadurch gekennzeichnet, daß das genannte Protein einen Methionin-Rest unmittelbar stromaufwärts von der Sequenz ($a_1$) aufweist.

72

**9.** Verfahren nach Anspruch 1, dadurch gekennzeichnet, daß das genannte Protein außerdem eine Signalsequenz mit der Sequenz (b₄) umfaßt:

Met Ala Leu Leu Leu Thr Thr Val Ile Ala Leu Thr Cys Leu Gly Gly

1 5 10 15

Phe Ala Ser Pro

20

**10.** Verfahren nach Anspruch 9, dadurch gekennzeichnet, daß die Sequenz (b₄) unmittelbar stromaufwärts von der Sequenz (a₁) angeordnet ist.

**11.** Verfahren nach Anspruch 1 oder Anspruch 2, dadurch gekennzeichnet, daß die rekombinante DNS für einen Vorläufer des genannten Proteins codiert, der eine Signalsequenz umfaßt.

**12.** Verfahren nach Anspruch 11, dadurch gekennzeichnet, daß die für das reife Protein codierende, rekombinante DNS-Sequenz die folgende Sequenz (Na1) umfaßt:

```
GGCCCTGTGC  CTCCCTCTAC  AGCCCTCAGG  GAGCTCATTG
AGGAGCTGGT  CAACATCACC  CAGAACCAGA  AGGCTCCGCT
CTGCAATGGC  AGCATGGTAT  GGAGCATCAA  CCTGACAGCT
GACATGTACT  GTGCAGCCCT  GGAATCCCTG  ATCAACGTGT
CAGGCTGCAG  TGCCATCGAG  AAGACCCAGA  GGATGCTGAG
CGGATTCTGC  CCGCACAAGG  TCTCAGCTGG  GCAGTTTTCC
AGCTTGCATG  TCCGAGACAC  CAAAATCGAG  GTGGCCCAGT
TTGTAAAGGA  CCTGCTCTTA  CATTTAAAGA  AACTTTTTCG
CGAGGGACGG  TTCAAC
```

**13.** Verfahren nach Anspruch 11, dadurch gekennzeichnet, daß die für das reife Protein codierende, rekombinante DNS-Sequenz die folgende Sequenz (Na1') umfaßt:

```
GGCCCTGTGC  CTCCCTCTAC  AGCCCTCAGG  GAGCTCATTG
AGGAGCTGGT  CAACATCACC  CAGAACCAGA  AGGCTCCGCT
CTGCAATGGC  AGCATGGTAT  GGAGCATCAA  CCTGACAGCT
GGCATGTACT  GTGCAGCCCT  GGAATCCCTG  ATCAACGTGT
CAGGCTGCAG  TGCCATCGAG  AAGACCCAGA  GGATGCTGAG
CGGATTCTGC  CCGCACAAGG  TCTCAGCTGG  GCAGTTTTCC
AGCTTGCATG  TCCGAGACAC  CAAAATCGAG  GTGGCCCAGT
TTGTAAAGGA  CCTGCTCTTA  CATTTAAAGA  AACTTTTTCG
CGAGGGACGG  TTCAAC
```

**14.** Verfahren nach einem der Ansprüche 11 bis 13, dadurch gekennzeichnet, daß die genannte Signalsequenz eine Sequenz ist, die aus den folgenden Sequenzen (b1), (b2), (b3) und (b4) ausgewählt wird:

(b1)

Met His Pro Leu Leu Asn Pro Leu Leu Leu Ala Leu Gly Leu Met Ala
1               5               10              15

Leu Leu Leu Thr Thr Val Ile Ala Leu Thr Cys Leu Gly Gly Phe Ala
20              25              30

(b2)

Met His Pro Leu Leu Asn Pro Leu Leu Leu Ala Leu Gly Leu Met Ala
1               5               10              15

Leu Leu Leu Thr Thr Val Ile Ala Leu Thr Cys Leu Gly Gly Phe Ala
20              25              30

Ser Pro


(b3)

Met Ala Leu Leu Leu Thr Thr Val Ile Ala Leu Thr Cys Leu Gly Gly
1               5               10              15

Phe Ala

(b4)

Met Ala Leu Leu Leu Thr Thr Val Ile Ala Leu Thr Cys Leu Gly Gly
1               5               10              15

Phe Ala Ser Pro
20


**15.** Verfahren nach Anspruch 14, dadurch gekennzeichnet, daß die Nucleotidsequenz, die für das Signalpeptid codiert, aus den folgenden Sequenzen (Nb1), (Nb2), (Nb3) und (Nb4) ausgewählt wird:

74

(Nb1)

ATGCATCCGC  TCCTCAATCC  TCTCCTGTTG  GCACTGGGCC

TCATGGCGCT  TTTGTTGACC  ACGGTCATTG  CTCTCACTTG

CCTTGGCGGC  TTTGCC

(Nb2)

ATGCATCCGC  TCCTCAATCC  TCTCCTGTTG  GCACTGGGCC

TCATGGCGCT  TTTGTTGACC  ACGGTCATTG  CTCTCACTTG

CCTTGGCGGC  TTTGCCTCCC  CA

(Nb3)

ATGGCGCTTT  TGTTGACCAC  GGTCATTGCT  CTCACTTGCC

TTGGCGGCTT  TGCC

(Nb4)

ATGGCGCTTT  TGTTGACCAC  GGTCATTGCT  CTCACTTGCC

TTGGCGGCTT  TGCCTCCCCA

**16.** Verfahren nach einem der Ansprüche 1 bis 15, dadurch gekennzeichnet, daß die Kulturzellen tierische Zellen, ausgewählt aus CHO-Zellen und COS-Zellen, sind.

**17.** Verfahren nach einem der Ansprüche 1 bis 15, dadurch gekennzeichnet, daß die Kulturzellen Hefezellen sind.

**Patentansprüche für folgenden Vertragsstaat : GR**

**1.** Protein mit Wirksamkeit vom Typ eines Cytokins, dadurch gekennzeichnet, daß es die nachstehende Sequenz (a₁):

Gly Pro Val Pro Pro Ser Thr Ala Leu Arg Glu Leu Ile Glu Glu Leu

1           5               10              15

Val Asn Ile Thr Gln Asn Gln Lys Ala Pro Leu Cys Asn Gly Ser Met

20              25              30

Val Trp Ser Ile Asn Leu Thr Ala Xaa Met Tyr Cys Ala Ala Leu Glu

35              40              45

Ser Leu Ile Asn Val Ser Gly Cys Ser Ala Ile Glu Lys Thr Gln Arg

50              55              60

Met Leu Ser Gly Phe Cys Pro His Lys Val Ser Ala Gly Gln Phe Ser

65              70              75              80

Ser Leu His Val Arg Asp Thr Lys Ile Glu Val Ala Gln Phe Val Lys

85              90              95

Asp Leu Leu Leu His Leu Lys Lys Leu Phe Arg Glu Gly Arg Phe Asn

100             105             110

EP 0 506 574 B1

worin Xaa Asp oder Gly darstellt, oder eine Sequenz, die einen hohen Grad der Homologie mit der Sequenz $(a_1)$ aufweist, umfaßt.

2. Protein nach Anspruch 1, dadurch gekennzeichnet, daß es unmittelbar stromaufwärts von der Sequenz $(a_1)$ die Sequenz Ser Pro aufweist.

3. Protein nach einem der Ansprüche 1 und 2, dadurch gekennzeichnet, daß es eine scheinbare Molmasse von 9,0 ± 2 kDa aufweist.

4. Protein nach einem der Ansprüche 1 und 2, dadurch gekennzeichnet, daß es eine scheinbare Molmasse von 16,0 ± 2 kDa aufweist.

5. Protein nach einem der Ansprüche 1 und 2, dadurch gekennzeichnet, daß es N-glykosyliert ist.

6. Protein nach einem der Ansprüche 1 und 2, dadurch gekennzeichnet, daß es einen Reinheitsgrad, bestimmt durch Elektrophorese auf Polyacrylamid-Gel in Anwesenheit von SDS und unter Silber-Färbung, von mehr als 70 % aufweist.

7. Protein nach einem der Ansprüche 1 und 2, dadurch gekennzeichnet, daß es einen Reinheitsgrad, bestimmt durch Elektrophorese auf Polyacrylamid-Gel in Anwesenheit von SDS und unter Silber-Färbung, von mehr als 90 % aufweist.

8. Protein nach Anspruch 1, dadurch gekennzeichnet, daß es einen Methionin-Rest unmittelbar stromaufwärts von der Sequenz $(a_1)$ aufweist.

9. Protein nach Anspruch 1, dadurch gekennzeichnet, daß es außerdem eine Signalsequenz mit der Sequenz $(b_4)$ umfaßt:

Met Ala Leu Leu Leu Thr Thr Val Ile Ala Leu Thr Cys Leu Gly Gly
1          5            10            15

Phe Ala Ser Pro
20

10. Protein nach Anspruch 9, dadurch gekennzeichnet, daß die Sequenz $(b_4)$ unmittelbar stromaufwärts von der Sequenz $(a_1)$ angeordnet ist.

11. Rekombinante DNS, dadurch gekennzeichnet, daß sie für ein Protein nach einem der Ansprüche 1 und 2 oder für einen Vorläufer eines Proteins nach einem der Ansprüche 1 und 2 codiert.

12. Rekombinante DNS, dadurch gekennzeichnet, daß sie für einen Vorläufer eines Proteins nach einem der Ansprüche 1 und 2 codiert, der eine Signalsequenz umfaßt.

13. Rekombinante DNS nach Anspruch 12, dadurch gekennzeichnet, daß die für das reife Protein codierende Sequenz die folgende Sequenz (Na1) umfaßt:

76

```
GGCCCTGTGC  CTCCCTCTAC  AGCCCTCAGG  GAGCTCATTG
AGGAGCTGGT  CAACATCACC  CAGAACCAGA  AGGCTCCGCT
CTGCAATGGC  AGCATGGTAT  GGAGCATCAA  CCTGACAGCT
GACATGTACT  GTGCAGCCCT  GGAATCCCTG  ATCAACGTGT
CAGGCTGCAG  TGCCATCGAG  AAGACCCAGA  GGATGCTGAG
CGGATTCTGC  CCGCACAAGG  TCTCAGCTGG  GCAGTTTTCC
AGCTTGCATG  TCCGAGACAC  CAAAATCGAG  GTGGCCCAGT
TTGTAAAGGA  CCTGCTCTTA  CATTTAAAGA  AACTTTTTCG
CGAGGGACGG  TTCAAC
```

14. Rekombinante DNS nach Anspruch 12, dadurch gekennzeichnet, daß die für das reife Protein codierende Sequenz die folgende Sequenz (Na1') umfaßt:

```
GGCCCTGTGC  CTCCCTCTAC  AGCCCTCAGG  GAGCTCATTG
AGGAGCTGGT  CAACATCACC  CAGAACCAGA  AGGCTCCGCT
CTGCAATGGC  AGCATGGTAT  GGAGCATCAA  CCTGACAGCT
GGCATGTACT  GTGCAGCCCT  GGAATCCCTG  ATCAACGTGT
CAGGCTGCAG  TGCCATCGAG  AAGACCCAGA  GGATGCTGAG
CGGATTCTGC  CCGCACAAGG  TCTCAGCTGG  GCAGTTTTCC
AGCTTGCATG  TCCGAGACAC  CAAAATCGAG  GTGGCCCAGT
TTGTAAAGGA  CCTGCTCTTA  CATTTAAAGA  AACTTTTTCG
CGAGGGACGG  TTCAAC
```

15. Rekombinante DNS nach einem der Ansprüche 12 bis 14, dadurch gekennzeichnet, daß die Signalsequenz eine Sequenz ist, die aus den folgenden Sequenzen (b1), (b2), (b3) und (b4) ausgewählt ist:

77

(b1)

Met His Pro Leu Leu Asn Pro Leu Leu Leu Ala Leu Gly Leu Met Ala
   1            5              10            15

Leu Leu Leu Thr Thr Val Ile Ala Leu Thr Cys Leu Gly Gly Phe Ala
          20            25           30

(b2)

Met His Pro Leu Leu Asn Pro Leu Leu Leu Ala Leu Gly Leu Met Ala
   1            5              10            15

Leu Leu Leu Thr Thr Val Ile Ala Leu Thr Cys Leu Gly Gly Phe Ala
          20            25           30

Ser Pro

(b3)

Met Ala Leu Leu Leu Thr Thr Val Ile Ala Leu Thr Cys Leu Gly Gly
   1            5              10            15

Phe Ala

(b4)

Met Ala Leu Leu Leu Thr Thr Val Ile Ala Leu Thr Cys Leu Gly Gly
   1            5              10            15

Phe Ala Ser Pro
          20

16. Rekombinante DNS nach Anspruch 15, dadurch gekennzeichnet, daß die Nucleotidsequenz, die für das Signalpeptid codiert, aus den folgenden Sequenzen (Nb1), (Nb2), (Nb3) und (Nb4) ausgewählt ist:

(Nb1)

ATGCATCCGC TCCTCAATCC TCTCCTGTTG GCACTGGGCC
TCATGGCGCT TTTGTTGACC ACGGTCATTG CTCTCACTTG
CCTTGGCGGC TTTGCC

(Nb2)

ATGCATCCGC TCCTCAATCC TCTCCTGTTG GCACTGGGCC
TCATGGCGCT TTTGTTGACC ACGGTCATTG CTCTCACTTG
CCTTGGCGGC TTTGCCTCCC CA

(Nb3)

ATGGCGCTTT TGTTGACCAC GGTCATTGCT CTCACTTGCC
TTGGCGGCTT TGCC

(Nb4)

ATGGCGCTTT TGTTGACCAC GGTCATTGCT CTCACTTGCC
TTGGCGGCTT TGCCTCCCCA

17. Expressionsvektor, dadurch gekennzeichnet, daß er, mit den für seine Expression notwendigen Mitteln, eine rekombinante DNS nach einem der Ansprüche 11 bis 16 enthält.

18. Eukaryotische Zellen, dadurch gekennzeichnet, daß sie, mit den für ihre Expression notwendigen Mitteln, eine rekombinante DNS nach einem der Ansprüche 11 bis 16 enthalten.

19. Eukaryotische Zellen nach Anspruch 18, dadurch gekennzeichnet, daß sie tierische Zellen sind.

20. Tierische Zellen nach Anspruch 19, dadurch gekennzeichnet, daß sie einen Expressionsvektor nach Anspruch 17 enthalten.

21. Tierische Zellen nach Anspruch 20, dadurch gekennzeichnet, daß sie CHO-Zellen sind.

22. Tierische Zellen nach Anspruch 20, dadurch gekennzeichnet, daß sie COS-Zellen sind.

23. Eukaryotische Zellen nach Anspruch 18, dadurch gekennzeichnet, daß sie Hefezellen sind.

24. Prokaryotischer Mikroorganismus, dadurch gekennzeichnet, daß er durch einen Expressionsvektor nach Anspruch 17 transformiert wird.

25. Prokaryotischer Mikroorganismus nach Anspruch 24, dadurch gekennzeichnet, daß er zur Gattung E. coli gehört.

26. Verfahren zur Herstellung eines Proteins nach einem der Ansprüche 1 bis 8, dadurch gekennzeichnet, daß es einen Kultivierungsschritt tierischer Zellen nach einem der Ansprüche 19 bis 22 umfaßt, gefolgt von der Isolierung und der Reinigung des rekombinanten Proteins.

27. Verfahren zur Herstellung eines Proteins nach einem der Ansprüche 1 bis 8, dadurch gekennzeichnet, daß es einen Kultivierungsschritt von Hefezellen nach Anspruch 23 umfaßt, gefolgt von der Isolierung und der Reinigung des rekombinanten Proteins.

28. Verfahren zur Herstellung eines Medikaments, das das Protein nach einem der Ansprüche 1 bis 8 enthält, dadurch gekennzeichnet, daß es darin besteht, daß das genannte Protein mit einem pharmazeutisch annehmbaren Exzipienten gemischt wird.

FIG. 1a

site liant à Hind III

▼AGCTGGCTCGCATCTCTCCTTCACGCGCCCGCCGCCCTACCTGAGGCCGCCATCCACGCC

1 ------------------------------------------------------------ 60

CCGAGCGTAGAGAGGAAGTGCGCGGGCGGCGGGATGGACTCCGGCGGTAGGTGCGG


GGTGAGTCGCGTTCTGCCGCCTCCCGCCTGTGGTGCCTCCTGAACTGCGTCCGCCGTCTA

61 -----------------------------------------------------------120

CCACTCAGCGCAAGACGGCGGAGGGCGGACACCACGGAGGACTTGACGCAGGCGGCAGAT


GGTAGGCTCCAAGGGAGCCGGACAAAGGCCCGGTCTCGACCTGAGCTCTAAACTTACCTA

121 ----------------------------------------------------------180

CCATCCGAGGTTCCCTCGGCCTGTTTCCGGGCCAGAGCTGGACTCGAGATTTGAATGGAT


GACTCAGCCGGCTCTCCACGCTTTGCCTGACCCTGCTTGCTCAACTCTACGTCTTTGTTT

181 ----------------------------------------------------------240

CTGAGTCGGCCGAGAGGTGCGAAACGGACTGGGACGAACGAGTTGAGATGCAGAAACAAA


# FIG.1b (voir suite)

EP 0 506 574 B1

```
     CGTTTTCTGTTCTGCGCCGTTACAACTTCAAGGTATGCGCTGGGACCTGGCAGGCGGCAT
241  ------------------------------------------------------------300
     GCAAAGACAAGACGCGGCAATGTTGAAGTTCCATACGCGACCCTGGACCGTCCGCCGTA


     CTGGGACCCCTAGGAAGGGCTTGGGGGTCCTCGTGCCCAAGGCAGGGAACATAGTGGTCC
301  ------------------------------------------------------------360
     GACCCTGGGGATCCTTCCCGAACCCCCAGGAGCACGGGTTCCGTCCCTTGTATCACCAGG


     CAGGAAGGGGAGCAGAGGCATCAGGGTGTCCACTTTGTCTCCGCAGCTCCTGAGCCTGCA
361  ------------------------------------------------------------420
     GTCCTTCCCCTCGTCTCCGTAGTCCCACAGGTGAAACAGAGGCGTCGAGGACTCGGACGT


     GA

     ------

     CTTCGA▲
          HindIII
```

FIG.1b (suite)

EP 0 506 574 B1

AGCTTGTCGACTAATACGACTCACTATAGGGCGGCCGCGGGCCCCTGCAGGAATTC

ACAGCTGATTATGCTGAGTGATATCCCGCCGGCGCCCGGGGACGTCCTTAAG

Hind III

BamHI

Site liant à BamHI

ApaI

GGATCCCCCGGGTGACTGACT

CCTAGGGGGCCCACTGACTGACTAG

# FIG.1c

```
  0   AAGCCACCCAGCCTATGCATCCGCTCCTCAATCCTCTCCTGTTGGCACTGGGCCTCATG   59
 -4                   MetHisProLeuLeuAsnProLeuLeuLeuAlaLeuGlyLeuMet    15

 60   GCGCTTTTGTTGACCACGGTCATTGCTCTCACTTGCCTTGGCGGCTTTGCCTCCCCAGGC  119
 16   AlaLeuLeuLeuThrThrValIleAlaLeuThrCysLeuGlyGlyPheAlaSerProGly   35

120   CCTGTGCCTCCCTCTACAGCCCTCAGGGAGCTCATTGAGGAGCTGGTCAACATCACCCAG  179
 36   ProValProProSerThrAlaLeuArgGluLeuIleGluGluLeuValAsnIleThrGln   55

180   AACCAGAAGGCTCCGCTCTGCAATGGCAGCATGGTATGGAGCATCAACCTGACAGCTGAC  239
 56   AsnGlnLysAlaProLeuCysAsnGlySerMetValTrpSerIleAsnLeuThrAlaAsp   75

240   ATGTACTGTGCAGCCCTGGAATCCCTGATCAACGTGTCAGGCTGCAGTGCCATCGAGAAG  299
 76   MetTyrCysAlaAlaLeuGluSerLeuIleAsnValSerGlyCysSerAlaIleGluLys   95

300   ACCCAGAGGATGCTGAGCGGATTCTGCCCGCACAAGGTCTCAGCTGGGCAGTTTTCCAGC  359
 96   ThrGlnArgMetLeuSerGlyPheCysProHisLysValSerAlaGlyGlnPheSerSer  115
```

# FIG. 2  (à suivre)

```
360  TTGCATGTCCGAGACACCAAAATCGAGGTGGCCCAGTTTGTAAAGGACCTGCTCTTACAT  419
116  LeuHisValArgAspThrLysIleGluValAlaGlnPheValLysAspLeuLeuLeuHis  135

420  TTAAAGAAACTTTTTCGCGAGGGACGGTTCAACTGAAACTTCGAAAGCATCATTATTTGC  479
136  LeuLysLysLeuPheArgGluGlyArgPheAsn.                           155

480  AGAGACAGGACCTGACTATTGAAGTTGCAGATTCATTTTTCTTTCTGATGTCAAAAATGT  539

540  CTTGGGTAGGCGGGAAGGAGGGTTAGGGAGGGGTAAAATTCCTTAGCTTAGACCTCAGCC  599

600  TGTGCTGCCCGTCTTCAGCCTAGCCGACCTCAGCCTTCCCCTTGCCCAGGGCTCAGCCTG  659

660  GTGGGCCTCCTCTGTCCAGGGCCCTGAGCTCGGTGGACCCAGGGATGACATGTCCCTACA  719

720  CCCCTCCCCTGCCCTAGAGCACACTGTAGCATTACAGTGGGTGCCCCCCTTGCCAGACAT  779

780  GTGGTGGGACAGGGACCCACTTCACACACAGGCAACTGAGGCAGACAGCAGCTCAGGCAC  839
```

# FIG. 2 (suite)

EP 0 506 574 B1

840 ACTTCTTCTTGGTCTTATTTATTATTGTGTGTTATTTAAATGAGTGTGTTTGTCACCGTT 899

900 GGGGATTGGGGAAGACTGTGGCTGCTGGCACTTGGAGCCAAGGGTTCAGAGACTCAGGGC 959

960 CCCAGCACTAAAGCAGTGGACCCCAGGAGTCCCTGGTAATAAGTACTGTGTACAGAATTC 1019

1020 TGCTACCTCACTGGGGTCCTGGGGCCTCGGAGCCTCATCCGAGGCAGGGTCAGGAGAGGG 1079

1080 GCAGAACAGCCGCTCCTGTCTGCCAGCCAGCAGCCAGCTCTCAGCCAACGAGTAATTTAT 1139

1140 TGTTTTTCCTCGTATTTAAATATTAAATATGTTAGCAAAGAGTTAATATATAGAAGGGTA 1199

1200 CCTTGAACACTGGGGGAGGGGACATTGAACAAGTTGTTTCATTGACTATCAAACTGAAGC 1259

1260 CAGAAATAAAGTTGGTGACAGATAAAAAAAAAAAAAAAA... 1300

FIG. 2 (fin)

EP 0 506 574 B1

```
 1 MetHisProLeuLeuAsnProLeuLeuLeuAlaLeuGlyLeuMetAlaLeuLeuLeuThr 20
                                         |   |   |           |
 1 .   .   .   .   .   .   .   .   .   .   .   .   . MetAlaLeuTrpValThr  6

21 ThrValIleAlaLeuThrCysLeuGlyGlyPheAlaSerProGlyProValProProSer 40
        |       |   |       |   |   |   |       |   |   |   |   |       |
 7 AlaValLeuAlaLeuAlaCysLeuGlyGlyLeuAlaAlaProGlyProValProArgSer 26

41 ThrAla .   .   .   . LeuArgGluLeuIleGluGluLeuValAsnIleThrGlnAsn 56
                       |   |   |   |   |   |   |       |   |   |   |
27 ValSerLeuProLeuThrLeuLysGluLeuIleGluGluLeuSerAsnIleThrGlnAsp 46

57 GlnLysAlaProLeuCysAsnGlySerMetValTrpSerIleAsnLeuThrAlaAspMet 76
        |       |   |   |   |   |   |   |   |   |       |       |
47 Gln . ThrProLeuCysAsnGlySerMetValTrpSerValAspLeuAlaAlaGlyGly 65
```

# FIG. 3 (voir suite)

```
 77 TyrCysAlaAlaLeuGluSerLeuIleAsnValSerGlyCysSerAlaIleGluLysThr 96
        |     | |     | |   |       |       |      | |          |
 66 PheCysValAlaLeuAspSerLeuThrAsnIleSerAsnCysAsnAlaIleTyrArgThr 85


 97 GlnArgMetLeuSerGlyPheCysProHisLysValSerAlaGlyGlnPheSerSerLeu 116
        | |     |       |         |                           |
 86 GlnArgIleLeuHisGlyLeuCysAsnArgLysAlaProThrThrValSerSer .  . 103


117 HisValArgAspThrLysIleGluValAlaGlnPheValLysAspLeuLeuLeuHisLeu 136
              |  |  |  |  |  |  |     |              | |
104  . LeuProAspThrLysIleGluValAlaHisPheIleThrLysLeuLeuSerTyrThr 122


137 LysLysLeuPheArgGluGlyArgPheAsn 146
       |     |  |  |     |     |
123 LysGlnLeuPheArgHisGlyProPhe . 131
```


FIG. 3 (suite)

```
                              .               .                        .
                    ATGCATCCGCTCCTCAATCC......TCT   23
                    |  |  |  | ||  ||   |        |||
                    AGCCCACAGTTCTACAGCTCCCTGGTTCT   29

            .              .             .            .            .
24  CCTGTTGGCACTGGGCCTCATGGCGCTTTTGTTGACCACGGTCATTGCTC   73
    |     |||| ||||||| ||||||||||| | | |||| | ||| | ||||
30  CTCACTGGCTCTGGGCTTCATGGCGCTCTGGGTGACTGCAGTCCTGGCTC   79

            .             .            .             .            .
74  TCACTTGCCTTGGCGGCTTTGCCTCCCCAGGCC............CTGTG   111
    |  ||||||||||| ||   |  ||| |||||||| |              |||||
80  TTGCTTGCCTTGGTGGTCTCGCCGCCCCAGGGCCGGTGCCAAGATCTGTG   129

            .            .              .            .            .
112 CCTCCCTCTACAGCCCTCAGGGAGCTCATTGAGGAGCTGGTCAACATCAC   161
    ||| | ||     |||| | ||||||| ||||||||||||  |||||||||
130 TCTCTCCCTCTGACCCTTAAGGAGCTTATTGAGGAGCTGAGCAACATCAC   179

            .            .            .            .            .
162 CCAGAACCAGAAGGCTCCGCTCTGCAATGGCAGCATGGTATGGAGCATCA   211
    ||    ||||||      |||| || ||||| |||||||||||||||||| |
180 ACAAGACCAGA...CTCCCCTGTGCAACGGCAGCATGGTATGGAGTGTGG   226
```

FIG.4 (voir suite)

```
212  ACCTGACAGCTGACATGTACTGTGCAGCCCTGGAATCCCTGATCAACGTG  261
     |||||  |  |||||   |    ||  ||||||  |||||||||| ||||||||  |||| |
227  ACCTGGCCGCTGGCGGGTTCTGTGTAGCCCTGGATTCCCTGACCAACATC  276

262  TCAGGCTGCAGTGCCATCGAGAAGACCCAGAGGATGCTGAGCGGATTCTG  311
     ||     ||||  ||||||||  |  |  |||||||||||||||  ||    ||    ||||
277  TCCAATTGCAATGCCATCTACAGGACCCAGAGGATATTGCATGGCCTCTG  326

312  CCCGCACAAGGTCTCAGCTGGGCAGTTTTCCAGCTTGCATGTCCGAGACA  361
       |  ||||||  |  |  |             |  |     ||        |||    || |
327  TAACCGCAAGGCCCCCAC.........TACGGTCTCCAGCCTCCCCGATA  367

362  CCAAAATCGAGGTGGCCCAGTTTGTAAAGGACCTGCTCTTACATTTAAAG  411
     ||||||||||  ||  |||||  |||  |||      |  ||||||||       ||||
368  CCAAAATCGAAGTAGCCCACTTTATAACAAAACTGCTCAGCTACACAAAG  417

412  AAACTTTTTCGCGAGGGACGGTTCAACTGA                      441
     ||||  ||||||| |  || |     ||| |  |||
418  CAACTGTTTCGCCACGGCCCCTTCTAATGA.....................  447
```

FIG.4 (suite)

EP 0 506 574 B1

EP 0 506 574 B1

H
i
n
d
I
I
I

AGCTTGGATAAAAGATCCCCAGGCCCTGTGCCTCCCTCTACGGCCCTCAGGGAGCTCAT
---------+---------+---------+---------+---------+---------+
ACCTATTTTCTAGGGGTCCGGGACACGGAGGGAGATGCCGGGAGTCCCTCGAGTA

TGAGGAGCTGGTCAACATCACCCAGAACCAGAAGGCTCCGCTCTGCAATGGCAGCATGGT
---------+---------+---------+---------+---------+---------+
ACTCCTCGACCAGTTGTAGTGGGTCTTGGTCTTCCGAGGCGAGACGTTACCGTCGTACCA

ATGGAGCAtCAACCTGACAGCTGACATGTACTGTGCAGCCCTGGAATCCCTGATCAACGT
---------+---------+---------+---------+---------+---------+
TACCTCGTaGTTGGACTGTCGACTGTACATGACACGTCGGGACCTTAGGGACTAGTTGCA

FIG.5 (voir suite)

```
GTCAGGCTGCAGTGCCATCGAGAAGACCCAGAGGATGCTGAGCGGATTCTGCCCGCACAA
--------+---------+---------+---------+---------+---------+
CAGTCCGACGTCACGGTAGCTCTTCTGGGTCTCCTACGACTCGCCTAAGACGGGCGTGTT

GGTCTCAGCTGGGCAGTTTTCCAGCTTGCATGTCCGAGACACCAAAATCGAGGTGGCCCA
--------+---------+---------+---------+---------+---------+
CCAGAGTCGACCCGTCAAAAGGTCGAACGTACAGGCTCTGTGGTTTTAGCTCCACCGGGT

GTTTGTAAAGGACCTGCTCTTACATTTAAAGAAACTTTTTCGCGAGGGACGGTTCAACTG
--------+---------+---------+---------+---------+---------+
CAAACATTTCCTGGACGAGAATGTAAATTTCTTTGAAAAAGCGCTCCCTGCCAAGTTGAC
```

B
a
m
H
I

```
AAACTTCGAAAGCATCATTATTTGG
--------+---------+----------
TTTGAAGCTTTCGTAGTAATAAACCCTAG
```

# FIG.5 (suite)

EP 0 506 574 B1

ACTIVITÉ DE PROLIFÉRATION DU NC30 SUR LA LIGNÉE B9

FIG.6

NC 30 (SURNAGEANT DE COS)
CONTRÔLE (SURNAGEANT DE COS )
TÉMOIN

FIG.7